# EUROPEAN PATENT SPECIFICATION

(11) **EP 2 287 191 B1**
(45) Date of publication and mention of the grant of the patent: **12.10.2016**
(21) Application number: 10180642.0
(22) Date of filing: 30.05.2000
(51) Int. Cl.: C07K 16/06, C07K 16/18, A61K 39/395, G01N 33/53, A61P 25/02, C12N 15/13, C12N 5/10, C12N 1/19, C12N 1/21, A61K 47/48, C07K 16/46, C12N 15/63, A61K 48/00

(54) **HUMAN IGM ANTIBODIES WITH THE CAPABILITY OF INDUCING REMYELINATION, AND DIAGNOSTIC AND THERAPEUTIC USES THEREOF PARTICULARLY IN THE CENTRAL NERVOUS SYSTEM**
MENSCHLICHE IGM-ANTIKÖRPER, WELCHE DIE FÄHIGKEIT BESITZEN, DEN WIEDERAUFBAU DER MYELINSTRUKTUR ZU INDUZIEREN UND DEREN DIAGNOSTISCHE UND THERAPEUTISCHE ANWENDUNG IM ZENTRALEN NERVENSYSTEM
ANTICORPS IGM HUMAINS DOTÉS DE LA CAPACITÉ D'INDUIRE UNE REMYÉLINISATION, UTILISATIONS DIAGNOSTIQUES ET THÉRAPEUTIQUES POUR LE SYSTÈME NERVEUX CENTRAL

(30) Priority: 10.05.2000 US 568351
(43) Date of publication of application: 23.02.2011
(62) Divisional of application: 00948498.1
(73) Proprietor: MAYO FOUNDATION FOR MEDICAL EDUCATION AND RESEARCH, Rochester, MN 55905 (US)
(72) Inventor: Rodriguez, Moses, Rochester, MN 55902 (US); Miller, David J., Chelsae, MI 48118 (US); Pease, Larry R., Rochester, MN 55906 (US)
(74) Representative: Goodfellow, Hugh Robin

(56) References cited:
- US-A- 5 591 629
- KIRSCHNING E ET AL: "Primary structure of the antigen-binding domains of a human oligodendrocyte-reactive IgM monoclonal antibody derived from a patient with multiple sclerosis.", JOURNAL OF NEUROIMMUNOLOGY, (1999 SEP 1) 99 (1) 122-30., 1 September 1999 (1999-09-01), XP000972985,
- ASAKURA, KUNIHIKO (1) ET AL: "Human IgM from healthy donors enhanced central nervous system remyelination in a murine virus model of multiple sclerosis: A potential novel treatment for multiple sclerosis.", NEUROLOGY, (APRIL 11, 2000) VOL. 54, NO. 7 SUPP. 3, PP. A126-A127. MEETING INFO.: 52ND ANNUAL MEETING OF THE AMERICAN ACADEMY OF NEUROLOGY. SAN DIEGO, CA, USA APRIL 29-MAY 06, 2000 AMERICAN ACADEMY OF NEUROLOGY., April 2000 (2000-04), XP000973012,
- ASAKURA K ET AL: "Targeting of IgMkappa antibodies to oligodendrocytes promotes CNS remyelination.", JOURNAL OF NEUROSCIENCE, (1998 OCT 1) 18 (19) 7700-8., 1 October 1998 (1998-10-01), XP000973031,
- MILLER D J ET AL: "A monoclonal autoantibody that promotes central nervous system remyelination in a model of multiple sclerosis is a natural autoantibody encoded by germline immunoglobulin genes.", JOURNAL OF IMMUNOLOGY, (1995 MAR 1) 154 (5) 2460-9., 1 March 1995 (1995-03-01), XP000941838,
- SORENSEN P S ET AL: "Intravenous immunoglobulin G reduces MRI activity in relapsing multiple sclerosis.", NEUROLOGY, (1998 MAY) 50 (5) 1273-81., May 1998 (1998-05), XP000973014,
- MCNULTY S ET AL: "Ethanolamine is released from glial cells in primary culture on stimulation with foetal calf serum and phorbol ester", NEUROSCIENCE LETTERS, LIMERICK, IE, vol. 139, no. 2, 25 May 1992 (1992-05-25), pages 183-187, XP024362629, ISSN: 0304-3940, DOI: DOI:10.1016/0304-3940(92)90547-K [retrieved on 1992-05-25]
- BANSAL R ET AL: "STIMULATION OF OLIGODENDROCYTE DIFFERENTIATION IN CULTURE BY GROWTH IN THE PRESENCE OF A MONOCLONAL ANTIBODY TO SULFATED GLYCOLIPID", JOURNAL OF NEUROSCIENCE RESEARCH, vol. 21, no. 2-4, 1988, pages 260-267, XP002622106, ISSN: 0360-4012
- STEVEN VERNINO ET AL: "New Purkinje cell antibody (PCA-2): Marker of lung cancer-related neurological autoimmunity", ANNALS OF NEUROLOGY, vol. 47, no. 3, 1 March 2000 (2000-03-01), pages 297-305, XP055198976,
- JOHN E. GREENLEE ET AL: "Antibodies to cerebellar Purkinje cells in patients with paraneoplastic cerebellar degeneration and ovarian carcinoma", ANNALS OF NEUROLOGY, vol. 14, no. 6, 1 December 1983 (1983-12-01), pages 609-613, XP055198975,

## Description

### FIELD OF THE INVENTION

The present invention relates generally to the field of neurobiology, and more particularly to the identification of autoantibodies that play a role in central nervous system function and therapy. The invention also relates to diagnostic and therapeutic materials and methods, including by way of example, pharmaceutical compositions, autoantibodies for use in methods of treatment of diseases associated with neurological impairment, autoantibodies for use in methods of regeneration and restoration of neural function, screening assays and vaccines.

### BACKGROUND OF THE INVENTION

Multiple sclerosis (MS) is a chronic, frequently progressive, inflammatory central nervous system (CNS) disease characterized pathologically by primary demyelination, usually without initial axonal injury. The etiology and pathogenesis of MS are unknown. Several immunological features of MS, and its moderate association with certain major histocompatibility complex alleles, has prompted the speculation that MS is an immune-mediated disease.

An autoimmune hypothesis is supported by the experimental autoimmune (allergic) encephalomyelitis (EAE) model, where injection of certain myelin components into genetically susceptible animals leads to T cell-mediated CNS demyelination. However, specific autoantigens and pathogenic myelin-reactive T cells have not been definitively identified in the CNS of MS patients, nor is MS associated with other autoimmune diseases. An alternative hypothesis, based upon epidemiological data, is that an environmental factor, perhaps an unidentified virus, precipitates an inflammatory response in the CNS, which leads to either direct or indirect ("bystander") myelin destruction, potentially with an induced autoimmune component. This hypothesis is supported by evidence that several naturally occurring viral infections, both in humans and animals, can cause demyelination. One commonly utilized experimental viral model is induced by Theiler's murine encephalomyelitis virus (TMEV) (Dal Canto, M.C., and Lipton, H.L., Am. J. Path., 88:497-500 (1977)).

Kirschning et al. (1999) J. Neuroimmunology 99:122-130 describes the primary structure of the antigen-binding domains of a human oligodendrocytes-reactive IgM monoclonal antibody derived from a patient with multiple sclerosis.

Asakura et al. (2000) Neurology 54:Suppl 3 PP A126-127 describes that human IgM from healthy donors enhanced central nervous system remyelination in a murine virus model of multiple sclerosis.

Asakura et al. (1998) Neuroscience 18(19):7700-8 describes that targeting of IgMkappa antibodies to oligodendrocytes promotes CNS remyelination.

Miller et al. (1995) Journal of Immunology 154(5):2460-9 describes a monoclonal autoantibody that promotes central nervous system remyelination in a model of multiple sclerosis.

Sorensen et al. (1998) Neurology 50(5):1273-81 describes that intravenous immunoglobulin G reduces MRI activity in relapsing multiple sclerosis.

US 5,591,629 describes monoclonal antibodies which promote central nervous system remyelination.

Vernino et al. (2000) Annals of Neurology 47(3):297-305 describes Purkinje cell antibody (PCA-2), a marker of lung cancer-related neurological autoimmunity.

Greenlee et al. (1983) Annals of Neurology 14(6):609-613 describes antibodies to cerebellar Purkinje cells in patients with paraneoplastic cerebellar degeneration and ovarian carcinoma.

The limited efficacy of current therapies for MS and other demyelinating diseases, has stimulated interest in novel therapies to ameliorate these diseases. However, due to the apparently complex etiopathogenesis of these diseases, potentially involving both environmental and autoimmune factors, the need still exists for an effective treatment of these demyelinating disorders.

A group of autoantibodies are known that exhibit activity in the central nervous system, and that were particularly associated with the stimulation of remyelination. One of the objectives of the applicants has been to investigate the full range of activities of the antibodies and concomitantly, to identify other members of the class that demonstrate such activities. Accordingly, it is toward the fulfillment of the foregoing and other objectives that the present invention is directed.

The citation of any reference herein should not be construed as an admission that such reference is available as "Prior Art" to the instant application.

### SUMMARY OF THE INVENTION

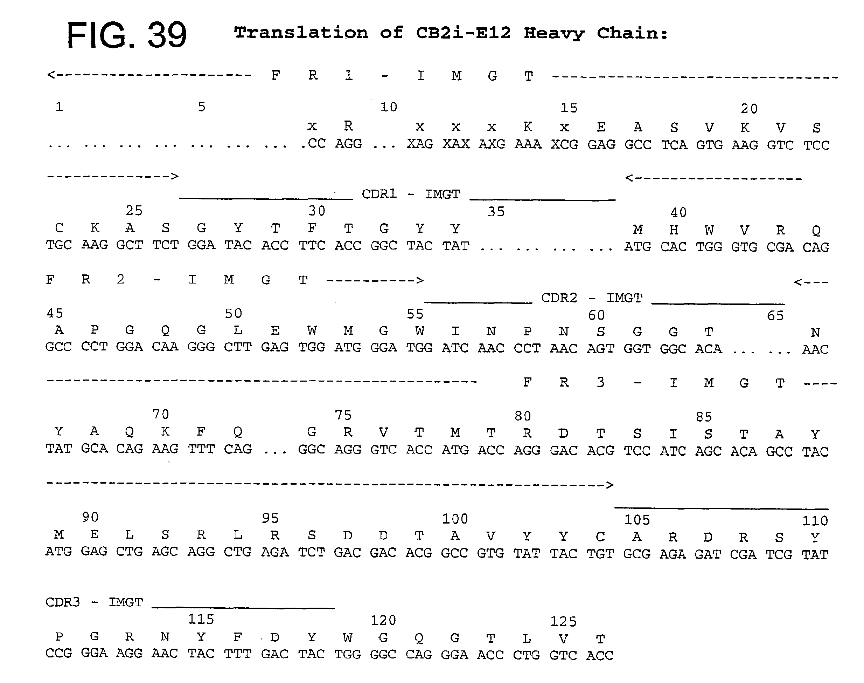

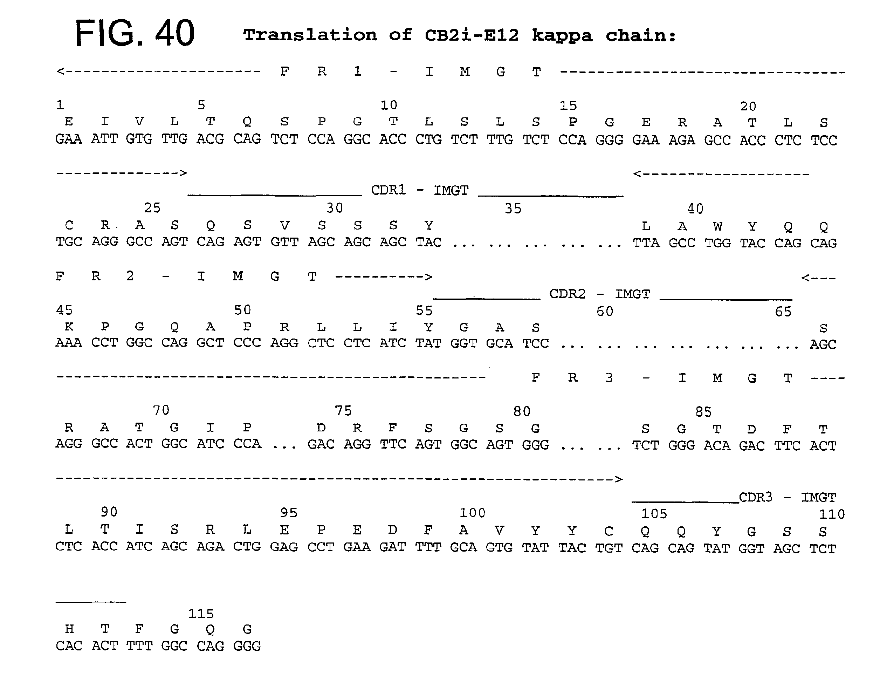

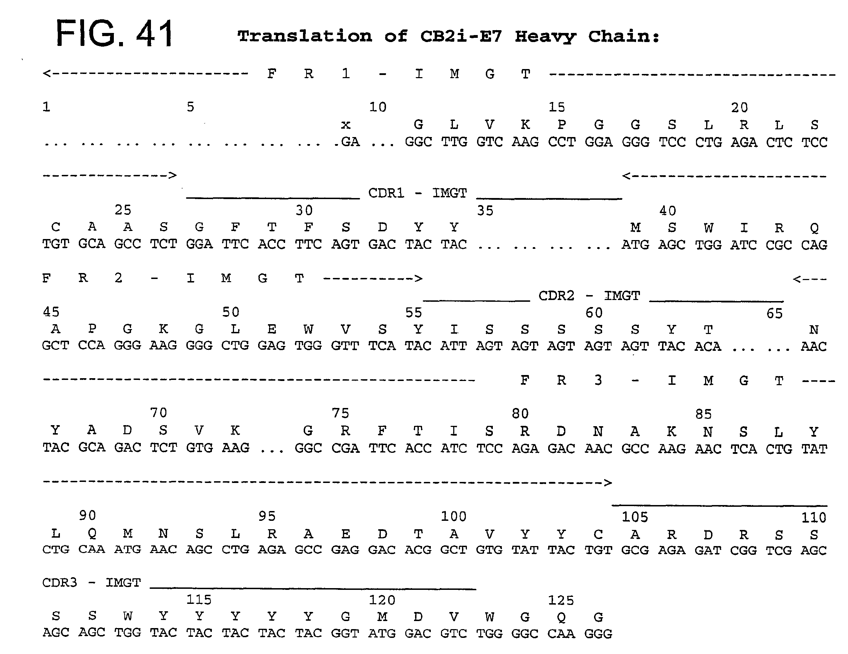

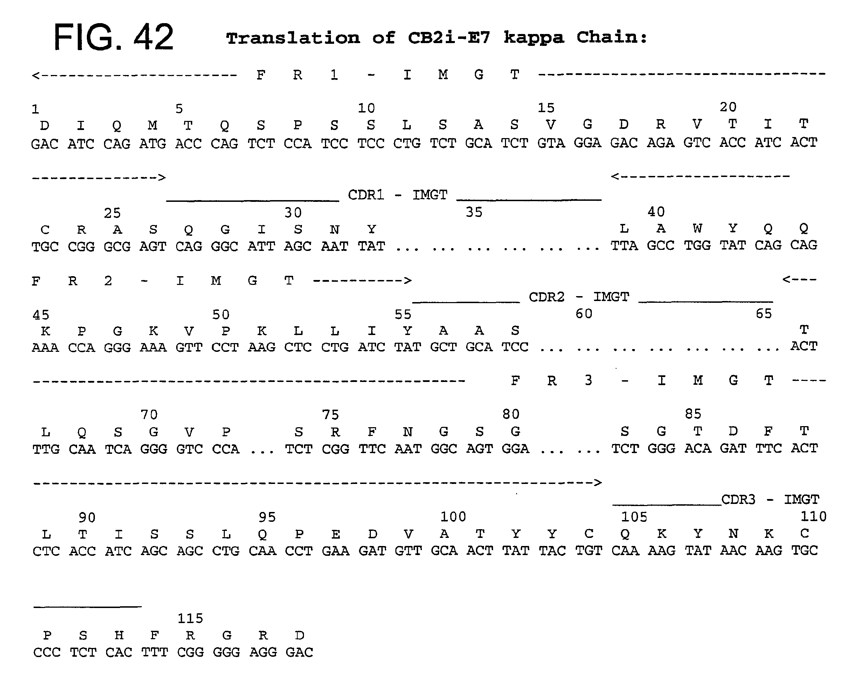

In a first aspect of the present invention, there is provided:
A human antibody, or a mixture, monomer or active fragment thereof which has:
   (i) a heavy chain variable domain comprising the CDR1, CDR2 and CDR3 region sequences as set forth in Figure 39, and a light chain variable domain comprising the CDR1, CDR2 and CDR3 region sequences as set forth in Figure 40; or,
   (ii) a heavy chain variable domain comprising the CDR1, CDR2 and CDR3 region sequences as set forth in Figure 41, and a light chain variable domain comprising the CDR1, CDR2 and CDR3 region sequences as set forth in Figure 42.

Particular antibodies have been identified and tested herein, and the invention accordingly extends to human autoantibodies, which human autoantibodies are exemplified by CB2iE12 and CB2iE7.

Herein disclosed is an assay for screening other antibodies and related binding partners, including haptens and peptide analogs, that may exhibit a like therapeutic activity. Such activities would include the treatment or prevention of neurological injuries or dysfunctions such as multiple sclerosis, ALS, stroke, Parkinsons disease and Alzheimers disease.

The present invention relates in another aspect to the promotion or stimulation of regeneration or remyelination of central nervous system axons in a mammal. Specifically, the present invention relates to autoantibodies of the first aspect for use in methods of stimulating the remyelination of central nervous system (CNS) axons using autoantibodies, including antibodies of the IgM subtype and monomers thereof, and particularly human autoantibodies, or mixtures and/or active fragments thereof, characterized by their ability to bind structures and cells within the central nervous system. The present invention also extends to the preparation and use of human autoantibodies, which human autoantibodies are exemplified by CB2iE12 and CB2iE7. The heavy and light chain variable region sequences of the exemplified antibodies are set forth in the Figures as follows: CB2iE12 is set forth in Figures 39 and 40 (SEQ ID NO: 27 and 29); and CB2iE7 is set forth in Figures 41 and 42 (SEQ ID NO: 31 and 33). The invention extends to antibodies and corresponding antibody proteins, and small molecules such as haptens, that have or correspond at least in part to the sequences set forth in the noted Figures.

The present invention utilizes an analysis of the Ig variable region cDNA sequences and the ability to bind structures and cells within the central nervous system, including but not limited to oligodendrocytes, of these mAbs to ascertain their utility in the methods described herein. Further, this work provides confirmation of the generic utility of this group of autoantibodies as effective in producing remyelination of the central nervous system.

In accordance with a further embodiment of the disclosure and as stated above, a broader class of antibodies has been defined and is disclosed herein. Specifically, human polyclonal and monoclonal autoantibodies are also disclosed and prepared in accordance herewith, that provide greater affinity for neural tissue and both diagnostic and therapeutic capability. The invention extends further in that the newly identified antibodies may be employed for a variety of purposes such as the promotion of remyelination, regeneration of damaged nerve cells, neuronal protection, neuronal outgrowth and the like.

A significant feature and advantage of the present invention resides in the source of the antibodies, as they may be obtained directly from the host or patient, and then used to promote safer self-therapies. More broadly, the development of synthetic antibodies, recombinant antibodies, peptides, small molecules and the like, based on these endogenous materials reduces, if not eliminates, possible pathologies or dysfunctions such as autoimmune reactions, that may result from the *in vivo* introduction and use of exogenous materials. Also, the endogenous origin of the antibodies offers a further advantage in that it may be possible to study the repair process in the patient or host, and potentially identify an underlying mechanism of action in the treatment of the condition, that itself may yield further therapeutic insights and strategies.

Moreover, the identification of the relationship between agents that promote calcium signaling, as by the induction of Ca⁺⁺ peaks, on oligodendrocytes, and the initiation and/or promotion of the noted therapeutic activities, is contemplated to provide a method of identifying therapeutic agents by the demonstration of calcium signaling on *eg*. oligodendrocytes. Accordingly, the invention extends to this use and activity as well.

The antibodies described herein may be used to screen peptide libraries or haptens whereby the reactive peptides or haptens can then be isolated and tested for their ability to remyelinate, induce cellular proliferation, differentiation, neural outgrowth, neurite sprouting and/or Ca⁺⁺ signaling. Once isolated and purified, such peptides can then be used to screen for other polyclonal or monoclonal antibodies or other molecules that may induce remyelination, cellular proliferation or differentiation, neuronal outgrowth, neurite sprouting and/or Ca⁺⁺ signaling, the last mentioned noted herein to be relevant to the proliferation and the corresponding activity of glial cells. Particularly, peptides, haptens, and other molecules corresponding to the antibodies of the invention may be identified by their ability to bind to oligodendrocytes and thereby inducing neural rehabilitation, such as remyelination, regeneration and neuroprotection.

Also herein described are peptides which bind to the autoantibodies described herein, whereby these peptides by virtue of their sequence, three- dimensional structure, or conformational changes arising from antibody binding, can be used in and of themselves as peptide vaccines. These peptides may have neuromodulatory and/or immunomodulatory properties and may provide a method of inducing a neural cell proliferative response and/or neuroprotective, neuroregenerative and/or remyelinating role in mammals in need of such therapy.

Likewise, herein described are haptens that may bind to the peptides, the antibodies and/or other relevant substrates and that may possess immunogenicity, so that they may also function as active components in therapeutic formulations, also including vaccines. As described herein, one or more haptens may be combined with other of the peptides of the present invention, in a vaccine formulation.

These peptides can be formulated as pharmaceutical compositions with stabilizers to prevent proteolytic degradation, thus extending their half-life to be given orally, subcutaneously, intravenously, intranasally, intrathecally or as liposome preparations to mammals in need of such therapy.

The present invention also relates to autoantibodies for use in methods of treating demyelinating diseases in mammals, such as multiple sclerosis in humans, and viral diseases of the central nervous system of humans and domestic animals, such as post-infectious encephalomyelitis, or prophylactically inhibiting the initiation or progression of demyelination in these disease states, using the monoclonal antibodies, or active fragments thereof, of this invention. This invention further relates to *in vitro* methods of producing, and stimulating the proliferation of glial cells, such as oligodendrocytes, and the use of these glial cells to treat demyelinating diseases.

In a further aspect, the invention extends to a group of molecules that will be referred to herein as neuromodulatory agents, and that are notable in their therapeutic activity in the CNS. Accordingly, the invention relates to neuromodulatory agents with particular effectiveness in the CNS, which agents comprise a material selected from the group consisting of an antibody of the present invention, including antibodies of the IgM subtype, monomers thereof, a peptide analog, a hapten, active fragments thereof, agonists thereof, mimics thereof, and combinations thereof. The neuromodulatory agents have the following characteristics: they induce remyelination and/or cellular proliferation of glial cells; and/or evoke Ca⁺⁺ signaling with oligodendrocytes.

More particularly, the antibodies comprehended within the scope of neuromodulatory agents of the invention may be selected from the group consisting of mAb, mixtures thereof, monomers thereof, active fragments thereof, and natural or synthetic autoantibodies having the characteristics of mAb CB2iE12 and CB2iE7, particularly human antibodies. The present neuromodulatory agents may be derived from mammalian cells and specifically, may be derived from human cells. Further, the neuromodulatory agents may comprise a polypeptide having an amino acid sequence selected from the group consisting of FIGURE 39 (SEQ ID NO: 27), FIGURE 40 (SEQ ID NO: 29), FIGURE 41 (SEQ ID NO: 31), FIGURE 42 (SEQ ID NO: 33) and active fragments thereof.

The present invention also relates to a recombinant DNA molecule or cloned gene, or a degenerate variant thereof, which encodes a class of molecules that will also be referred to herein as neuromodulatory agents, and that include and may be selected from the antibodies of the invention, and particularly antibodies having sequences corresponding at least in part, to the sequences presented in FIGURES 39-42 and peptides that may correspond at least in part to the antibodies of the present invention, that will also be referred to herein as antibody peptides, and for example, peptides having one or more sequences corresponding at least in part to FIGURES 39-42 and small molecules such as haptens; including recombinant DNA molecules or cloned genes having the same or complementary sequences.

More particularly, the recombinant DNA molecule comprises a DNA sequence or degenerate variant thereof, which encodes an antibody, a peptide analog thereof, a hapten corresponding thereto, or an active fragment thereof, and which may be selected from the group consisting of:
(I) the DNA sequence encoding a protein having a sequence corresponding to at least a portion of FIGURE 39 (SEQ ID NO: 27);
(J) the DNA sequence encoding a protein having a sequence corresponding to at least a portion of FIGURE 40 (SEQ ID NO: 29);
(K) the DNA sequence encoding a protein having a sequence corresponding to at least a portion of FIGURE 41 (SEQ ID NO: 31);
(L) the DNA sequence encoding a protein having a sequence corresponding to at least a portion of FIGURE 42 (SEQ ID NO: 33);
(N) DNA sequences that hybridize to any of the foregoing DNA sequences under standard hybridization conditions; and
(O) DNA sequences that code on expression for an amino acid sequence encoded by any of the foregoing DNA sequences.

The present disclosure also includes proteins derived from or corresponding to said antibodies, or fragments or derivatives thereof, having the activities noted herein, and that display the amino acid sequences set forth and described above and selected from FIGURES 39-42. The present disclosure likewise extends to haptens that demonstrate the same activities as the proteins or antibody peptides, and that may be administered for therapeutic purposes in like fashion, as by formulation in a therapeutic composition or vaccine. In one embodiment, a therapeutic composition or vaccine including both peptides and haptens may be prepared.

In a further embodiment of the invention, the full DNA sequence of the recombinant DNA molecule or cloned gene so determined may be operatively linked to an expression control sequence which may be introduced into an appropriate host. The invention accordingly extends to unicellular hosts transformed with the cloned gene or recombinant DNA molecule comprising a DNA sequence encoding the present antibody peptides.

In a particular embodiment, the variable region DNA sequence of an antibody of the present invention may be utilized in generating synthetic antibody(ies). In particular, variable region sequence may be combined with its natural or a genetically provided constant region sequence to provide a synthetic antibody. The present invention provides vectors for generating synthetic antibodies derived from and comprising the DNA sequences, particularly variable region sequences, of the antibodies of the present invention.

According to other preferred features of certain preferred embodiments of the present invention, a recombinant expression system is provided to produce biologically active animal or particularly human antibody peptides.

The present invention includes several means for the preparation of clones of the autoantibodies, peptides, corresponding haptens, or other small molecule analogs thereof, including as illustrated herein known recombinant techniques, and the invention is accordingly intended to cover such synthetic preparations within its scope. The isolation of the cDNA and amino acid sequences disclosed herein facilitates the reproduction of the present antibodies or their analogs by such recombinant techniques, and accordingly, the invention extends to expression vectors prepared from the disclosed DNA sequences for expression in host systems by recombinant DNA techniques, and to the resulting transformed hosts.

The disclosure includes an assay system for screening of potential drugs effective to modulate the neurological activity of target mammalian neural cells by potentiating the activity of the present autoantibodies or their analogs. In one instance, the test drug could be administered to a cellular sample with the ligand that suppresses or inhibits the activity of the autoantibodies, or an extract containing the suppressed antibodies, to determine its effect upon the binding activity of the autoantibodies to any chemical sample (including DNA), or to the test drug, by comparison with a control.

The assay system could more importantly be adapted to identify drugs or other entities that are capable of binding to the autoantibodies and/or their targets, including peptides, haptens, other factors or proteins, whether found in the cytoplasm, the nucleus or elsewhere, thereby potentiating antibody activity, including e.g. immune response, neural growth, neuroprotection and remyelination, and the corrresponding therapeutic activities noted herein. Such assay would be useful in the identification of drug candidates from among peptide and other small molecule libraries, sera, and other relevant body fluids, and in the development of drugs that would be specific either in the promotion or the inhibition of particular cellular activity, or that would potentiate such activity, in time or in level of activity. For example, such drugs might be used to promote remyelination, or to treat other pathologies or injuries, as for example, in making CNS neurons able or better able to engage in regrowth or regeneration.

The present invention likewise extends to the development of antibodies corresponding to the neuromodulatory agents of the invention, including naturally raised and recombinantly prepared antibodies. For example, the antibodies could be used to screen expression libraries to obtain the gene or genes that encode the peptides that may function as neuromodulatory agents, and that could function e.g. in a vaccine. Such antibodies could include both polyclonal and monoclonal antibodies prepared by known genetic techniques, as well as bi-specific (chimeric) antibodies, and antibodies including other functionalities suiting them for additional diagnostic use conjunctive with their capability of emulating or modulating the activity of the human autoantibodies that are a part of the neuromodulatory agents of the present invention.

Thus, the neuromodulatory agents, their analogs and/or analogs, and any antagonists or antibodies that may be raised thereto, are capable of use in connection with various diagnostic techniques, including immunoassays, such as a radioimmunoassay, using for example, an antibody to the neuromodulatory agents that has been labeled by either radioactive addition, or radioiodination.
In an immunoassay, a control quantity of the antagonists or antibodies thereto, or the like may be prepared and labeled with an enzyme, a specific binding partner and/or a radioactive element, and may then be introduced into a cellular sample. After the labeled material or its binding partner(s) has had an opportunity to react with sites within the sample, the resulting mass may be examined by known techniques, which may vary with the nature of the label attached.

In the instance where a radioactive label, such as the isotopes ³H, ¹⁴C, ³²P, ³⁵S, ³⁶Cl, ⁵¹Cr, 57Co ⁵⁸Co, ⁵⁹Fe, ⁹⁰Y, ¹²⁵I, ¹³¹I, and ¹⁸⁶Re are used, known currently available counting procedures may be utilized. In the instance where the label is an enzyme, detection may be accomplished by any of the presently utilized colorimetric, spectrophotometric, fluorospectrophotometric, amperometric or gasometric techniques known in the art.

The present disclosure includes an assay system which may be prepared in the form of a test kit for the quantitative analysis of the extent of the presence of the neuromodulatory agents, or to identify drugs or other agents that may mimic or block their activity. The system or test kit may comprise a labeled component prepared by one of the radioactive and/or enzymatic techniques discussed herein, coupling a label to the neuromodulatory agents, their agonists and/or antagonists, and one or more additional immunochemical reagents, at least one of which is a free or immobilized ligand, capable either of binding with the labeled component, its binding partner, one of the components to be determined or their binding partner(s).

In a further embodiment, the present invention relates to autoantibodies for use in certain therapeutic methods which would be based upon the activity of the neuromodulatory agents, their subunits, or active fragments thereof, peptide equivalents thereof, analogs thereof, or upon agents or other drugs determined to possess the same activity. A first therapeutic method is associated with the prevention of the manifestations of conditions causally related to or following from the binding activity of the antibodies or their subunits, and comprises administering an agent capable of stimulating the production and/or activity of the neuromodulatory agents, the corresponding autoantibodies, antibody peptides, active fragments or subunits thereof, either individually or in mixture with each other in an amount effective to prevent or treat the development of those conditions in the host. For example, drugs or other binding partners to the antibodies or their fragments, or the like, may be administered to potentiate neuroregenerative and/or neuroprotective activity, or to stimulate remyelination as in the treatment of multiple sclerosis.

More specifically, the therapeutic method generally referred to herein could include the method for the treatment of various pathologies or other cellular dysfunctions and derangements by the administration of pharmaceutical compositions that may comprise effective inhibitors or enhancers of activation of the neuromodulatory agents, or other equally effective drugs developed for instance by a drug screening assay prepared and used in accordance with an aspect of the present invention discussed above. For example, drugs or other binding partners to the neuromodulatory agents or like proteins, having sequences corresponding at least in part to the sequences as represented by FIGURE 39 (SEQ ID NO: 27), FIGURE 40 (SEQ ID NO: 29), FIGURE 41 (SEQ ID NO: 31), FIGURE 42 (SEQ ID NO: 33) may be administered to inhibit or potentiate neuroregeneration, neuroprotection, or remyelination, as in the treatment of Parkinsons disease or multiple sclerosis. In particular, the protein of CB2iE12 presented in FIGURES 39 and 40, and/or those of CB2iE7 presented in FIGURES 41 and 42, their antibodies, agonists, antagonists, or active fragments thereof, could be prepared in pharmaceutical formulations including vaccines, for administration in instances wherein neuroregenerative and/or neuroprotective therapy or remyelination is appropriate, such as to treat Alzheimers disease, ALS, MS, Parkinsons disease, or spinal cord injury. The present invention includes combinations or mixtures of the antibodies provided herein, wherein more than one of the antibodies, particularly human antibodies, most particularly selected from the group of CB2iE12 and CB2iE7, can be prepared in pharmaceutical and therapeutic compositions or formulations. In addition, the invention provides further combinations of the antibody(ies) with therapeutic compounds, drugs or agents useful in any such neuroregenerative and/or neuroprotective therapy or remyelination. For instance, the antibody formulation or composition of the present invention may be combined with therapeutic compounds for the treatment of multiple sclerosis, including but not limited to beta interferon formulations (Betaseron, etc.) and coploymer 1 (Copaxone).

Accordingly, it is a principal object of the present invention to provide neuromodulatory agents, including the human autoantibodies and corresponding antibody peptides, haptens, analogs and active fragments thereof in purified form that exhibits certain characteristics and activities associated with the promotion of neuroregenerative and/or neuroprotective activity.

It is a further object of the present disclosure to provide a method for detecting the presence, amount and activity of the autoantibodies in mammals in which invasive, spontaneous, or idiopathic pathological states are suspected to be present.

It is a further object of the present disclosure to provide a method and associated assay system for screening substances such as drugs, agents and the like, potentially effective in either mimicking the activity or combating any adverse effects of the autoantibodies and/or their fragments, subunits or the like, in mammals.

It is thus an object of the present invention to provide methods for treating demyelinating diseases in mammals, such as multiple sclerosis in humans, and viral diseases of the central nervous system of humans and domestic animals, such as post-infectious encephalomyelitis, or prophylactically inhibiting the initiation or progression of demyelination in these disease states, using the described monoclonal autoantibodies, active fragments thereof, or other natural or synthetic autoantibodies having the characteristics of the human antibodies exemplified by CB2iE12 and CB2iE7.

It is further an object of the present invention to provide *in vitro* methods of producing, and stimulating the proliferation of, glial cells, such as oligodendrocytes, and the use of these glial cells to treat demyelinating diseases.

It is a still further object of the present invention to provide the present neuromodulatory agents, and pharmaceutical compositions, including vaccines comprising the same, for use in therapeutic methods which comprise or are based upon the present neuromodulatory agents, and particularly the human autoantibodies, fragments, including peptide fragments, haptens, subunits, agonists, binding partner(s), or upon agents or drugs that control the production, or that mimic or antagonize the activities of the neuromodulatory agents.

It is a still further object of the present disclosure to provide assay methods including screening assays, for the identification of drugs and other molecules that mimic or antagonize the neuromodulatory agents of the disclosure and that can consequently be considered for use as therapeutic agents.

Other objects and advantages will become apparent to those skilled in the art from a review of the following description which proceeds with reference to the following illustrative drawings.

### DESCRIPTION OF THE FIGURES

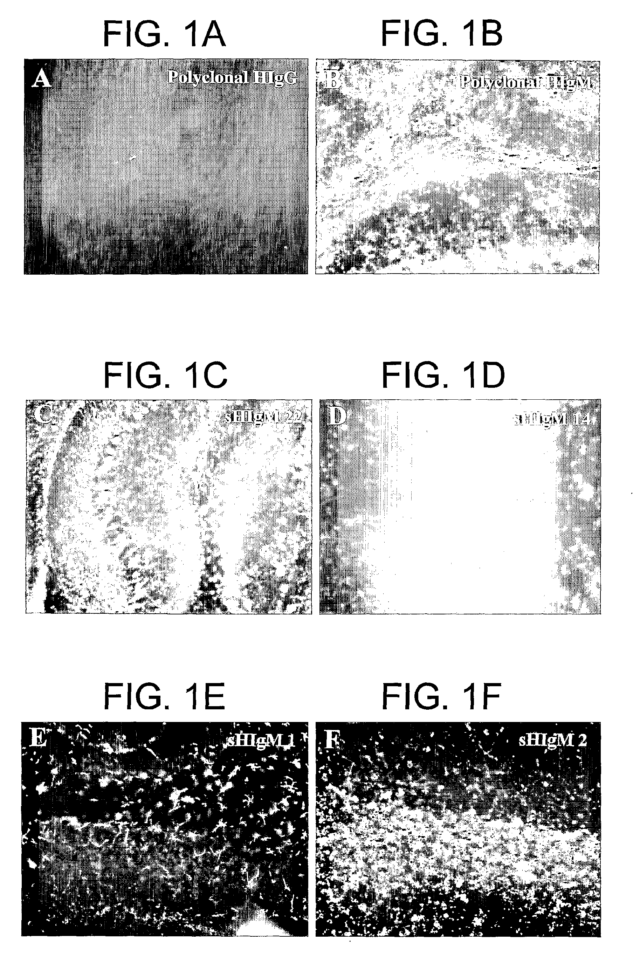

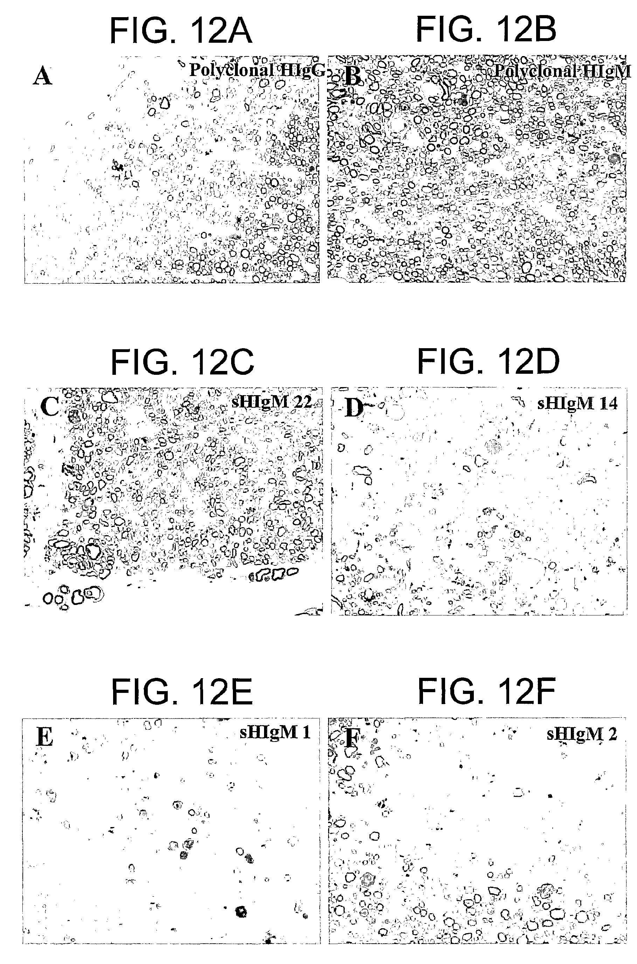

**FIGURE** 1 comprises photographs showing that polyclonal human antibodies and serum-derived human monoclonal IgM antibodies (sHIgMs) bind with high specificity to surface antigens on cells in slices of cerebellum. Indirect immunofluorescent labeling of unfixed slices of postnatal rat cerebellum. sHIgMs demonstrate a variety of specificities to cell populations and structures within an unfixed brain slice. This property was used as one of the criteria to select candidate antibodies to test *in vivo* for the ability to promote remyelination (see **Table 1** and **Fig 12****).** Polyclonal human IgG binds very weakly to many structures within the cerebellum, including white matter and Purkinje cells (**A**), while polyclonal human IgM strongly binds to myelin and presumptive oligodendrocytes within the central white matter of the folia, Purkinje cell bodies and many small cells within the granular and molecular layer **(B).** sHIgM 22 **(C)** binds well to the cytoskeleton of damaged astrocytes overlying the central white matter of the folia, Purkinje cells and their dendritic arborizations, and to small round cells in the molecular layer. sHIgM 22 weakly, but uniformly, labels the surface of granule cells. sHIgM 14 **(D)** binds well to cells of the granular layer and Purkinje cells located at the surface of the slice, while the central white matter of the folia is largely devoid of label. sHIgM 1 **(E)** labels the cytoskeleton of astrocytes overlying the central white matter of the folia. All other structures are identified just above background levels. sHIgM 2 **(F)** binds to cells of the granular layer and to fibers traversing the central white matter of the folia. Magnification x.

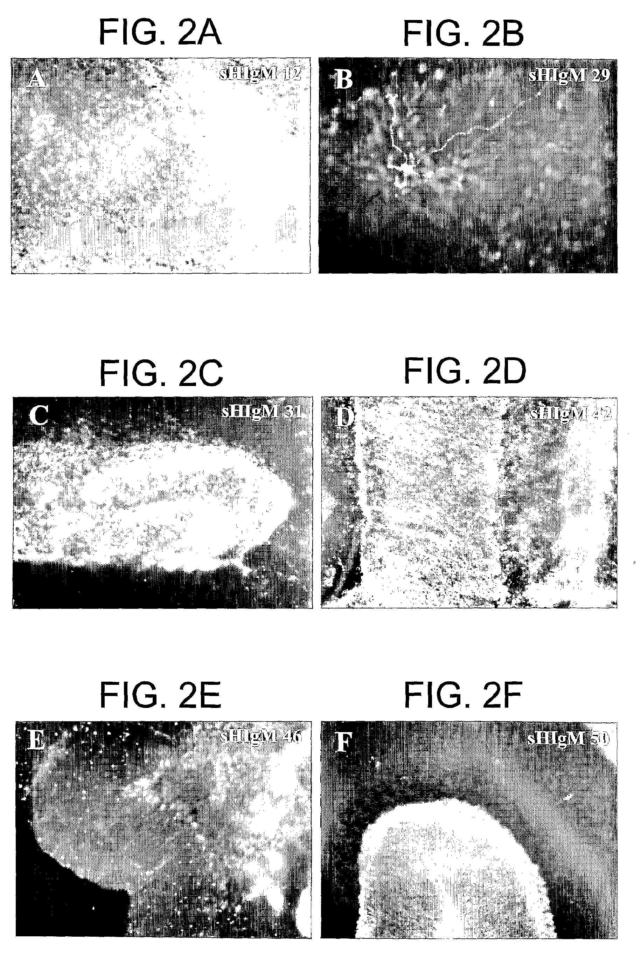

**FIGURE 2** comprises photographs showing that additional sHIgMs bind with high specificity to cells in slices of cerebellum. Indirect immunofluorescent labeling of unfixed slices of postnatal rat cerebellum. sHIgM demonstrate a variety of specificities to cell populations and structures within an unfixed brain slice. sHIgM 12 (A) binds to lend a spongy appearance to the central white matter of the folia, and a uniform label over the molecular layer, reminiscent of an extracellular matrix molecule. Overlying astrocytes are also well defined. sHIgM 29 **(B)** binds weakly to many structures in the cerebellum with an intensity just above background, except for a small population of neurons in the granular and molecular layer. Axon extensions over 100 (m long are clearly delineated. sHIgM 31 **(C)** and sHIgM 50 **(F)** each bind predominately to the granular layer, with little binding to the white matter, Purkinje cells or astrocytes. The binding pattern of sHIgM 50 is also reminiscent of an extracellular matrix molecule. sHIgM 42 **(D)** binds in a fibrous pattern to the entire folia, molecular and granular layers and white matter. sHIgM 46 **(E)** binds in a fibrous pattern to the granular layer and white matter. The Purkinje cell bodies are well defined.

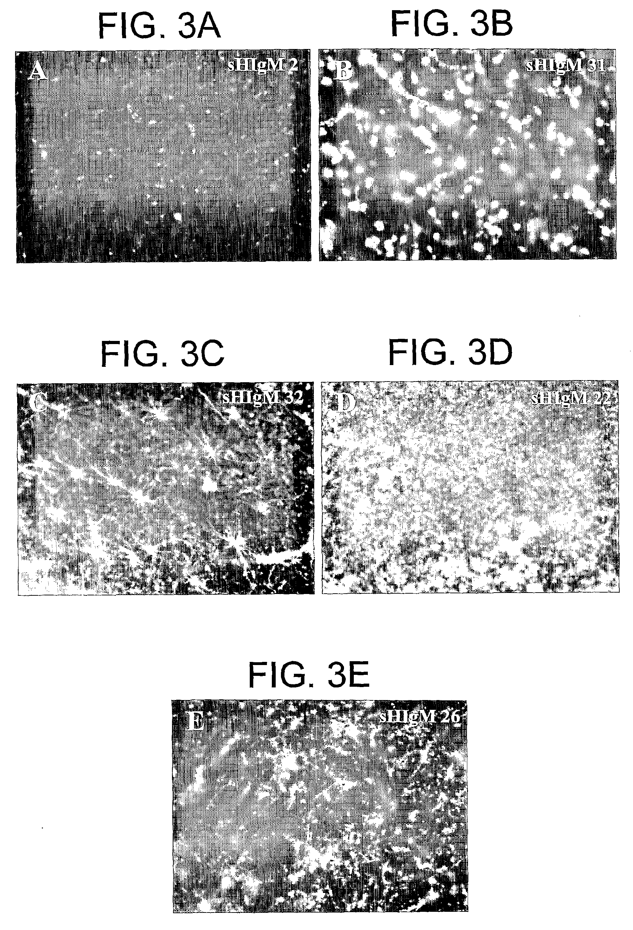

**FIGURE 3** comprise photographs that show that sHIgMs bind with high specificity to unfixed slices of adult human cortical white matter. Indirect immunofluorescent labeling of unfixed slices of adult human cortical white matter. Cortical human white matter was obtained at autopsy from an individual with no CNS infection or trauma. The cause of death was other than CNS-related. Tissue was obtained on ice and maintained cold throughout the antibody labeling procedure. sHIgM 2 (**A**) binds to only a few cells within the field of view. In contrast, others sHIgMs bind human white matter quite well and with a high degree of specificity. sHIgM 32 binds to type 2 astrocyte-appearing cells **(C),** while sHIgM 31 binds to many unidentified round cell bodies **(B).** sHIgM 26 binds to oligodendrocyte-appearing cells and fibrous white matter **(E).** sHIgM 22 binds to human cortical white matter in a manner that is suggestive of an extracellular matrix bound molecule **(D).** Magnification x.

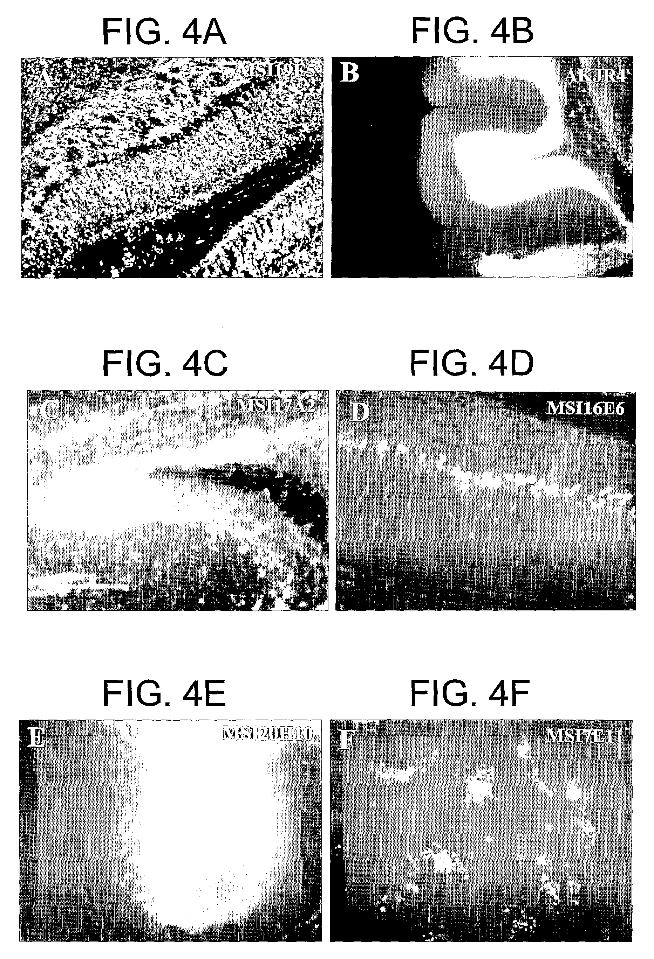

**FIGURE 4** comprise photographs that show that EBV-immortalized human B-cell clone-derived monoclonal IgM antibodies (ebvHIgMs) bind with high specificity to surface antigens on cells in the cerebellum. Indirect immunofluorescent labeling of unfixed slices of postnatal rat cerebellum. ebvHIgMs demonstrate a variety of specificities to cell populations and structures within an unfixed rat brain slice. ebvHIgM MSI19E15 (**A**) binds to fibrous structures within the white matter and to the granular and molecular layer in a pattern of near confluency. ebvHIgM AKJJR4 **(B)** binds almost exclusively to the granular layer. Small cells within the molecular layer are also identified. ebvHIgMs MSI17A2 **(C)** and MSI20H10 **(E)** bind to the central white matter, the granular and molecular layer and Purkinje cells with varying degrees of intensity. ebvHIgM MSI16E6 **(D)** demonstrates a very strong affinity for Purkinje cells and their dendrictic arbors, while the granular layer is far less distinctly labeled. ebvHIgM MSI7E11 **(F)** binds in a punctate manner to only a few glial-appearing cells at the surface of the brain slice. Magnification x.

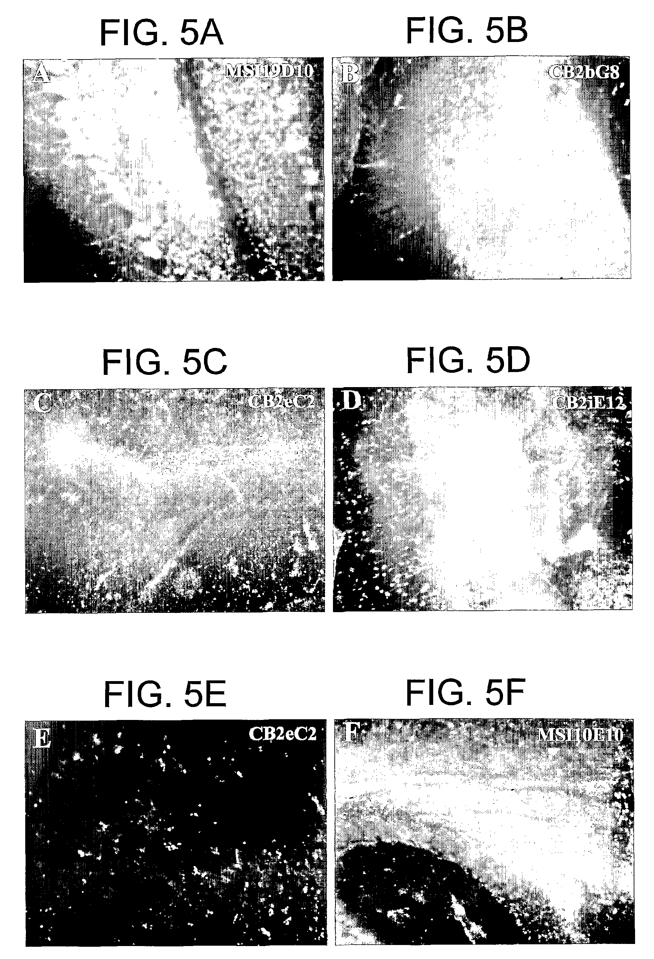

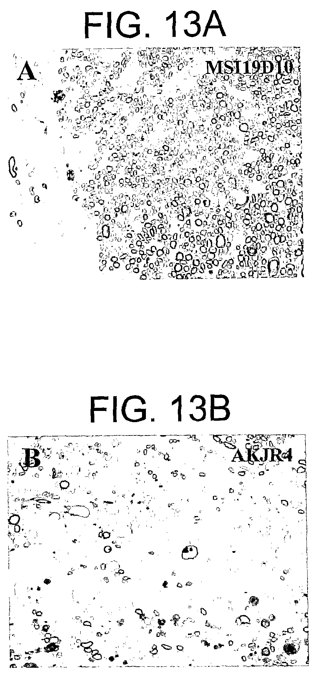

**FIGURE 5** shows that additional ebvHIgMs that bind with high specificity to surface antigens on cells in slices of cerebellum. Indirect immunofluorescent labeling of unfixed slices of postnatal rat cerebellum. ebvHIgMs demonstrate a variety of specificities to cell populations and structures within an unfixed brain slice. Each panel shows the terminal end of a single cerebellar folia, including the central white matter, and the granular, Purkinje and molecular layers. Supernatants containing ebvHIgMs were incubated 1:1 with buffered media on slices of brain. Many ebvHIgMs bind to white matter, Purkinje cell bodies, and small cells within the molecular layer, but with varying affinities. ebvHIgM MSI19D10 (**A**) binds strongly to cells of the granular layer and to Purkinje cells and their dendritic arbors, in addition to weakly identifying white matter and astrocytes. ebvHIgM MSI19D10 was tested for the ability to promote remyelination *in vivo* (see **Table 1** and **Fig 13****).** Other brain-binding ebvHIgMs, CB2bG8**(B)**, CB2eC2 **(C)**, CB2iE12 **(D),** and MSI10E10 **(F)** have been isolated and warrant further study, but have not been tested *in vivo.* CB2eC2 **(E)** is the typical intensity of a non-reactive supernantant. Magnification x.

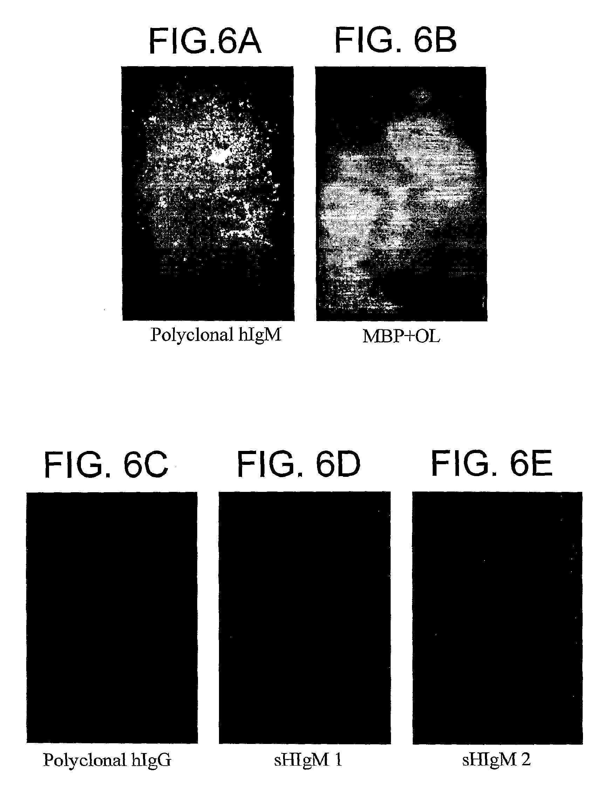

**FIGURE 6** shows that polyclonal human IgM binds to oligodendrocytes in culture. By immunocytochemistry, polyclonal human IgM stains the surface of a subpopulation of oligodendrocytes. No reactivity to oligodendrocyte surface antigens was observed with polyclonal human IgG, or sera from sHIgM 1 or SHIgM 2. Immunocytochemistry with pooled human IgM or IgG in fixed and permeabilized cells showed minimal staining of intracellular structures.

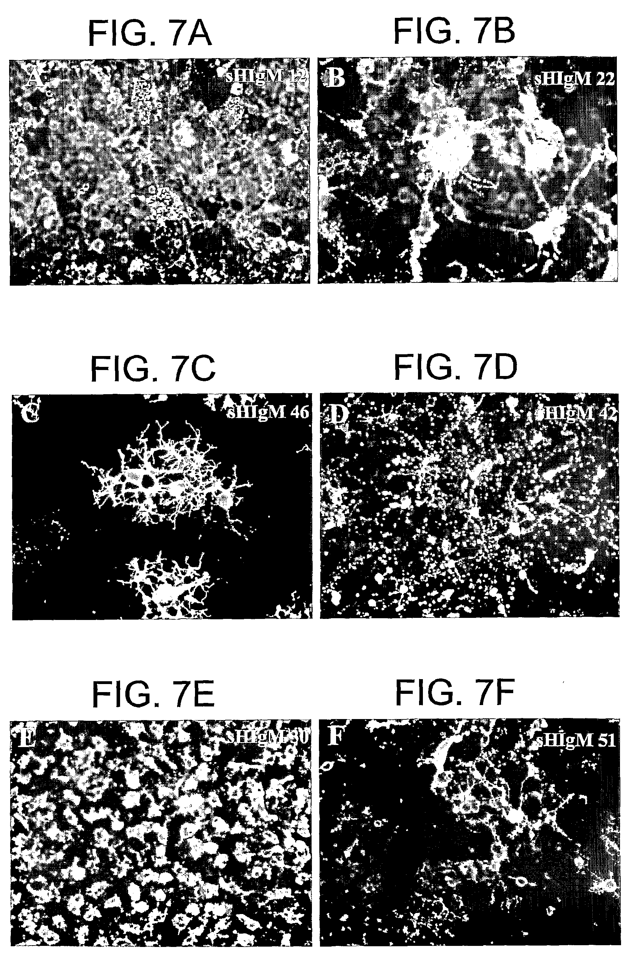

**FIGURE 7** shows that sHIgMs bind with high specificity to surface antigens on glial cells in culture. Indirect immunofluorescent labeling of live rat primary mixed glial cell cultures at nine days post seeding. sHIgMs demonstrate a variety of specificities as to the cell types bound as well as the cell differentiation-stage identified in mixed glial cultures. sHIgM 12 binds to clusters of presumptive oligodendrocyte progenitors (**A**, green label) in the midst of more mature 04 + oligodendrocytes (**A**, red label). sHIgM 22 binds to mature stages of oligodendrocytes **(B)** adherent to the surface of the glial culture. sHIgM 46 strongly binds to both mature stages of oligodendrocyte **(C,** center of figure) and immature stages of oligodendrocyte with a fainter, punctate label **(C,** left side of figure). sHIgM 42 **(D)** and sHIgM 51 **(F)** both bind to mature stages of the oligodendrocyte and faintly, the underlying astrocytes. sHIgM 30 binds to the cell bodies of most cells in the culture, while no process extensions are delineated **(E).** Magnification x.

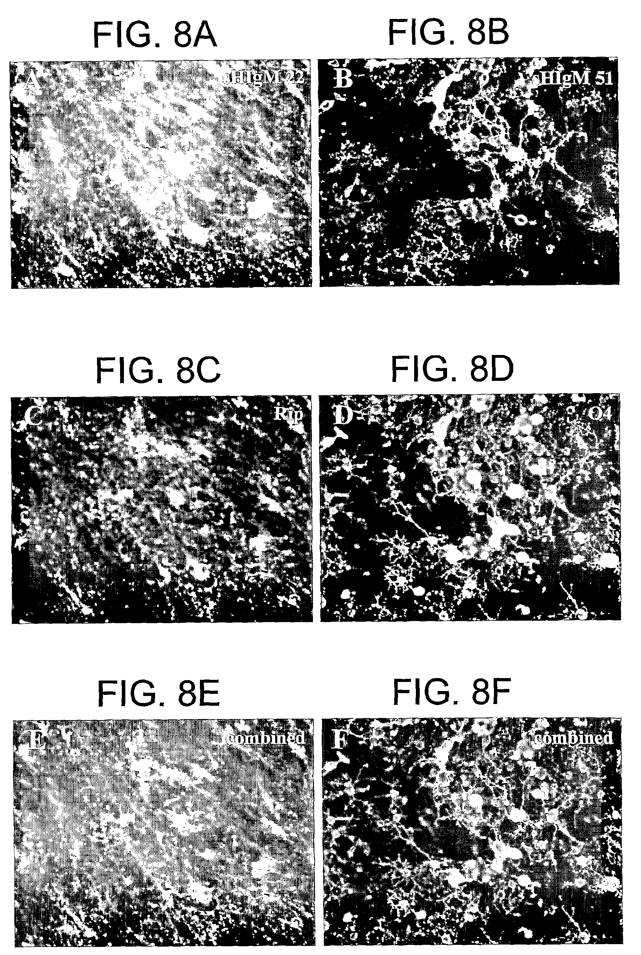

**FIGURE 8** shows that sHIgMs bind to cells of the oligodendrocyte lineage in slices of cerebellum and cultures of mixed primary glial cells. Cells identified by sHIgMs co-label with markers for the oligodendrocyte lineage. Cells that bind sHIgM 22 in an unfixed slice of neonatal rat cerebellum co-label the Rip antibody, a cytoplasmic marker for mature stages of the oligodendrocyte. Double label confocal images demonstrate sHIgM 22 positive cells **(A)** that are also Rip positive **(C).** Images (**A**) and **(C)** are merged in **(E).** Cells that bind sHIgM 51 in mixed primary rat glial cell cultures **(B)** are also 04 positive **(D).** 04, an anti-sulfatide, is a well established marker for the oligodendrocyte lineage that appears prior the cessation of proliferation and is maintained on into the adult myelin sheath. Images **(B)** and **(D)** are merged in **(F).** Magnification x.

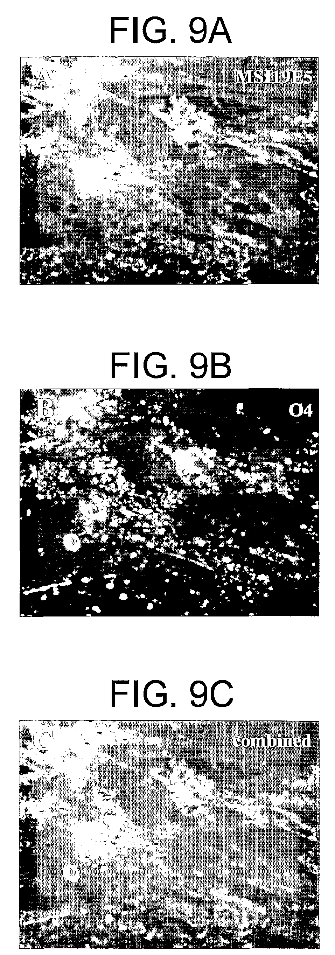

**FIGURE 9** shows that ebvHIgMs bind to cells of the oligodendrocyte lineage in slices of cerebellum. Cells identified by ebvHIgM MSI19E5 in a an unfixed slice of neonatal rat cerebellum co-label with the 04 antibody, an anti-sulfatide and cell-surface marker for oligodendrocytes. Double label confocal images of cells within the white matter of the folia demonstrate ebvHIgM MSI19E5 positive cells (**A**) that are also 04 positive **(B).** Images (**A**) and **(B)** are merged in **(C).**

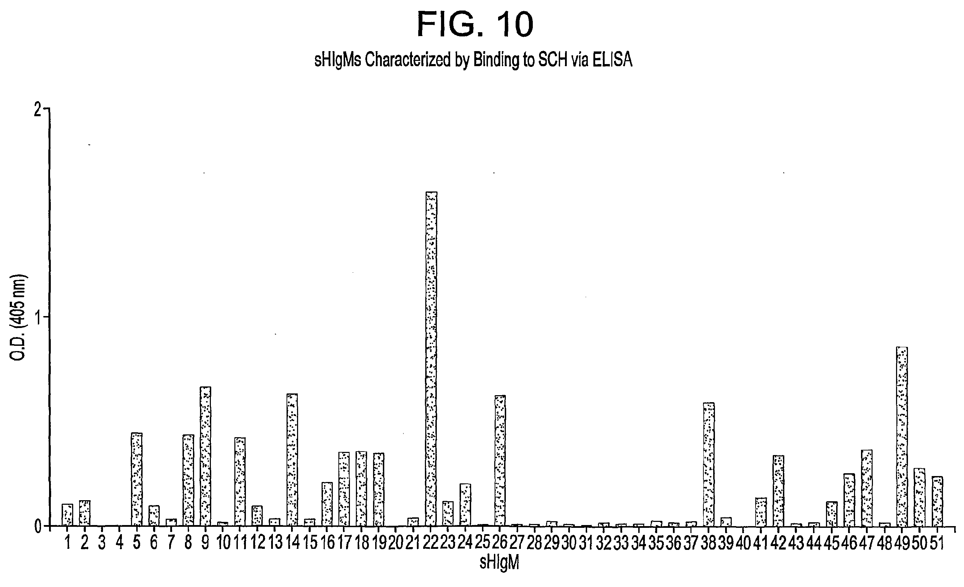

**FIGURE 10** presents the results of screening sHIgMs for binding to CNS antigens found in spinal cord homogenate. sHIgMs were screened for their binding to spinal cord homogenate bound to polystyrene plates. Most of antigens that bind to the plate are lipids and proteins from the white matter of the spinal cord. Thus, strong antibody binding to SCH homogenate may be interpreted as binding to white matter components. Only 1 sHIgM binds to SCH with an OD greater than 1, sHIgM 22. This antibody also binds well to brain slices, oligodendrocytes in culture and has been tested for the ability to promote remyeliation in vivo (see **Table 1**)**.** This simple assay has proven to be a powerful tool in predicting the capacity of an antibody to promote remyelination *in vivo.* Others SHIgMs that bind well to SCH (such as 38 and 49) are under study.

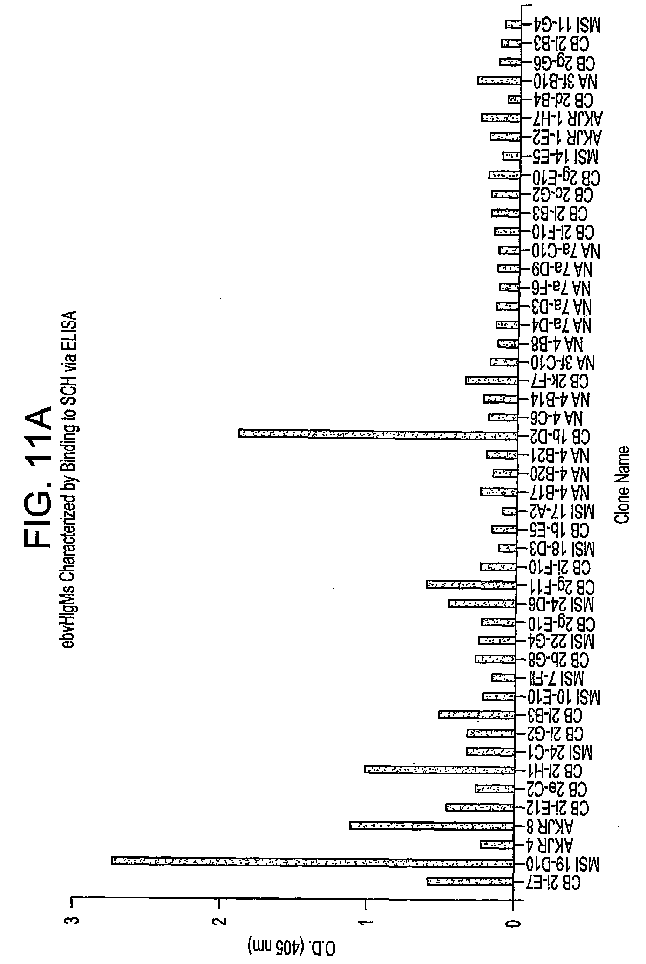

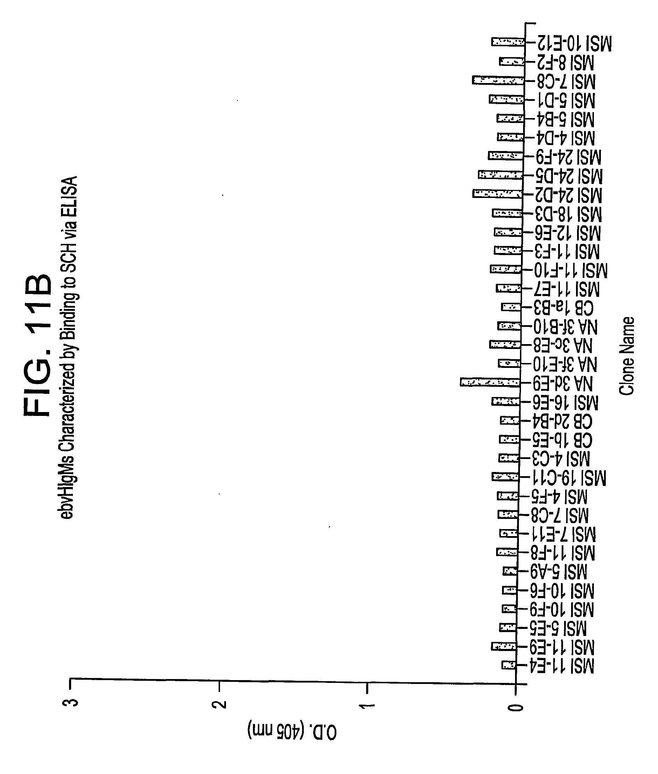

**FIGURE 11** shows the results of screening ebvHIgMs for binding CNS antigens found in spinal cord homogenate. ebvHIgMs were screened for their binding to spinal cord homogenate bound to polystyrene. Four ebvHIgMs bound to SCH homogenate with a OD greater than 1. One of these, MSI19D10 has been tested for the ability to promote remyelination *in vivo.* (see **Table 1**). A low binding antibody, AKJR4 has also been tested in vivo (see **Table 1**). One other strong binding antibody, AKJR8 is under study. The clones CB2iH1 and CB1bD2, produce very little antibody in culture. Again, this simple assay has proven to be a very powerful tool for screening antibodies, and predicting which antibodies are capable of promoting remyelination *in vivo.*
**FIGURE 12** demonstrates that polyclonal human antibodies and a sHIgM promote remyelination in TMEV infected mice. Light photomicrographs of regions of myelin pathology in the spinal cords of SJL/J mice chronically infected with TMEV. Extensive CNS remyelination, characterized by thin myelin sheaths in relation to axon diameter, is observed in mice after treatment with polyclonal human IgG (**A**), polyclonal human IgM **(B),** and sHIgM 22**(C)**. Demyelination without significant remyelination was observed in mice treated with sHIgM 14 (D), sHIgM 1(E) and sHIgM 2. Aradite embedded sections were stained with 1% p-phenylenediamine. Magnification x. Polyclonal human IgM proved to be superior in the ability to promote remyelination *in vivo* than polyclonal human IgG (Table 1). Strong CNS specificity appears to be one of the requirements for an antibody to promote remyelination *in vivo,* but alone is not sufficient to predict an antibody's capacity to promote remyelination.
**FIGURE 13** shows that an ebvHIgM can promote remyelination in TMEV infected mice. Light photomicrographs of regions of myelin pathology in the spinal cords of SJL/J mice chronically infected with TMEV. Extensive CNS remyelination, characterized by thin myelin sheaths in relation to axon diameter, is observed in mice after treatment with ebvHIgM MSI19D10 **(A).** Demyelination without significant remyelination was observed in mice treated with ebvHIgM AKJR4 **(B).** Aradite embedded sections were stained with 1 % p-phenylenediamine. Again, strong CNS specificity appears to be one of the requirements for an antibody to promote remyelination *in vivo,* but alone is not sufficient to predict an antibody's capacity to promote remyelination.

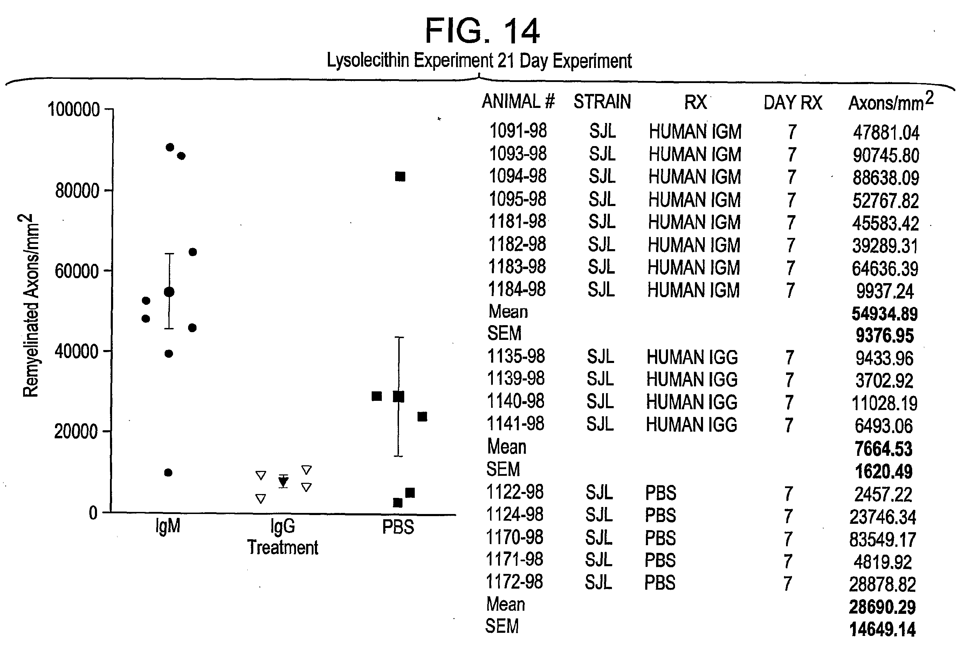

**FIGURE 14** presents the quantitation of myelinated axons in lysolecithin lesions treated with human polyclonal IgM. Remyelinated axons/mm2 in treated vs untreated lysolecithin lesions. There are significantly more myelinated axons in lysolecithin lesions treated with polyclonal human IgM than animals treated with polyclonal human IgG (p < 0.05). One animal in the PBS control group spontaneously remyelinated and thus the difference between the human anitbody treated groups and the control group is not statistically significant (p>0.05).

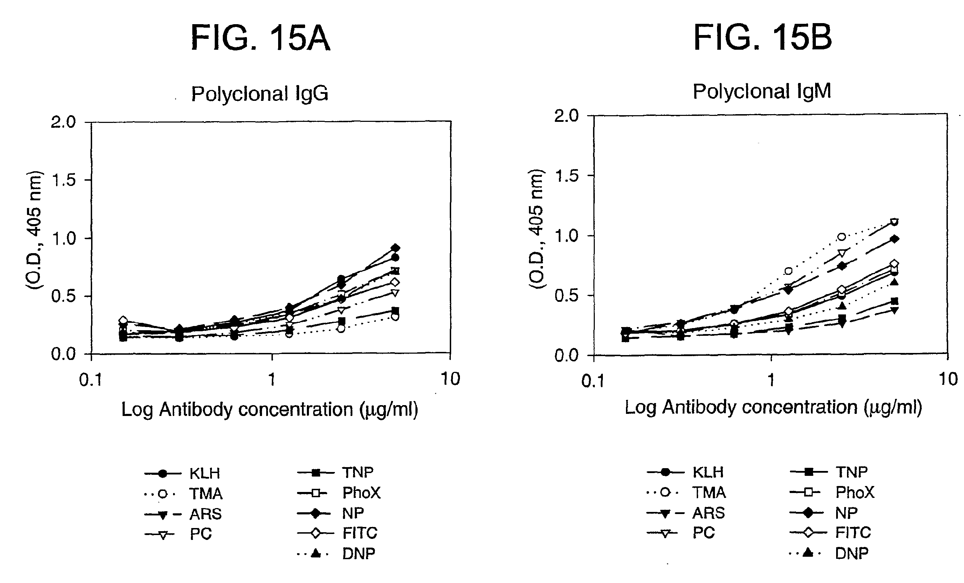

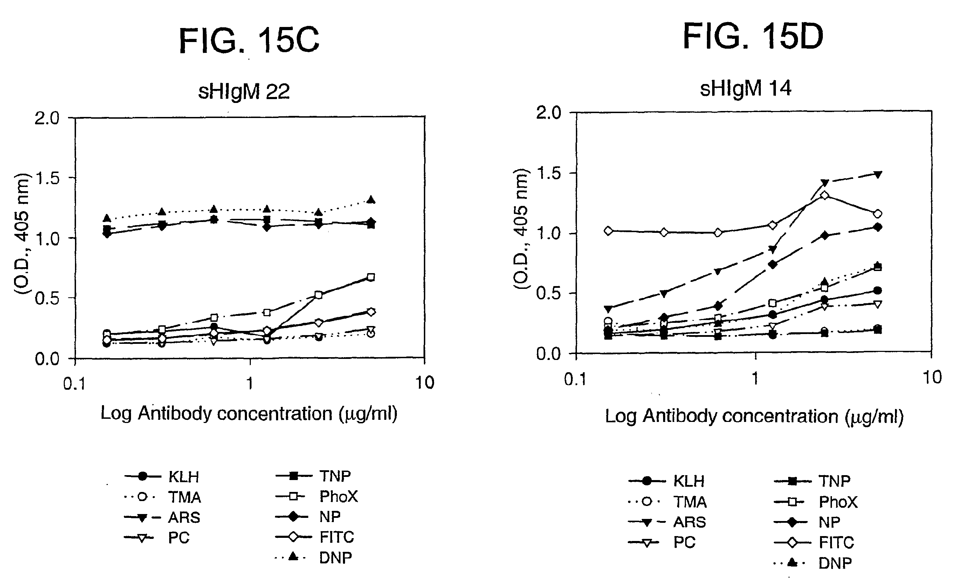

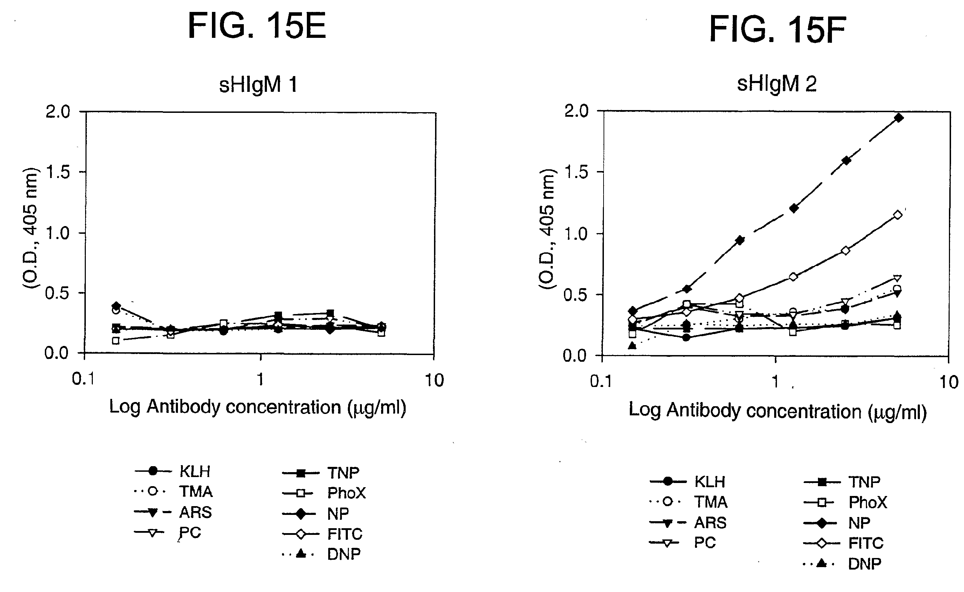

**FIGURE 15** demonstrates that human antibodies are polyreactive to chemical haptens via ELISA. Antigen binding specificities of immunoglobulins assessed by direct ELISA. Chemical hapten reactivities of polyclonal human IgM, polyclonal human IgG. Abbreviations used in these figures: NP, (4-hydroxy-3-nitrophenyl)acetyl; PhoX, phenyloxazolone; TMA, azophenyltrimethylammonium; FITC, fluorescein; PC, azophenylphosphoryl-choline; ARS, azophenylarsonate; TNP, trinytrophenyl acetyl.

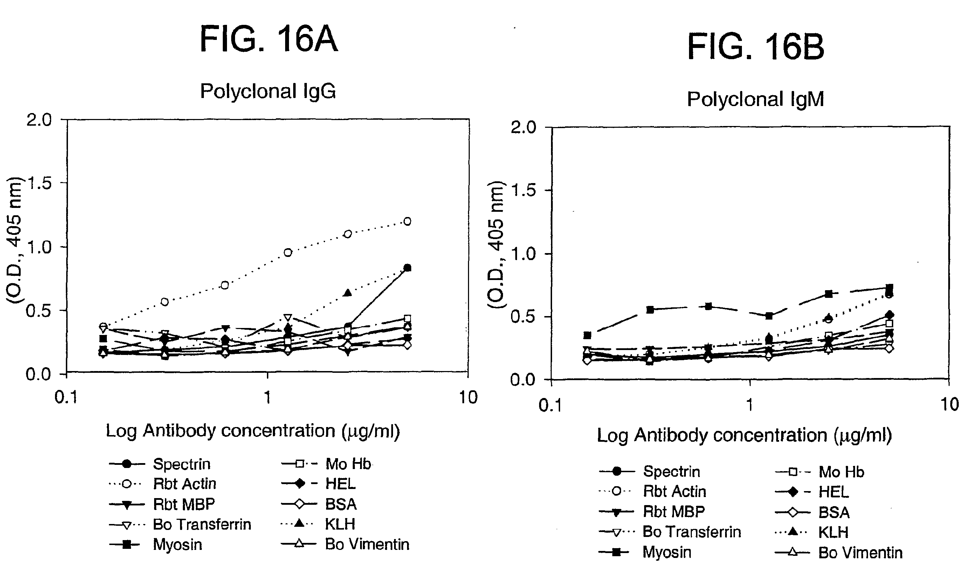

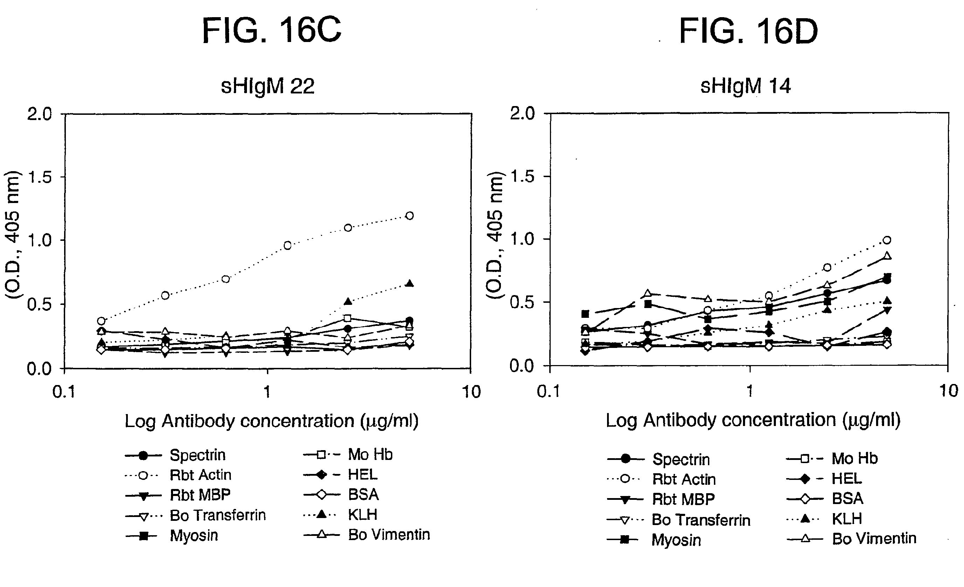

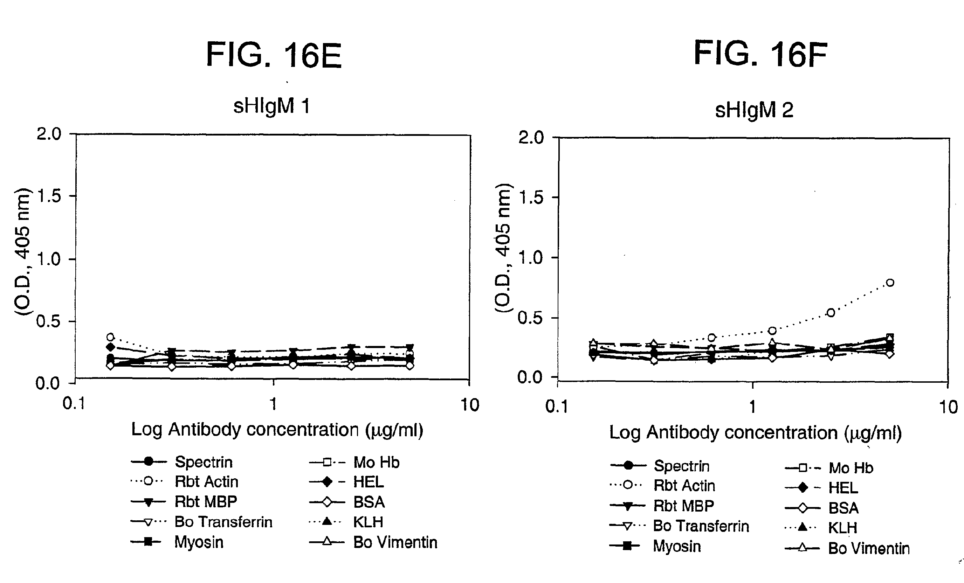

**FIGURE 16** shows that human antibodies are polyreactive to self protein via ELISA. Protein antigen binding specificities of immunoglobulins assessed by direct ELISA. Abbreviations used in these figures: MBP, myelin basic protein; KLH, keyhole limpet hemocyanin; HEL, hen egg lysozyme; BSA, bovine serum albumin;Rbt, rabbit; Bo, bovine; Mo Hb, mouse hemoglobin.

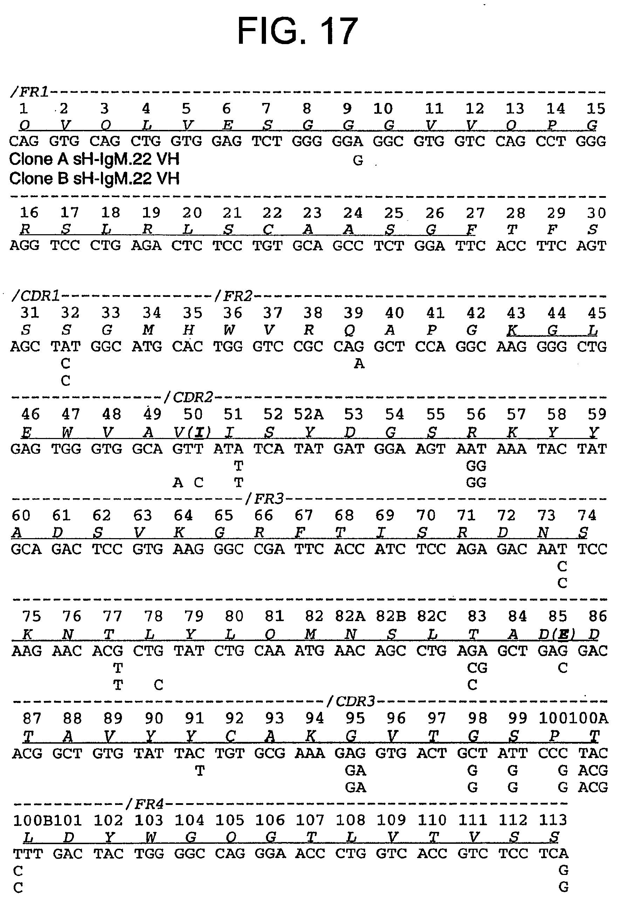

**FIGURE 17** presents the sHIgM 22 heavy chain variable region sequences. The sequence is aligned according to the numbering system of human V_{H} sequences in the publication: Sequence of Proteins of Immunological Interest, Vol I, Fifth Edition (1991), KabatE.A., Wu, T.T., Perry, H.M. Gottesman, K.S. and Foeller, C., NIH Publication. The sHIgM 22 V_{H} is a member of the V_{H} subgroup III. Underlined amino acids have been confirmed by protein sequencing. Amino acid sequence corresponds to sHIgM 22 nucleotide sequence. SHIgM 22 V_{H} type A and B sequences are represented only with nucleotides that differ from the IGHV3-30/3-30-05*01, IGHJ4*02 and IGHD2-21*02 germline sequences. Two amino acid replacements in the protein sequence of sHIgM 22 V_{H} type B are printed in bold. The sequences of both SHIgM 22 V_{H} type A and B most closely matched the IGHV3-30/3-30-5*01 germline sequence (96% homology). References for germline sequences: IMGT, the international ImMunoGeneTics database [http://imgt.cnusc.fr:8104]. (Initiator and coordinator: Marie-Paule Lefranc, Montpellier, France)

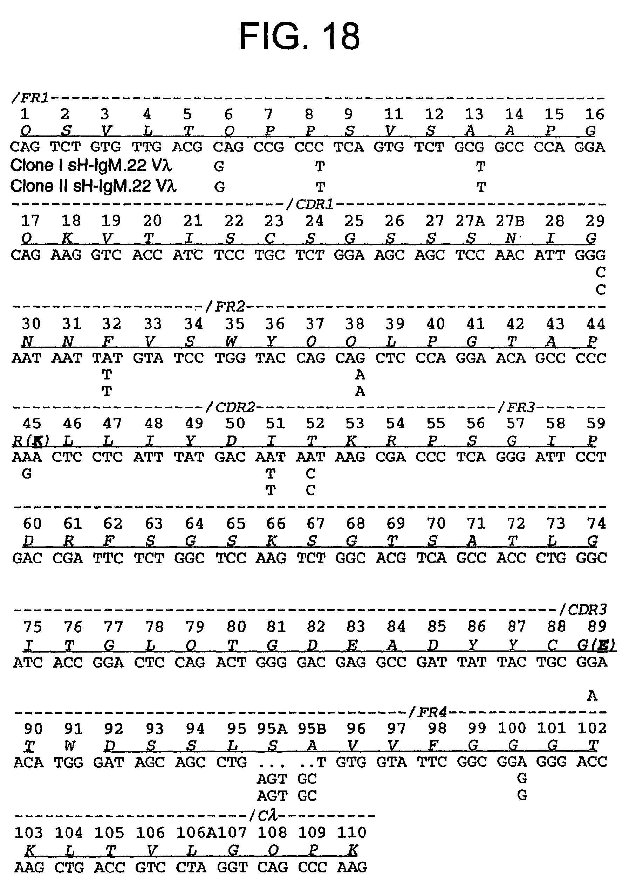

**FIGURE 18** presents the sHIgM 22 light chain variable region sequences. The sequence is aligned according to the numbering system of human V_{H} sequences in the publication: Sequences of Proteins of Immunological Interest, Vol I, Fifth Edition (1991), Kabat E.A., Wu, T.T., Perry, H.M. Gottesman, K.S. and Foeller, C., NIH Publication. V_{λ} sHIgM 22 is a member of the lambda subgroup I. Underlined amino acids have been confirmed by protein sequencing. Amino acid sequence corresponds to sHIgM 22 nucleotide sequence. SHIgM 22 V_{λ} type I and II sequences are represented only with nucleotides that differ from the IGLV1-51*01 and IGLJ3*01 germline sequences. Two amino acid replacements in the protein sequence of sHIgM 22 V_{λ} type II are printed in bold. The V_{λ} sequences from SHIgM 22 most closely matched the IGLV-51*01 germline sequence (97% homology). The two genes differ from their common ancestor by a single nucleotide change. References for germline sequences: IMGT, the international ImMunoGeneTics database [http://imgt.cnusc.fr:8104]. (Initiator and coordinator: Marie-Paule Lefranc, Montpellier, France).

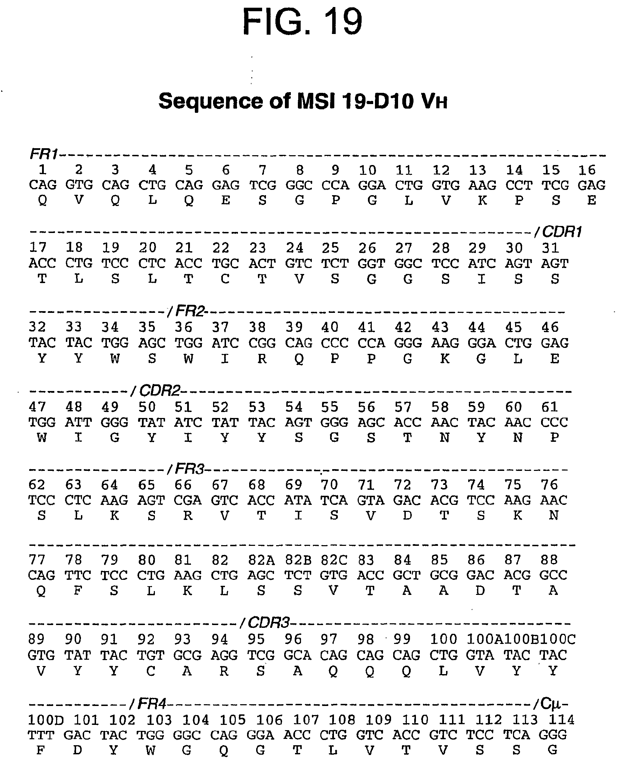

**FIGURE 19** presents the ebvHIgM MSI19D10 heavy chain variable region sequence.

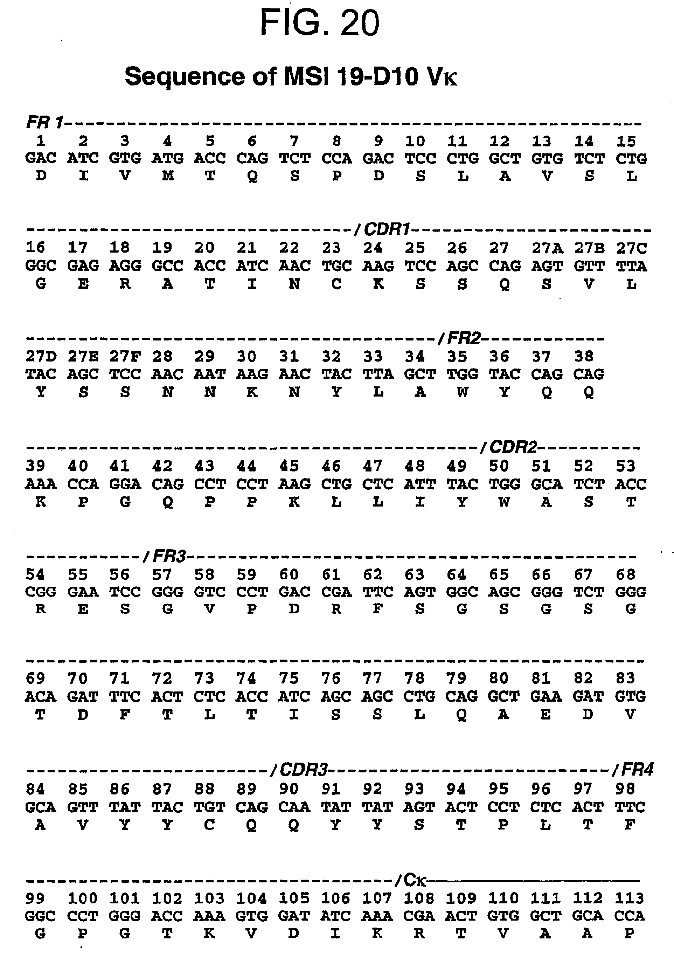

**FIGURE 20** presents the ebvHIgM MSI19D10 light chain variable region sequence.

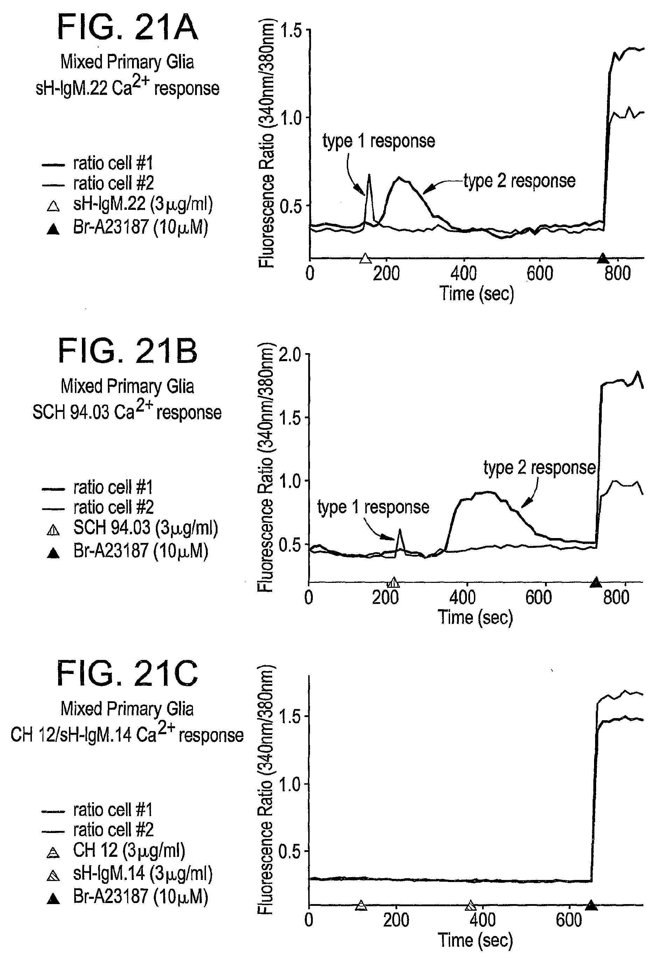

**FIGURE 21** demonstrates that monoclonal antibodies that promote remyelination cause Ca²⁺ flux in glial cells in culture. The three panels demonstrate glial Ca²⁺ responses to four different antibodies: two which promote remyelination in vivo, sHIgM 22 (**A**) and SCH94.03 (B), and two which do not promote remyelination, sHIgM 14 (panel C) and CH12 (C). Cells which responded exhibited one of two different types of calcium spikes, either a fast spike immediately upon addition of antibody (**A & B, red traces),** or a broader spike which appears with a short delay after addition of antibody **(A & B, black traces).** The small colored triangles on the time axis represent the moment antibody (or ionophore) were added. Antibodies sHIgM 22 and SCH94.03 elicited both types of responses but from different subsets of glial cells (panels **A & B).** Antibodies sHIgM14 and CH12, which do not promote remyelination in vivo, were not observed to cause calcium flux in cultured glia (panel C). At the end of each experiment the calcium ionophore Br-A23187 was added to each culture as a control for cellular integrity. Addition of ionophore to viable cells causes a large Ca²⁺ influx which is apparent in each of the experiments that are depicted.

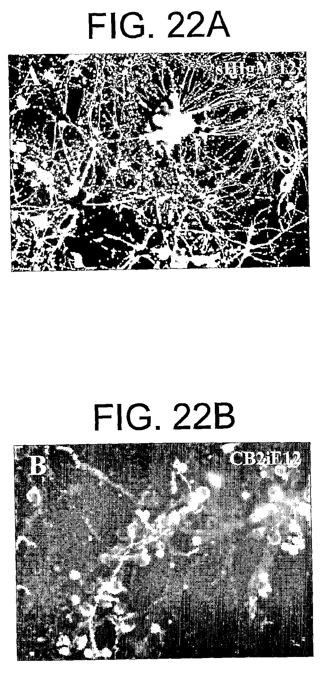

**FIGURE 22** demonstrates that sHIgMs and ebvHIgMs bind to primary neurons in culture. Indirect immunofluorescent labeling of live primary rat granule cells at six days in culture. sHIgM 12 binds to virtually all axon and dendritic extensions of cerebellar granule cells in culture (**A**). The binding pattern is similar to that observed with anti-ganglioside antibodies, such as mouse antibody A2B5. A2B5 has been shown to promote remyelination *in vivo* (Asakara et al, 1998). ebvHIgM CB2iE12 binds only to granule cell bodies and their proximal axon extensions **(B).** The antigen recognized by CB2iE12 is developmentally regulated, for granule cells in culture are negative for CB2iE12 staining until 4-5 days after plating. Magnification x.

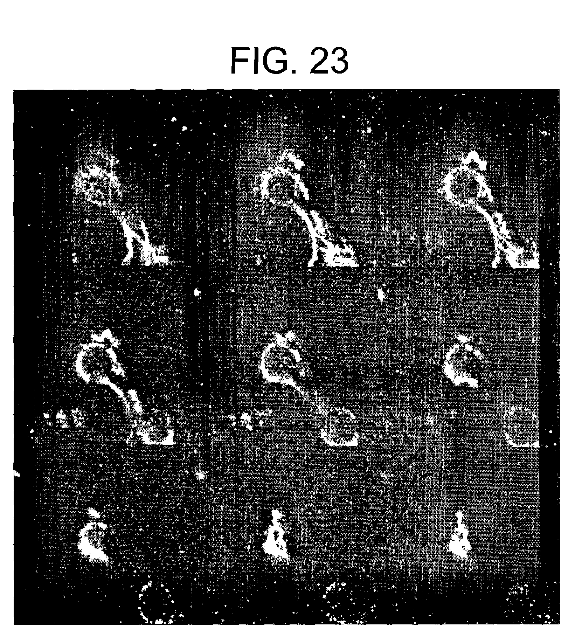

**FIGURE 23** demonstrates that mouse monoclonal antibody SCH94.03 binds to the surface of granule cells in culture. Indirect immunofluorescent labeling and confocal serial imaging demonstrates that the mouse monoclonal antibody SCH94.03 binds only to the surface in granule cell neurons in culture. The series of images were taken 1 um apart and clearly show the concentric circular rings expected of an externally labeled spherical cell body with process extensions.

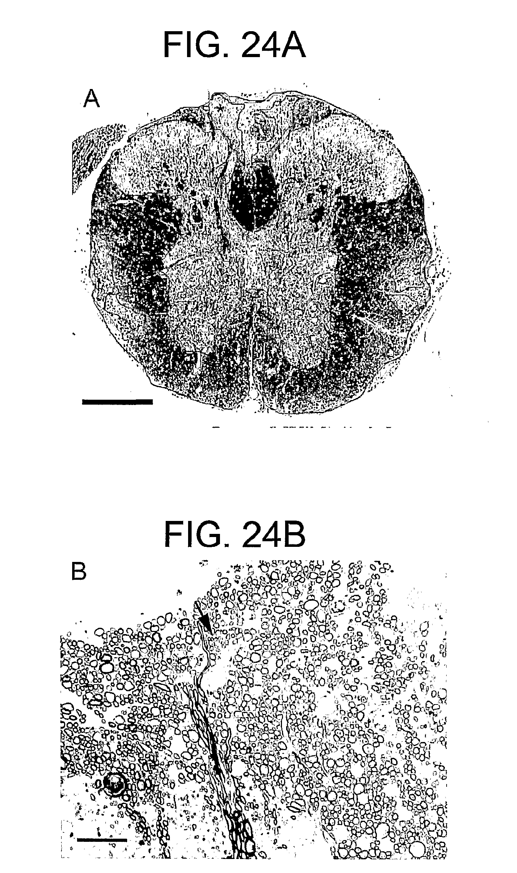

**FIGURE 24** depicts the methodology used to quantify white matter, white matter pathology and remyelination in the spinal cords of TMEV-infected mice. Light photomicrograph of a thorasic level spinal cord section from an SJL/J mouse chronically infected with TMEV and treated with polyclonal human IgM (A). White matter at the periphery stains darker than the lighter central gray matter. The area of total white matter is traced (indicated by the red outlines), at a magnification of 40x. Then at a magnification of 100x the areas of white matter pathology are traced (indicated by the green outlines). In this example, the areas of white matter pathology appear as lighter areas at the periphery of the section. Finally, at a magnification of 250x the areas of OL remyelination (indicated by the blues outlines) and SC remyelination (indicated by the yellow outline) are traced. OL remyelination is characterized by thin myelin sheaths in relation to axon diameter. The percent area of white matter pathology is calculated by dividing the area in green by the area in red x 100. The percent area of OL remyelination is calculated by dividing the area in blue by the area in green x 100. Ten spinal cord cross sections are traced for each animal considered and the areas combined to calculate a score for that animal. Generally, 7-8 animals are treated in each experimental group to allow for deaths and animals that do not contain at least 5% total white matter pathology. Usually 4-5 treated animals meet the criteria for inclusion into the final data set. A high magnification field of the dorsal column white matter (B, from the area indicated by the asterisk in A) demonstrates significant OL remyelination (arrow). Scale bars are 250 µm in A and 20 µm in B.

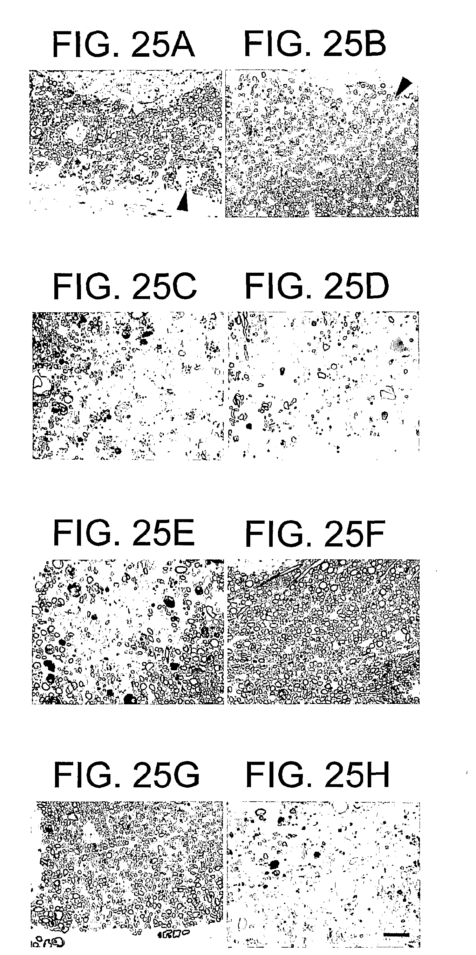

**FIGURE 25** Following treatment with human Abs, chronically TMEV-infected mice demonstrate significant OL remyelination. Light photomicrographs of representative areas of spinal cord white matter pathology of different treatment groups. Treatment with IVIg resulted in significant OL remyelination (A). Almost complete OL remyelination, characterized by densely packed thin myelin sheaths in relation to axon diameter (B, arrowhead), was observed in sections from the spinal cords of mice following treatment with polyclonal human IgM (B) and human mAbs sHIgM 22 (F) and sHIgM46 (G). In contrast, following treatment with human mAbs sHIgM1 (C), sHIgM2 (D), sHIgM14 (E) or PBS (H) mice demonstrated white matter pathology without significant OL remyelination. Infiltrating inflammatory cells and macrophages ingesting myelin debris (A, arrowhead), signs of active myelin destruction were also evident. Spinal cord cross sections in four of eight animals treated with sHIgM22 and five of five animals treated with sHIgM46 contained at least one area of nearly confluent OL remyelination, a rare event indicating significant tissue repair. In contrast, the 10 spinal cord cross sections from each mouse treated with sHIgM1, sHIgM2, sHIgM14, or PBS contained none. Scale bar is 20 mm.

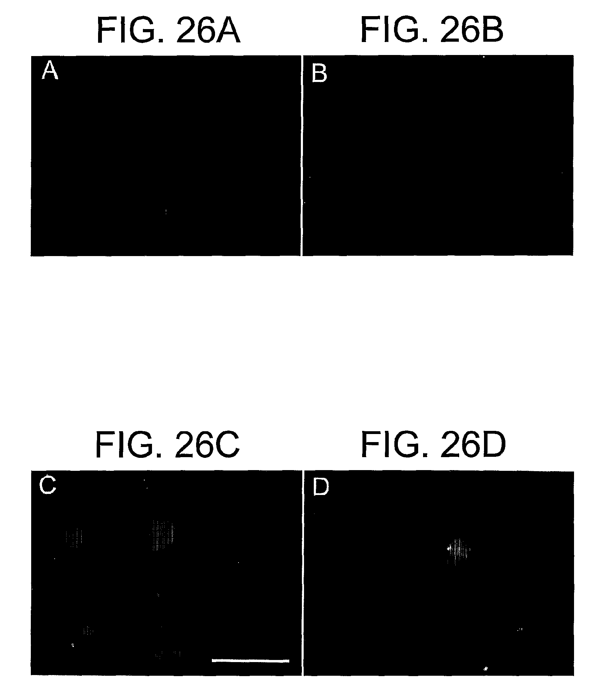

**FIGURE 26** Human mAbs isolated for their ability to bind to rat OLs also bind to the surface of human OLs in culture. sHIgM14 (A), which did not promote remyelination, and sHIgM22 (B) and sHIgM46 (C), which did promote remyelination, bound to the perikaryon and elaborate process and membrane extensions of sulfatide positive human OLs maintained in culture for 3 weeks. sHIgM2 (D, green channel) is an example of a human mAb that did not bind to sulfatide positive (D, red channel) human OLs. Nuclei are labeled blue. IVIg, polyclonal human IgM, and human mAbs sHIgM1 and sHIgM2 did not bind to the surface of human OLs at any time point examined. Scale bar is 25 mm.

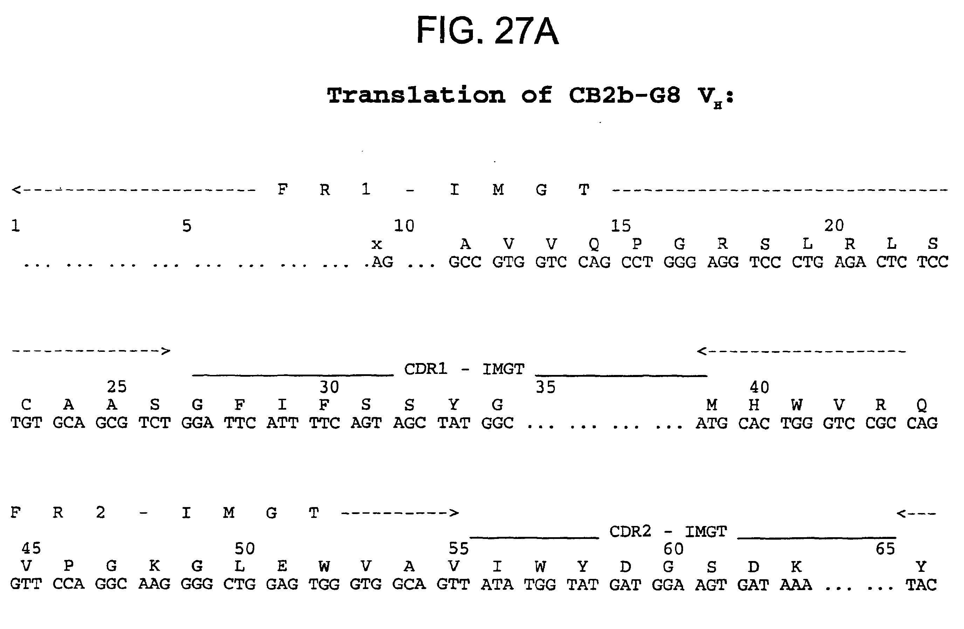

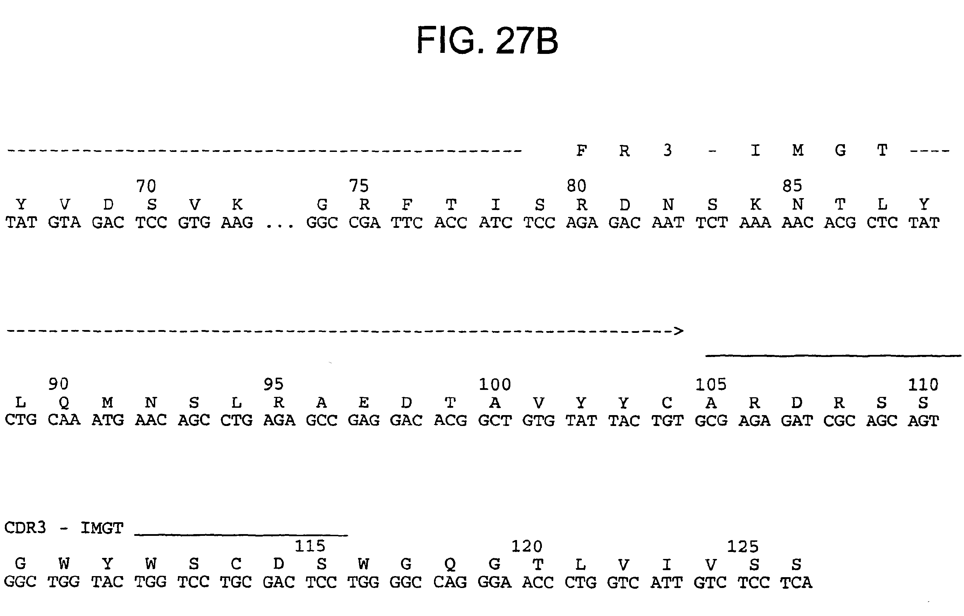

**FIGURE 27** presents the heavy chain variable region sequence of EBV transformant antibody CB2b-G8.

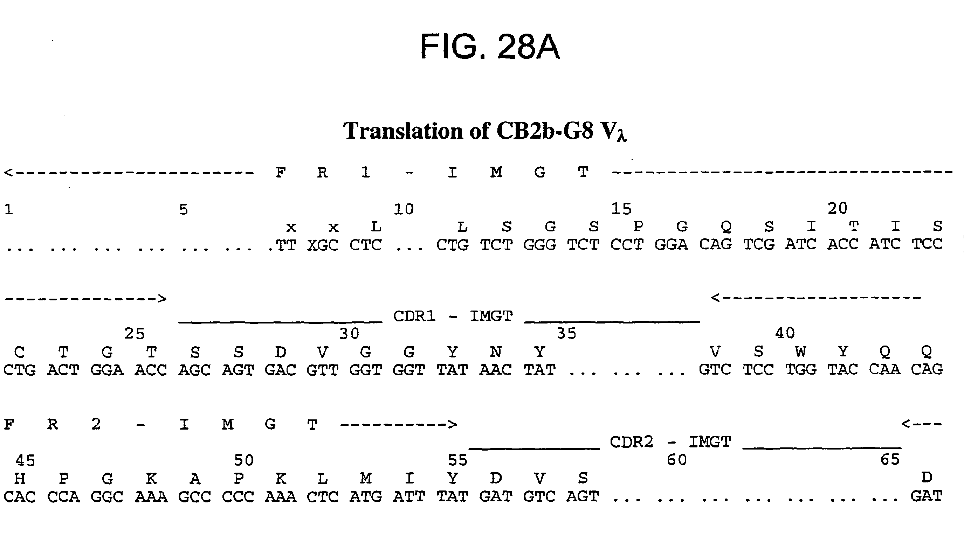

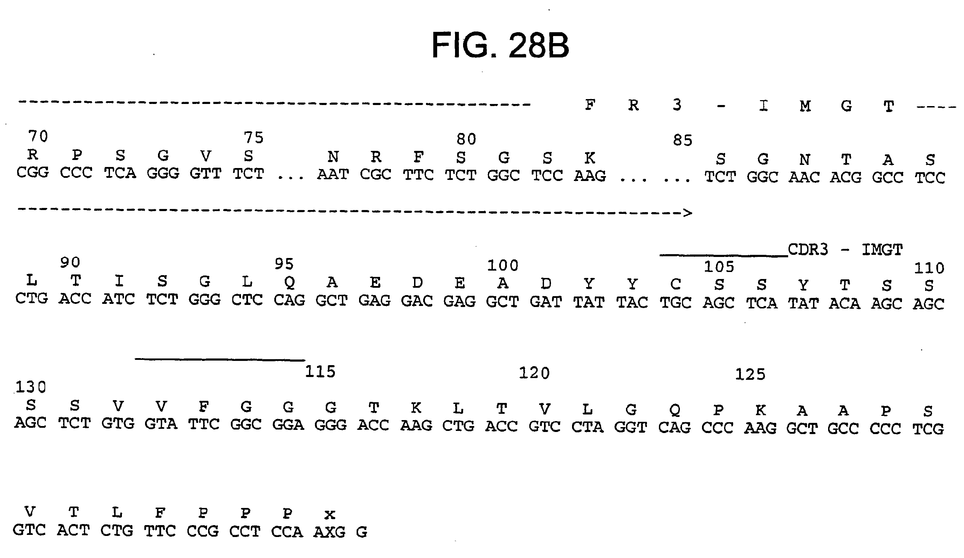

**FIGURE 28** presents the light chain variable region sequence of EBV transformant antibody CB2b-G8.

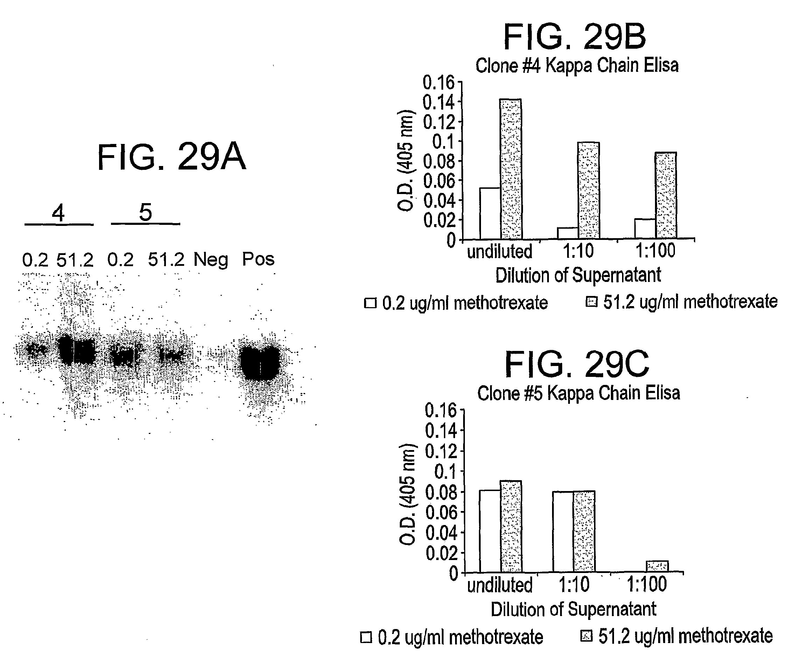

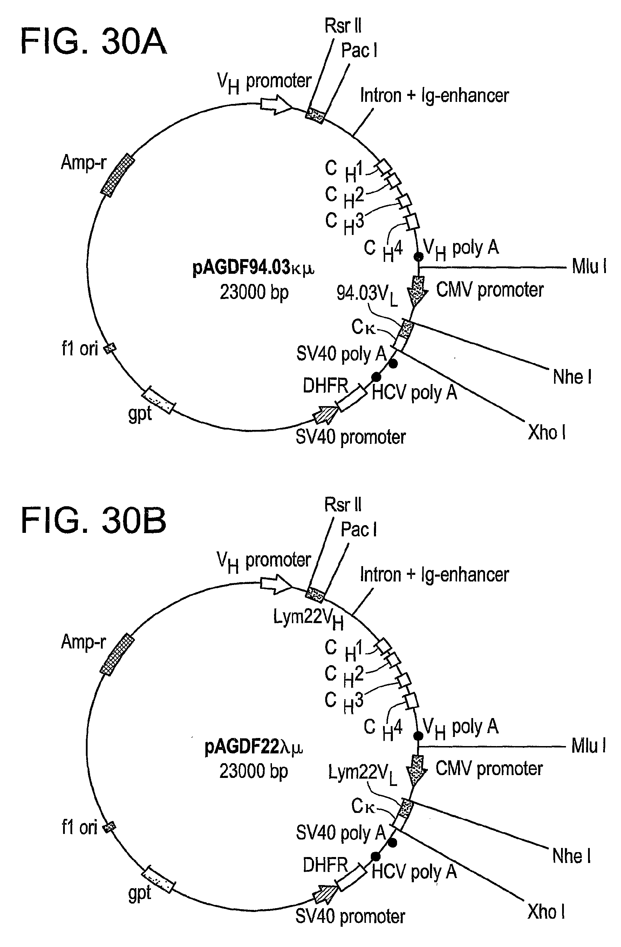

**FIGURE 29** Amplification of light chain RNA and protein expression in transfected hybridoma cells by methotrexate amplification of a dHfR-containing expression plasmid. Expression plasmid containing the coding sequence for humanized 94.03 kappa light chain under control of the CMV promoter along with a linked dHfR gene under control of the SV40 promoter was introduced by electroporation into the immunoglobulin negative F3B6 human/mouse hybridoma cell line. Cell under minimal methotrexate selection (0.5 µg/ml) and those that had undergone more stringent selection (51.2 µg/ml) were cultured to harvest supernatant to assess light chain secretion and RNA to assess light chain gene expression. Northern blot analysis indicates substantial amplification of RNA expression in one clone (#5). Protein expression was increased following methotrexate selection in clone 4, but not in clone 5. These findings indicate that methotrexate amplification sometimes results in the amplification of mRNA and protein expression by closely linked genes, but in other cases no amplification of transcription and protein synthesis is seen. **FIGURE 30** Amplifiable vectors encoding humanized 94.03 and sHIgM 22. Top panel is the prototype vector containing the coding sequence for humanized 94.03 light chain (κ) and a hybrid genomic construct encoding humanized 94.03 heavy chain (µ). Bottom panel is a similar construct that contains the coding sequences derived from the sHIgM 22 sequence.

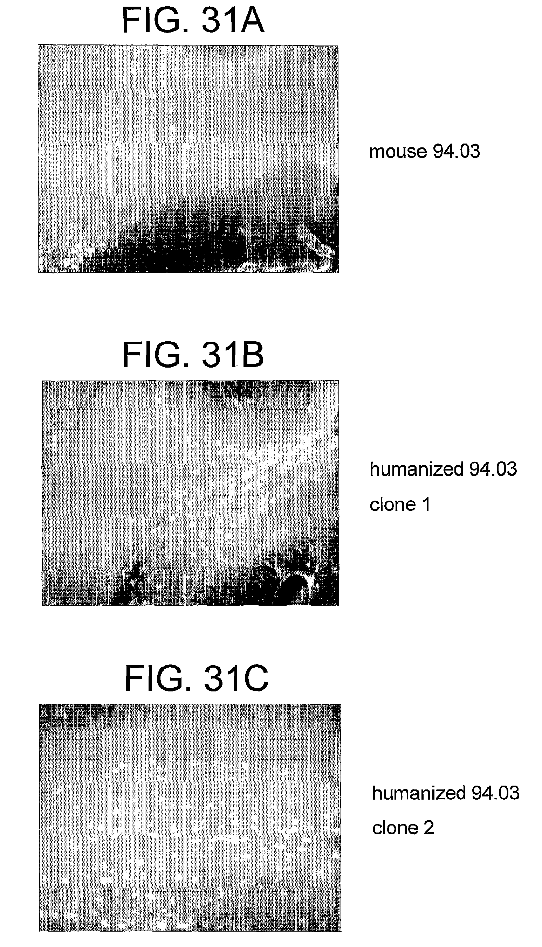

**FIGURE 31** Postnatal Rat cerebellum stained with murine and humanized 94.03. Cerebellum sections were stained with mouse and humanized 94.03. Bound antibody was localized using fluorescent secondary antibody reagent specific for mouse or human IgM, respectively. Both antibodies showed similar staining patterns to white matter tracks and astrocytes in the cerebellum.

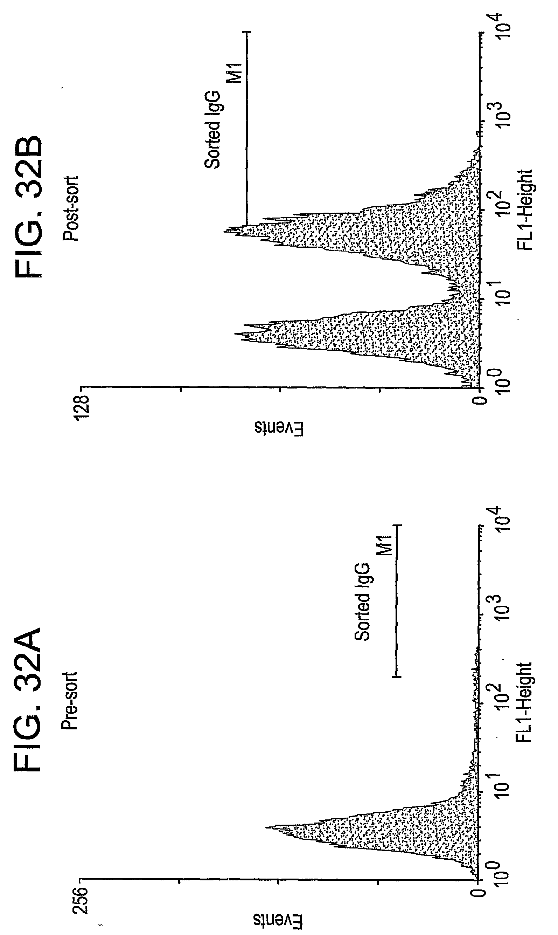

**FIGURE 32** Isolation of an IgG variant of 94.03. A natural switch variant of 94.03 was isolated from culture by sorting for cells expressing IgG on their surface. Pre-sort and post-sort profiles of the cell cultures are shown. IgG cells were isolated from the post-sort population by limiting dilution cloning. The antibody produced was identified as IgG1 using IgG isotype specific antibodies.

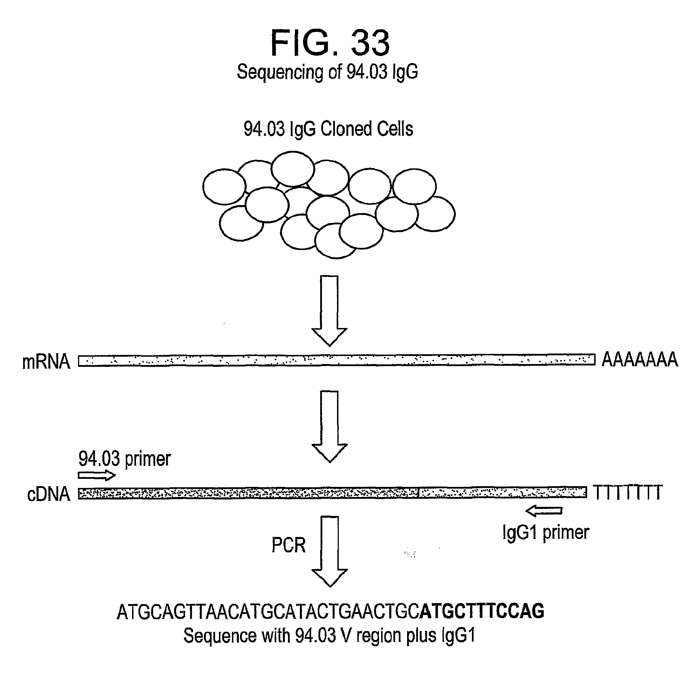

**FIGURE 33** Demonstration that the IgG1 producing cells were in fact a variant of 94.03. RNA was isolated from the clonal IgG1 expressing cells. cDNA was generated using RTPCR with primers specific for the variable region of 94.03 and the constant region of the γ 1 isotype. The resulting DNA was sequenced to demonstrate the precise splicing junction expected for a spontaneous switch variant.

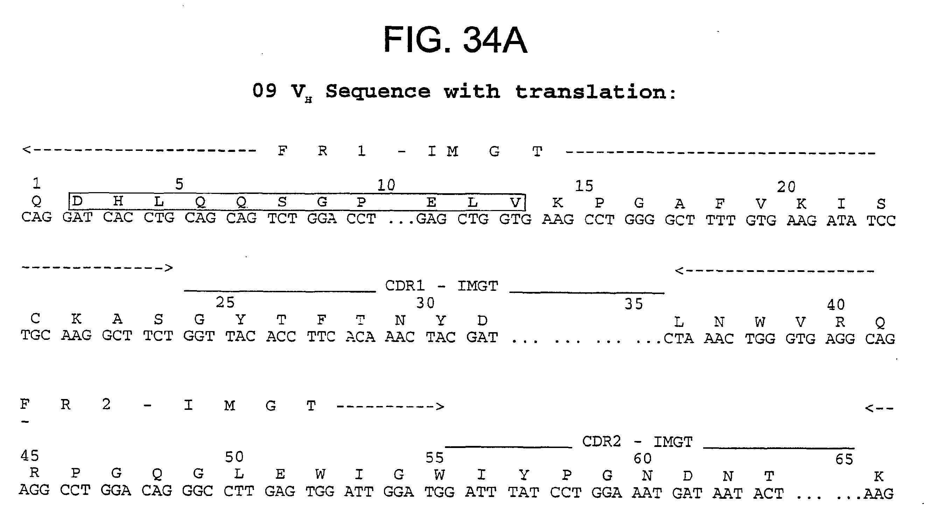

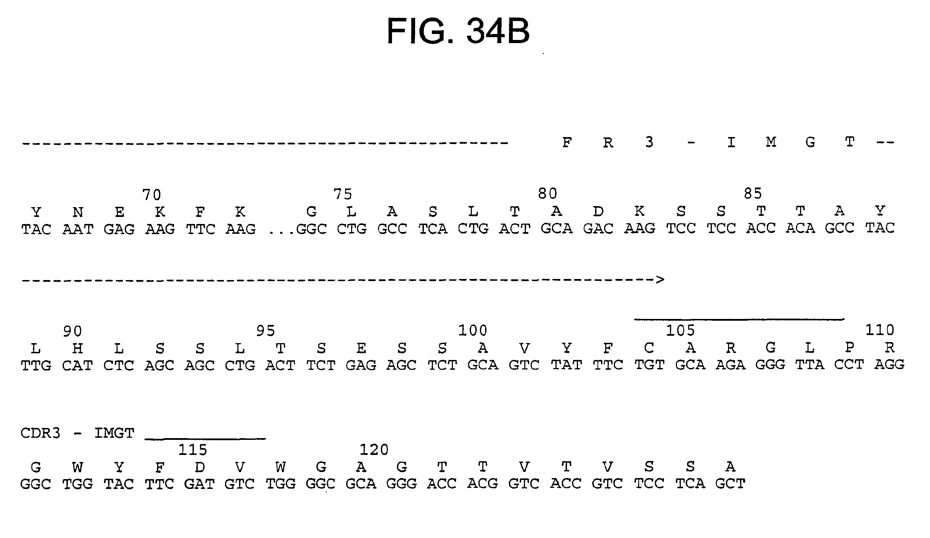

**FIGURE 34** presents the heavy chain variable region sequence of mouse 09 antibody.

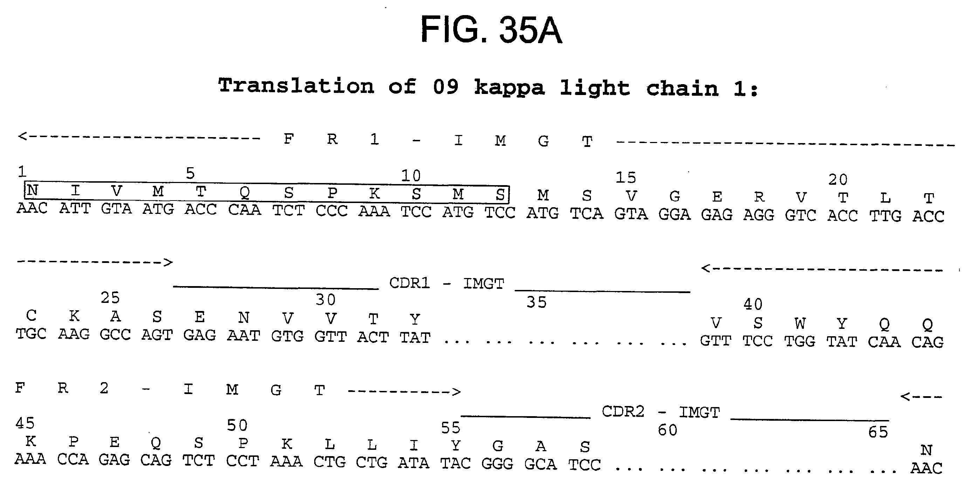

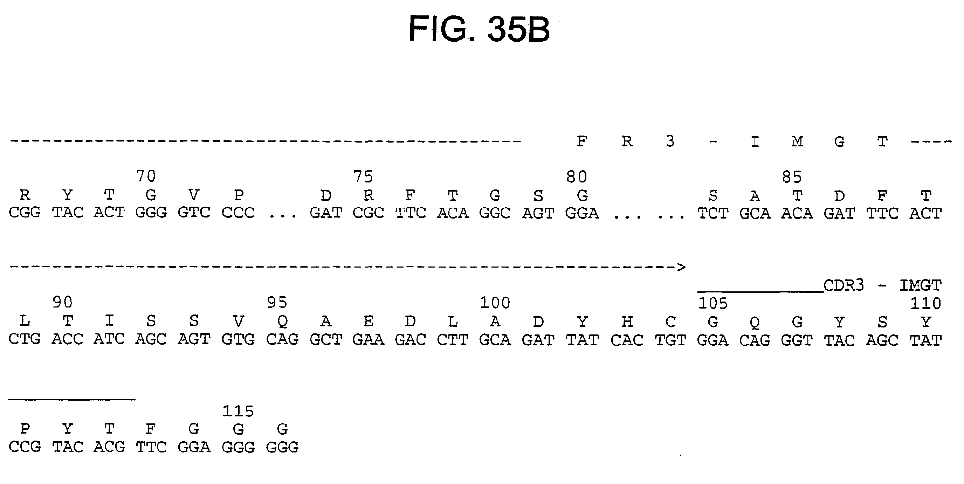

**FIGURE 35** presents the kappa light chain 1 variable region sequence of mouse 09 variable region sequence of mouse 09 antibody.

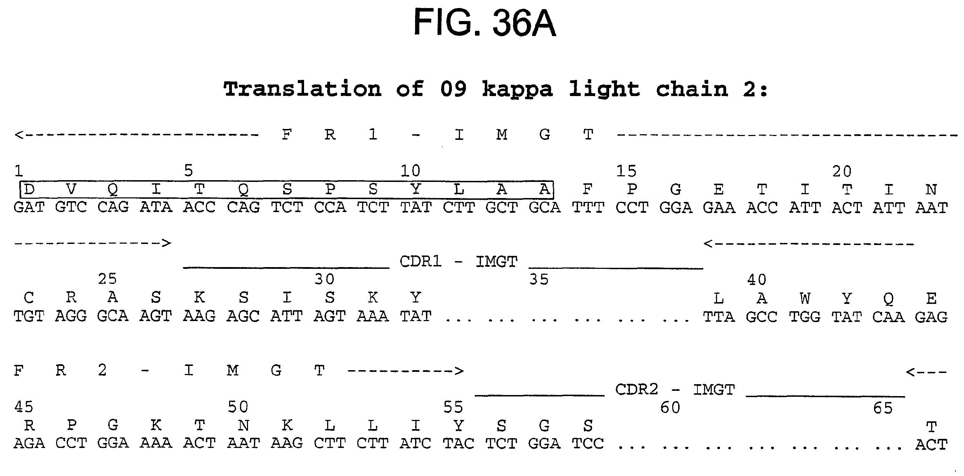

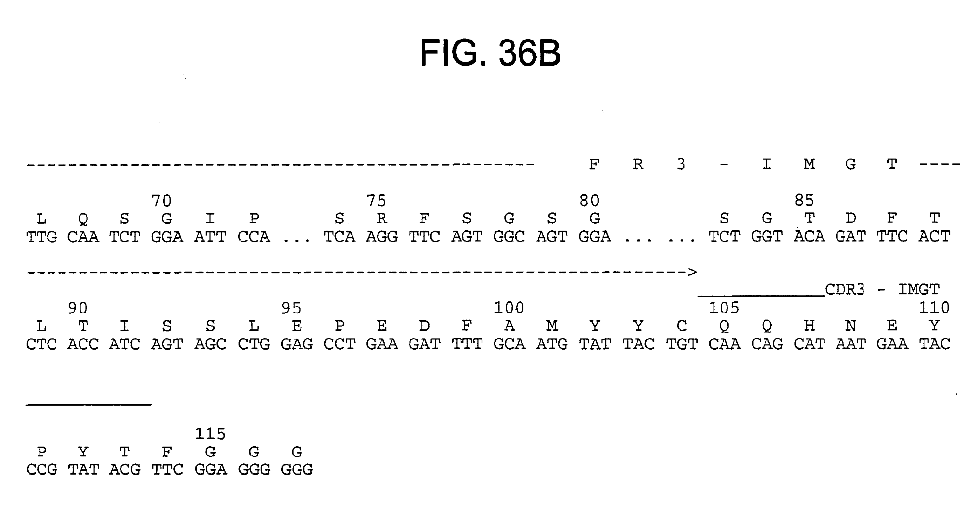

**FIGURE 36** presents the kappa light chain 2 variable region sequence of mouse 09 antibody.

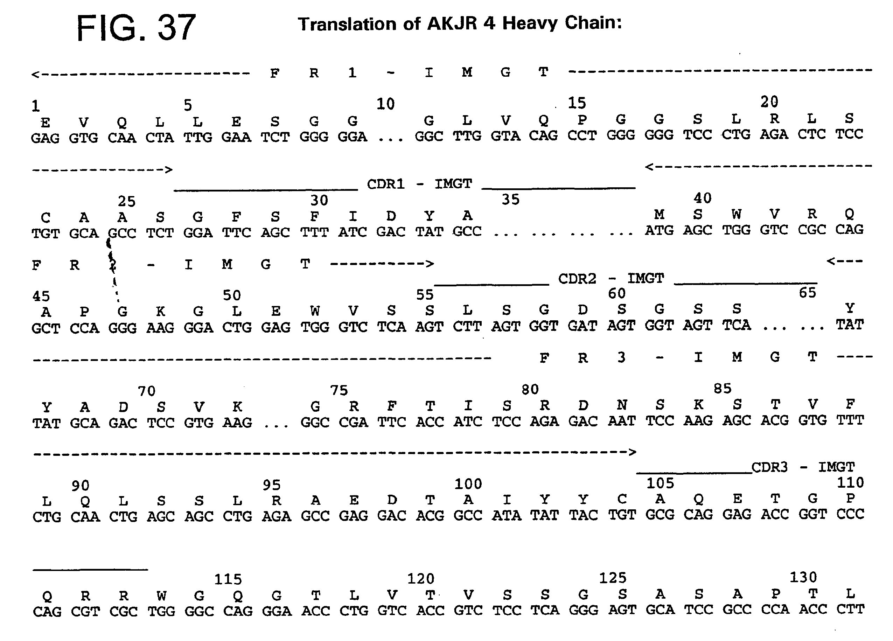

**FIGURE 37** presents the AKJR4 heavy chain variable region sequence.

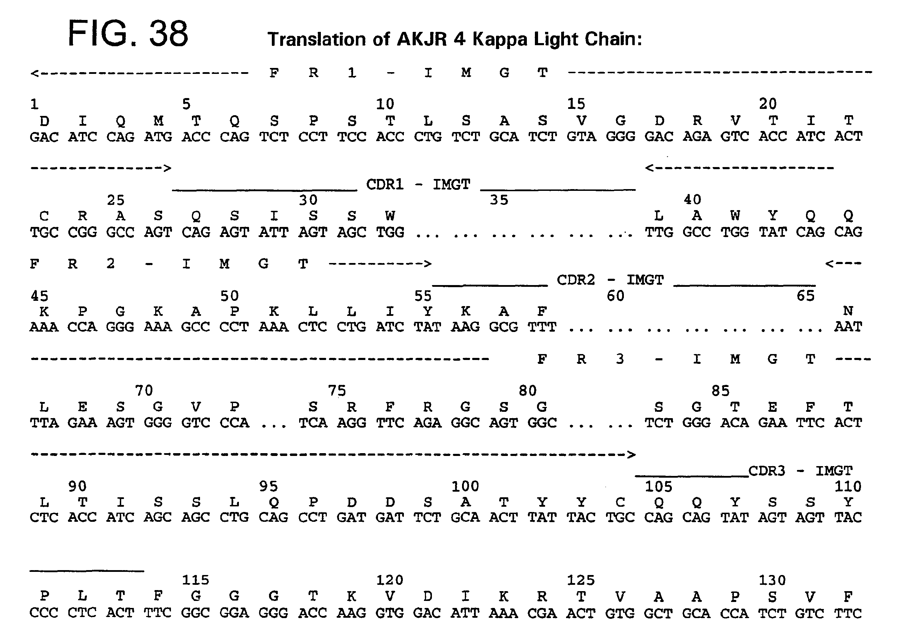

**FIGURE 38** presents the AKJR4 kappa light chain variable region sequence.
**FIGURE 39** presents the CB2iE12 heavy chain variable region sequence.
**FIGURE 40** presents the CB2iE12 kappa light chain variable region sequence.
**FIGURE 41** presents the CB2iE7 heavy chain variable region sequence.
**FIGURE 42** presents the CB2iE7 kappa light chain variable region sequence.

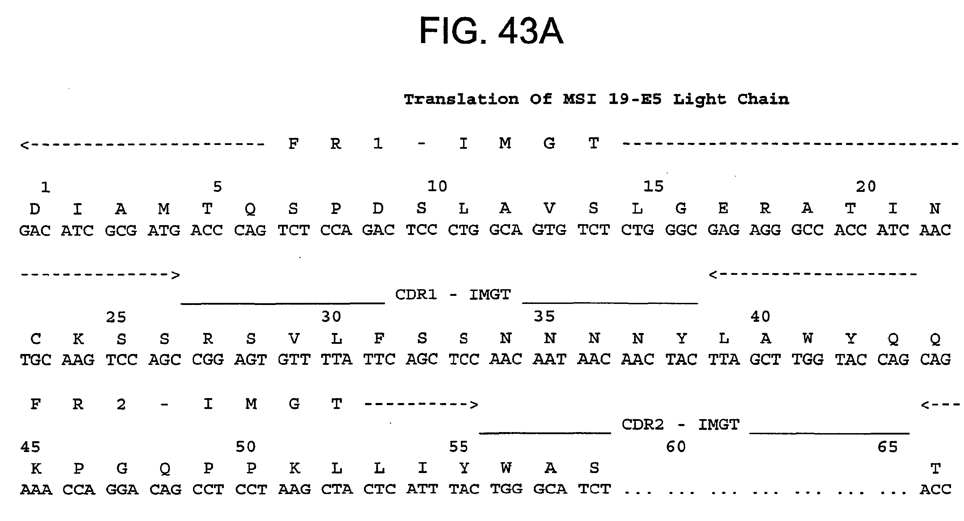

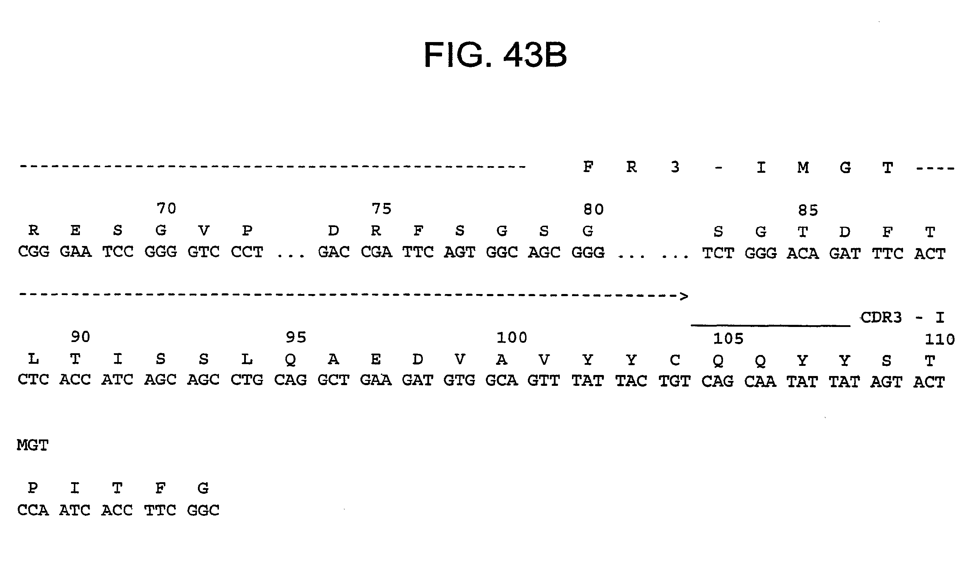

**FIGURE 43** presents the MSI 19E5 light chain variable region sequence.

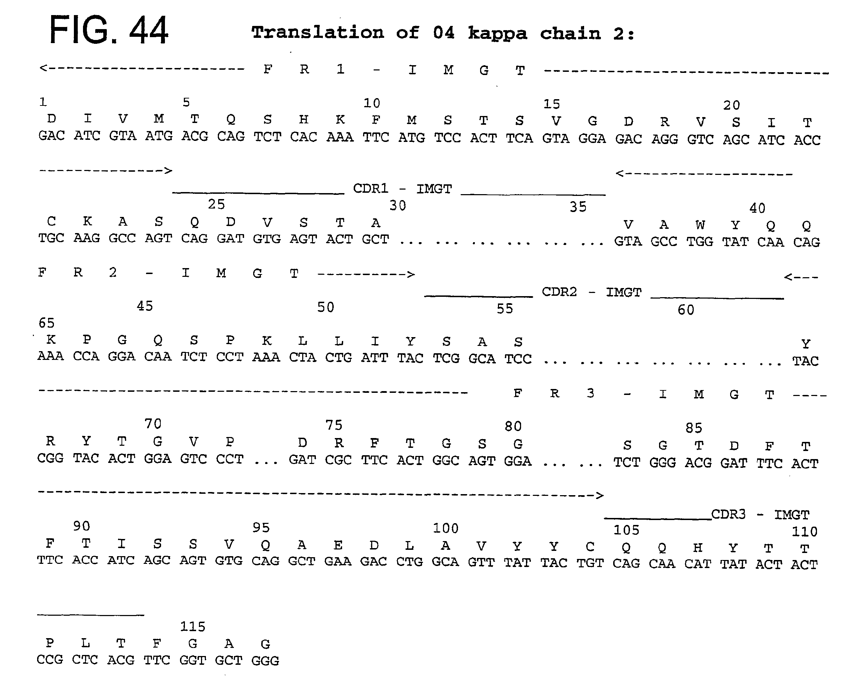

**FIGURE 44** presents the kappa light chain 2 of the mouse 04 antibody.

### DETAILED DESCRIPTION

The present invention relates to the promotion, stimulation, regeneration, protection, and/or remyelination of central nervous system axons in a mammal. Specifically, the present invention relates to autoantibodies for use in methods of stimulating the remyelination of central nervous system (CNS) axons using an autoantibody, particularly a human autoantibody, including antibodies of the IgM subtype and monomers thereof, or an active fragment thereof, characterized by the ability to bind structures and cells within the central nervous system, or a natural or synthetic analog thereof. In a particular embodiment, the antibodies provided herein and utilized in the methods of the present invention are characterized by their ability to bind to oligodendrocytes, and further, in particular, are capable of stimulating the proliferation of glial cells.

In accordance with the present invention there may be employed conventional molecular biology, microbiology, and recombinant DNA techniques within the skill of the art. Such techniques are explained fully in the literature . See, e.g., Sambrook et al, "Molecular Cloning: A Laboratory Manual" (1989); "Current Protocols in Molecular Biology" Volumes I-III [Ausubel, R. M., ed. (1994)]; "Cell Biology: A Laboratory Handbook" Volumes I-III [J. E. Celis, ed. (1994))]; "Current Protocols in Immunology" Volumes I-III [Coligan, J. E., ed. (1994)]; "Oligonucleotide Synthesis" (M.J. Gait ed. 1984); "Nucleic Acid Hybridization" [B.D. Hames & S.J. Higgins eds. (1985)]; "Transcription And Translation" [B.D. Hames & S.J. Higgins, eds. (1984)]; "Animal Cell Culture" [R.I. Freshney, ed. (1986)]; "Immobilized Cells And Enzymes" [IRL Press, (1986)]; B. Perbal, "A Practical Guide To Molecular Cloning" (1984).

Therefore, if appearing herein, the following terms shall have the definitions set out below.

The term "neuromodulatory agent(s)" as used herein singularly throughout the present application and claims, is intended to refer to a broad class of materials that function to promote neurite outgrowth, regeneration and remyelination with particular benefit and effect in the CNS, and therefore includes the antibodies of the present invention, including antibodies of the IgM subtype and monomers thereof, and particularly, the human autoantibodies provided herein, including in Tables 2 and 3 hereof, and most particularly referred to herein as CB2iE12 and CB2iE7, peptide analogs, haptens, monomers, active fragments thereof, agonists, mimics and the like, including such materials as may have at least partial similarity to the peptide sequences set forth in FIGURES 39-42. Neuromodulatory agent(s) also includes and encompasses combinations or mixtures of more than one of the antibodies provided herein, including monomers or active fragments thereof.

Also, the terms "neuromodulatory agent," "autoantibody," "antibody peptide," "peptide," "hapten" and any variants not specifically listed, may be used herein interchangeably, to the extent that they may all refer to and include proteinaceous material including single or multiple proteins, and extends to those proteins comprising the amino acid sequences provided herein and presented in FIGURES 39-42 (SEQ ID NOS: 27, 29, 31 and 33), and the profile of activities set forth herein and in the Claims. Accordingly, proteins (particularly antibodies, synthetic antibodies, monomers thereof and active fragments thereof) displaying substantially equivalent or altered activity are likewise contemplated. These modifications may be deliberate, for example, such as modifications obtained through site-directed mutagenesis, or may be accidental, such as those obtained through mutations in hosts that are producers of the complex or its named subunits. Also, the terms "neuromodulatory agent," "autoantibody," "antibody peptide," "peptide," "hapten" are intended where appropriate, to include within their scope proteins specifically recited herein as well as all substantially homologous analogs and allelic variations.

The amino acid residues described herein are preferred to be in the "L" isomeric form. However, residues in the "D" isomeric form can be substituted for any L-amino acid residue, as long as the desired fuctional property of immunoglobulin-binding is retained by the polypeptide. NH₂ refers to the free amino group present at the amino terminus of a polypeptide. COOH refers to the free carboxy group present at the carboxy terminus of a polypeptide. In keeping with standard polypeptide nomenclature, J. Biol. Chem., 243:3552-59 (1969), abbreviations for amino acid residues are shown in the following Table of Correspondence:

**TABLE OF CORRESPONDENCE**

| SYMBOL | | AMINO ACID |
|---|---|---|
| 1-Letter | 3-Letter | |
| Y | Tyr | tyrosine |
| G | Gly | glycine |
| F | Phe | phenylalanine |
| M | Met | methionine |
| A | Ala | alanine |
| S | Ser | serine |
| I | Ile | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Gln | glutamic acid |
| W | Trp | tryptophan |
| R | Arg | arginine |
| D | Asp | aspartic acid |
| N | Asn | aspargine |
| C | Cys | cysteine |

It should be noted that all amino-acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino-acid residues. The above Table is presented to correlate the three-letter and one-letter notations which may appear alternately herein.

A "replicon" is any genetic element (e.g., plasmid, chromosome, virus) that functions as an autonomous unit of DNA replication *in vivo;* i.e., capable of replication under its own control.

A "vector" is a replicon, such as plasmid, phage or cosmid, to which another DNA segment may be attached so as to bring about the replication of the attached segment.

A "DNA molecule" refers to the polymeric form of deoxyribonucleotides (adenine, guanine, thymine, or cytosine) in its either single stranded form, or a double-stranded helix. This term refers only to the primary and secondary structure of the molecule, and does not limit it to any particular tertiary forms. Thus, this term includes double-stranded DNA found, *inter alia,* in linear DNA molecules (*e.g.,* restriction fragments), viruses, plasmids, and chromosomes. In discussing the structure of particular double-stranded DNA molecules, sequences may be described herein according to the normal convention of giving only the sequence in the 5' to 3' direction along the nontranscribed strand of DNA (i.e., the strand having a sequence homologous to the mRNA).

An "origin of replication" refers to those DNA sequences that participate in DNA synthesis.

A DNA "coding sequence" is a double-stranded DNA sequence which is transcribed and translated into a polypeptide *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (*e.g*., mammalian) DNA, and even synthetic DNA sequences. A polyadenylation signal and transcription termination sequence will usually be located 3' to the coding sequence.

Transcriptional and translational control sequences are DNA regulatory sequences, such as promoters, enhancers, polyadenylation signals, terminators, and the like, that provide for the expression of a coding sequence in a host cell.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined by mapping with nuclease S1), as well as protein binding domains (consensus sequences) responsible for the binding of RNA polymerase. Eukaryotic promoters will often, but not always, contain "TATA" boxes and "CAT" boxes. Prokaryotic promoters contain Shine-Dalgarno sequences in addition to the -10 and -35 consensus sequences.

An "expression control sequence" is a DNA sequence that controls and regulates the transcription and translation of another DNA sequence. A coding sequence is "under the control" of transcriptional and translational control sequences in a cell when RNA polymerase transcribes the coding sequence into mRNA, which is then translated into the protein encoded by the coding sequence.

A "signal sequence" can be included before the coding sequence. This sequence encodes a signal peptide, N-terminal to the polypeptide, that communicates to the host cell to direct the polypeptide to the cell surface or secrete the polypeptide into the media, and this signal peptide is clipped off by the host cell before the protein leaves the cell. Signal sequences can be found associated with a variety of proteins native to prokaryotes and eukaryotes.

The term "oligonucleotide, as used herein in referring to probes of the present invention, is defined as a molecule comprised of two or more ribonucleotides, preferably more than three. Its exact size will depend upon many factors which, in turn, depend upon the ultimate function and use of the oligonucleotide.

The term "primer" as used herein refers to an oligonucleotide, whether occurring naturally as in a purified restriction digest or produced synthetically, which is capable of acting as a point of initiation of synthesis when placed under conditions in which synthesis of a primer extension product, which is complementary to a nucleic acid strand, is induced, *i.e.*, in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and use of the method. For example, for diagnostic applications, depending on the complexity of the target sequence, the oligonucleotide primer typically contains 15-25 or more nucleotides, although it may contain fewer nucleotides.

The primers herein are selected to be "substantially" complementary to different strands of a particular target DNA sequence. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence of the strand to hybridize therewith and thereby form the template for the synthesis of the extension product.

As used herein, the terms "restriction endonucleases" and "restriction enzymes" refer to bacterial enzymes, each of which cut double-stranded DNA at or near a specific nucleotide sequence.

A cell has been "transformed" by exogenous or heterologous DNA when such DNA has been introduced inside the cell. The transforming DNA may or may not be integrated (covalently linked) into chromosomal DNA making up the genome of the cell. In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or common ancestor by mitosis. A "cell line" is a clone of a primary cell that is capable of stable growth *in vitro* for many generations.

Two DNA sequences are "substantially homologous" when at least about 75% (preferably at least about 80 %, and most preferably at least about 90 or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, e.g., Maniatis et al., *supra;* DNA Cloning, Vols. I & II, *supra;* Nucleic Acid Hybridization, *supra.* In particular, the heavy chain and light chain variable region sequences of the antibodies of the present invention are substantially homologous to a corresponding germline gene sequence, having at least about 90% homology to a corresponding germline gene sequence.

It should be appreciated that also within the scope of the present invention are DNA sequences encoding an antibody of the invention, or a peptide analog, hapten, or active fragment thereof, which code for a peptide that defines in at least a portion thereof, or has the same amino acid sequence as set forth in FIGURES 39-42 (SEQ ID NOS: 27, 29, 31 and 33), but which are degenerate to the same SEQ ID NOS. By "degenerate to" is meant that a different three-letter codon is used to specify a particular amino acid. It is well known in the art that the following codons can be used interchangeably to code for each specific amino acid:

| | |
|---|---|
| Phenylalanine (Phe or F) | UUU or UUC |
| Leucine (Leu or L) | UUA or UUG or CUU or CUC or CUA or CUG |
| Isoleucine (IIe or I) | AUU or AUC or AUA |
| Methionine (Met or M) | AUG |
| Valine (Val or V) | GUU or GUC of GUA or GUG |
| Serine (Ser or S) | UCU or UCC or UCA or UCG or AGU or AGC |
| Proline (Pro or P) | CCU or CCC or CCA or CCG |
| Threonine (Thr or T) | ACU or ACC or ACA or ACG |
| Alanine (Ala or A) | GCU or GCG or GCA or GCG |
| Tyrosine (Tyr or Y) | UAU or UAC |
| Histidine (His or H) | CAU or CAC |
| Glutamine (Gin or Q) | CAA or CAG |
| Asparagine (Asn or N) | AAU or AAC |
| Lysine (Lys or K) | AAA or AAG |
| Aspartic Acid (Asp or D) | GAU or GAC |
| Glutamic Acid (Glu or E) | GAA or GAG |
| Cysteine (Cys or C) | UGU or UGC |
| Arginine (Arg or R) | CGU or CGC or CGA or CGG or AGA or AGG |
| Glycine (Gly or G) | GGU or GGC or GGA or GGG |
| Tryptophan (Trp or W) | UGG |
| Termination codon | UAA (ochre) or UAG (amber) or UGA (opal) |

It should be understood that the codons specified above are for RNA sequences. The corresponding codons for DNA have a T substituted for U.

Mutations can be made in a particular DNA sequence or molecule such that a particular codon is changed to a codon which codes for a different amino acid. Such a mutation is generally made by making the fewest nucleotide changes possible. A substitution mutation of this sort can be made to change an amino acid in the resulting protein in a non-conservative manner (i.e., by changing the codon from an amino acid belonging to a grouping of amino acids having a particular size or characteristic to an amino acid belonging to another grouping) or in a conservative manner (i.e., by changing the codon from an amino acid belonging to a grouping of amino acids having a particular size or characteristic to an amino acid belonging to the same grouping). Such a conservative change generally leads to less change in the structure and function of the resulting protein. A non-conservative change is more likely to alter the structure, activity or function of the resulting protein. The present invention should be considered to include seguences containing conservative changes which do not significantly alter the activity or binding characteristics of the resulting protein.

The following is one example of various groupings of amino acids:

### Amino acids with nonpolar R groups

Alanine
Valine
Leucine
Isoleucine
Proline
Phenylalanine
Tryptophan
Methionine

### Amino acids with uncharged polar R groups

Glycine
Serine
Threonine
Cysteine
Tyrosine
Asparagine
Glutamine

### Amino acids with charged polar R groups (negatively charged at Ph 6.0)

Aspartic acid
Glutamic acid

### Basic amino acids (positively charged at pH 6.0)

Lysine
Arginine
Histidine (at pH 6.0)

Another grouping may be those amino acids with phenyl groups:
Phenylalanine
Tryptophan
Tyrosine

Another grouping may be according to molecular weight (i.e., size of R groups):

| | |
|---|---|
| Glycine | 75 |
| Alanine | 89 |
| Serine | 105 |
| Proline | 115 |
| Valine | 117 |
| Threonine | 119 |
| Cysteine | 121 |
| Leucine | 131 |
| Isoleucine | 131 |
| Asparagine | 132 |
| Aspartic acid | 133 |
| Glutamine | 146 |
| Lysine | 146 |
| Glutamic acid | 147 |
| Methionine | 149 |
| Histidine (at pH 6.0) | 155 |
| Phenylalanine | 165 |
| Arginine | 174 |
| Tyrosine | 181 |
| Tryptophan | 204 |

Particularly preferred substitutions are:
- Lys for Arg and vice versa such that a positive charge may be maintained;
- Glu for Asp and vice versa such that a negative charge may be maintained;
- Ser for Thr such that a free -OH can be maintained; and
- Gln for Asn such that a free NH₂ can be maintained.

Amino acid substitutions may also be introduced to substitute an amino acid with a particularly preferable property. For example, a Cys may be introduced a potential site for disulfide bridges with another Cys. A His may be introduced as a particularly "catalytic" site (i.e., His can act as an acid or base and is the most common amino acid in biochemical catalysis). Pro may be introduced because of its particularly planar structure, which induces β-turns in the protein's structure.

Two amino acid sequences are "substantially homologous" when at least about 70% of the amino acid residues (preferably at least about 80%, and most preferably at least about 90 or 95%) are identical, or represent conservative substitutions. In particular, the heavy chain and light chain variable region sequences of the antibodies of the present invention are substantially homologous to a corresponding germline gene amino acid sequence, having at least about 90%, and preferably at least about 95 % homology to a corresponding germline gene amino acid sequence.

A "heterologous" region of the DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous coding sequence is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein.

As used herein, the term "antibody" is any immunoglobulin, including antibodies and fragments thereof, that binds a specific epitope. The term is intended to encompass polyclonal, monoclonal, and chimeric antibodies, the last mentioned described in further detail in U.S. Patent Nos. 4,816,397 and 4,816,567. Such antibodies include both polyclonal and monoclonal antibodies prepared by known generic techniques, as well as bi-specific (chimeric) antibodies, and antibodies including other functionalities suiting them for additional diagnostic use conjunctive with their capability of modulating activity, *e.g.* that stimulates the remyelenation and/or regeneration of CNS axons, or that provides neuroprotection. An "antibody combining site" is that structural portion of an antibody molecule comprised of heavy and light chain variable and hypervariable regions that specifically binds antigen. The phrase "antibody molecule" in its various grammatical forms as used herein contemplates both an intact immunoglobulin molecule and an immunologically active portion of an immunoglobulin molecule. Exemplary antibody molecules are intact immunoglobulin molecules, substantially intact immunoglobulin molecules and those portions of an immunoglobulin molecule that contains the paratope, including those portions known in the art as Fab, Fab', F(ab')₂ and F(v).

Fab and F(ab')₂ portions of antibody molecules can be prepared by the proteolytic reaction of papain and pepsin, respectively, on substantially intact antibody molecules by methods that are well-known. See for example, U.S. Patent No. 4,342,566 to Theofilopolous et al. Fab' antibody molecule portions are also well-known and can be produced from F(ab')₂ portions followed by reduction of the disulfide bonds linking the two heavy chains portions as with mercaptoethanol, and followed by alkylation of the resulting protein mercaptan with a reagent such as iodoacetamide.

The phrase "monoclonal antibody" in its various grammatical forms refers to an antibody having only one species of antibody combining site capable of immunoreacting with a particular antigen. A monoclonal antibody thus typically displays a single binding affinity for any antigen with which it immunoreacts. A monoclonal antibody may therefore contain an antibody molecule having a plurality of antibody combining sites, each immunospecific for a different antigen; *e.g.,* a bi-specific (chimeric) monoclonal antibody.

The general methodology for making monoclonal antibodies by hybridomas is well known. Immortal, antibody-producing cell lines can also be created by techniques other than fusion, such as direct transformation of B lymphocytes with oncogenic DNA, or transfection with Epstein-Barr virus. See, e.g., M. Schreier et al., "Hybridoma Techniques" (1980); Hammerling et al., "Monoclonal Antibodies And T-cell Hybridomas" (1981); Kennett et al., "Monoclonal Antibodies" (1980); see also U.S. Patent Nos. 4,341,761; 4,399,121; 4,427,783; 4,444,887; 4,451,570; 4,466,917; 4,472,500; 4,491,632; 4,493,890.

Panels of monoclonal antibodies produced against neuromodulatory agent peptides or autoantibody peptides can be screened for various properties; i.e., isotype, epitope, affinity, etc. Of particular interest are monoclonal antibodies, capable of binding to structures and cells in the central nervous system and that exhibit the same activity as the neuromodulatory agents, and particularly the present autoantibodies. Such antibodies can be readily screened and characterized in assays, including binding, staining and immunocytochemistry methods presented and illustrated herein. Such monoclonals can be readily identified in activity assays such as the Theilers virus, EAE and lysolecithin models presented and illustrated herein. High affinity antibodies are also useful when immunoaffinity purification of native or recombinant autoantibodies is possible.

Preferably, the anti-peptide antibody used in the therapeutic and diagnostic methods of this invention is a monoclonal antibody (mAb), most preferably a human or humanized antibody. The antibody is also preferably a synthetic antibody. In addition, it is preferable for the anti-peptide antibody molecules used herein be in the form of Fab, Fab', F(ab')₂ or F(v) portions of whole antibody molecules.

As suggested earlier, the diagnostic method of the present invention comprises examining a cellular sample or medium by means of an assay including an effective amount of an antagonist to an antibody peptide/protein, such as an anti-peptide antibody, preferably an affmity-purified polyclonal antibody, and more preferably a mAb. In addition, it is preferable for the anti-peptide antibody molecules used herein be in the form of Fab, Fab', F(ab')₂ or F(v) portions or whole antibody molecules. As previously discussed, patients capable of benefiting from this method include those suffering from a neurological condition such as multiple sclerosis, Alzheimers disease, Parkinsons disease, a viral infection or other like neuropathological derangement, including damage resulting from physical trauma. Methods for isolating the peptides and inducing anti-peptide antibodies and for determining and optimizing the ability of anti-peptide antibodies to assist in the examination of the target cells are all well-known in the art.

Methods for producing polyclonal anti-polypeptide antibodies are well-known in the art. See U.S. Patent No. 4,493,795 to Nestor et al. A monoclonal antibody, typically containing Fab and/or F(ab')₂portions of useful antibody molecules, can be prepared using the hybridoma technology described in Antibodies - A Laboratory Manual, Harlow and Lane, eds., Cold Spring Harbor Laboratory, New York (1988), which is incorporated herein by reference. Briefly, to form the hybridoma from which the monoclonal antibody composition is produced, a myeloma or other self-perpetuating cell line is fused with lymphocytes obtained from the spleen of a mammal hyperimmunized with an antibody peptide-binding portion thereof, or the antibody peptide or fragment, or an origin-specific DNA-binding portion thereof.

Splenocytes are typically fused with myeloma cells using polyethylene glycol (PEG) 6000. Fused hybrids are selected by their sensitivity to HAT. Hybridomas producing a monoclonal antibody useful in practicing this invention are identified by their ability to immunoreact in the same fashion as the present autoantibodies and their ability to inhibit or promote specified activity in target cells and tissues.

A monoclonal antibody useful in practicing the present invention can be produced by initiating a monoclonal hybridoma culture comprising a nutrient medium containing a hybridoma that secretes antibody molecules of the appropriate antigen specificity. The culture is maintained under conditions and for a time period sufficient for the hybridoma to secrete the antibody molecules into the medium. The antibody-containing medium is then collected. The antibody molecules can then be further isolated by well-known techniques.

Media useful for the preparation of these compositions are both well-known in the art and commercially available and include synthetic culture media, inbred mice and the like. An exemplary synthetic medium is Dulbecco's minimal essential medium (DMEM; Dulbecco et al., Virol. 8:396 (1959)) supplemented with 4.5 gm/l glucose, 20 mm glutamine, and 20% fetal calf serum. An exemplary inbred mouse strain is the Balb/c.

Methods for producing monoclonal anti-peptide antibodies are also well-known in the art. See Niman et al., Proc. Natl. Acad. Sci. USA, 80:4949-4953 (1983). Typically, the present antibody peptides, or a peptide analog or fragment, is used either alone or conjugated to an immunogenic carrier, as the immunogen in the before described procedure for producing anti-peptide monoclonal antibodies. The hybridomas are screened for the ability to produce an antibody that immunoreacts with the antibody peptide analog and thereby reacts similarly to the antibodies of the present invention.

The present invention also relates to autoantibodies for use in methods of treating demyelinating diseases in mammals, such as multiple sclerosis in humans, and viral diseases of the central nervous system of humans and domestic animals, such as post-infectious encephalomyelitis, as exemplified by the human antibodies CB2iE12 and CB2iE7, monomers thereof, analogs thereof including haptens, active fragments thereof, or a isolated or synthetic autoantibody having the characteristics thereof. Methods of prophylactic treatment using these mAb, monomers thereof, active fragments thereof, or other isolated or synthetic autoantibodies having the same characteristics, including to inhibit the initiation or progression demyelinating diseases are also encompassed by this invention.

Oligodendrocytes (OLs), the myelin-forming cells of the central nervous system (CNS), originate as neuroectodermal cells of the subventricular zones, and then migrate and mature to produce myelin. The sequential development of OLs is identified by well-characterized differentiation stage-specific markers. Proliferative and migratory bipolar precursors, designated oligodendrocyte/type-3 astrocyte (O-2A) progenitors, are identified by monoclonal antibodies (mAbs) anti-GD₃ and A2B5 [Eisenbarth et al., Proc. Natl. Acad. Sci. USA, 76 (1979), 4913-4917]. The next developmental stage, characterized by multipolar, postmigratory, and proliferative cells, is recognized by mAb 04 [Gard et al., Neuron, 5 (1990), 615-625; Sommer et al., Dev. Biol., 83 (1981), 311-327]. Further development is defined by the cell surface expression of galactocerebroside, recognized by mAb O1 [Schachner, J. Neurochem., 39 (1982), 1-8; Sommer et al., *supra*], and by the expression of 2',3'-cyclic nucleotide 3'-phosphohydrolase. The most mature cells express terminal differentiation markers such as myelin basic protein and proteolipid protein.

The mAbs (A2B5, O1, and 04) used to characterize the stages of OL development were made by immunizing BALB/c mice with chicken embryo retina cells or homogenate of bovine corpus callosum [Eisenbarth et al., *supra*; Sommer et al., *supra*]. A2B5 recognizes not only O-2A progenitors but also neurons and reacts with cell surface ganglioside GQ1c [Kasai et al., Brain Res., 277 (1983), 155-158] and other gangliosides [Fredman et al., Arch. Biochem. Biophys., 233 (1984), 661-666]. 04 reacts with sulfatide, seminolipid and cholesterol [Bansal et al., J. Neurosci. Res., 24 (1989), 548-557], whereas O1 reacts with galactocerebroside, monogalactosyl-diglyceride and psychosine [Bansal et al., *supra*]. These mAbs belong to the IgM immunoglobulin (Ig) subclass and recognize cytoplasmic structures as well as the surface antigens of OLs [Eisenbarth et al., *supra*; Sommer et al., *supra*]. Mouse mAb HNK-1 (anti-Leu-7), made by immunizing BALB/c mice with the membrane suspension of HSB-2 T lymphoblastoid cells, was first reported as a marker for natural killer cells [Abo et al., J. Immunol., 127 (1981), 1024-1029]. Later, HNK-1 was shown to share antigenic determinants with the nervous system [Schuller-Petrovic et al., Nature, 306 (1983), 179-181]. The carbohydrate epitope on myelin-associated glycoprotein, found in both central and peripheral myelin sheaths, was shown to be a principal antigen of nervous tissue the reacted with HNK-1 [McGarry et al., Nature, 306 (1983), 376-378]. However, other glycoproteins in nervous tissue react with this mAb, some of which are important in embryogenesis, differentiation, and myelination [Keilhauer et al., Nature, 316 (1985), 728-730; Kruse et al., Nature, 311 (1984), 153-155; Kruse et al., Nature, 316 (1985), 146-148; McGarry et al., J. Neuroimmunol., 10 (1985), 101-114]. Of interest, HNK-1 also reacts with cytoplasmic structures and belongs to the IgM Ig subclass.

A monoclonal antibody, disclosed and claimed in U.S. Patent Number 5,591,629, issued January 7, 1997, and designated SCH94.03, was found to promote CNS remyelination in mice infected chronically with Theiler's murine encephalomyelitis virus (TMEV) [Miller et al., J. Neurosci., 14 (1994), 6230-6238]. SCH94.03 belongs to the IgM(x) Ig subclass and recognizes an unknown surface antigen on OLs, but cytoplasmic antigens in all cells (Asakura et al., Molecular Brain Research, in press). The polyreactivity of SCH94.03 by ELISA, and the unmutated Ig variable region germline sequences indicated that SCH94.03 is a natural autoantibody [Miller et al., J. Neurosci., 14 (1994), 6230-6238]. A close study of SCH94.03, and comparison thereof with well-known OL-reactive mAbs A2B5, O1 04, and HNK-1 raised the possibility that these are natural autoantibodies. A subsequent analysis of the Ig variable region cDNA sequences and the polyreactivity of these mAbs by ELISA confirmed that this is a generic group of natural autoantibodies having similar utilities.

The antigen reactivity of the monoclonal antibody, IgM monoclonal antibody SCH 94.03 (also referred to as SCH94.32) and SCH 79.08 (both prepared from a mammal immunized with spinal cord homogenate from a normal mammal (*i.e.,* uninfected with any demyelinating disease)), have been characterized and described in the aforesaid U.S. Patent Number 5,591,629, issued January 7, 1997, using several biochemical and molecular assays, including immunohistochemistry, immunocytochemistry, Western blotting, solid-phase enzyme-linked immunosorbant assays (ELISA), and Ig variable region sequencing.

Natural or physiologic autoantibodies are present normally in serum, are characterized by being reactive or capable of binding to self structures, antigens or cells. They are often polyreactive, are frequently of the IgM subtype, and are encoded by unmutated germline genes or are substantially homologous to germline genes with few sequence differences. By sequencing immunoglobulin (Ig) cDNAs of the oligodendrocyte-reactive 01, 04, A2B5, and HNK-1 IgM K monoclonal antibodies and comparing these with published germline sequences, it was determined that these were natural autoantibodies. O1 V_{H} was identical with unrearranged V_{H} segment transcript A1 and A4, 04 V_{H} had three and HNK-1 V_{H} had six nucleotide differences from germline V_{H}101 in the V_{H} coding region. The D segment of O1 was derived from germline SP2 gene family, J_{H}4, whereas O1 J_{H} was encoded by germline J_{H}1 with one silent nucleotide change. O1 and 04 light chains were identical with myeloma MOPC21 except for one silent nucleotide change. HNK-1 V_{K} was identical with germline V_{K}41 except for two silent nucleotide changes. O1 J_{K}, 04J_{K} and HNK J_{K} were encoded by unmutated germline J_{K}2. In contrast, A2B5 V_{H} showed seven nucleotide differences from germline V1, whereas no germline sequence encoding A2B5 V_{K} was identified. O1 and 04, but not A2B5 were polyreactive against multiple antigens by direct ELISA. Therefore, O1, 04 and HNK-1 Igs are encoded by germline genes, and have the genotype and phenotype of natural autoantibodies.

The identification and characterization of an entire family of autoantibodies, referred to as "natural" or "physiological" autoantibodies, has influenced traditional view of autoimmunity and self-reactivity. The autoantibodies that have been studied extensively are typically IgMs, although other isotypes have been identified, are reactive toward a wide range of self structures or antigens, including cytoskeletal proteins, surface proteins, nucleic acids, phospholipids, bacterial antigens such as lipopolysaccharides, and various chemical haptens (reviewed by Avrameas and Ternynck, Mol. Immunol., 30:1133-1142 (1993)). Natural autoantibodies share extensive idiotypic cross-reactivity or "connectivity", which includes expression of similar idiotypes, some of which are expressed by pathogenic autoantibodies, as well as reactivity toward common idiotypes expressed on other antibodies. Molecular analysis has shown that natural autoantibodies are typically encoded by unmutated germline immunoglobulin (Ig) genes, or substantially homologous thereto, with few somatic mutations, and therefore represent a substantial fraction of the Ig repertoire, especially in neonatal animals which have not had extensive exogenous antigen exposure.

The function of natural autoantibodies remains enigmatic. Several hypotheses have been proposed based upon their biochemical and molecular characteristics. These include: (1) clearance of senescent or damage tissue, (2) providing a first line of immunological defense in the lag period between pathogen exposure and an Ag-specific immune response, (3) masking autoantigens from a potentially pathogenic autoimmune response, (4) immunomodulation, including shaping of the neonatal immune repertoire via an idiotypic network, and (5) participation in the positive selection of B cells in the bone marrow, similar to the process proposed for T cells in the thymus.

That certain autoantibodies, broadly characterized as antibodies recognizing self antigens and including natural autoantibodies, are capable of stimulating remyelination in the central nervous system suggests an important physiological function of autoantibodies. Autoantibodies that are produced either during normal physiology, or in response to tissue damage and the subsequent release of previously sequestered antigens, might actively participate to promote repair in the damaged tissue. In line with previously proposed functions of autoantibodies, this active participation might be to facilitate removal of damaged tissue, mask autoantigens thereby preventing vigorous pathogenic autoimmune response, modulate the immune response which actually resulted in the tissue destruction, thereby allowing normal endogenous tissue repair to occur, or directly stimulate cells involved in the repair process.

The results and Examples provided herein now demonstrate the isolation of human autoantibodies, generated and screened for their autoantigen-binding capability, particularly capable of binding structures and cells in the central nervous system. The ability of these antibodies to promote CNS remyelination is also demonstrated. Mice chronically infected with TMEV and treated with IgM mAbs from human hybridomas, particularly exemplified by sHIgM22 (LIM 22), sHIgM46, ebvHIgM MSI19D10, CB2bG8, had significantly more CNS repair than control animals, measured by a detailed quantitative morphological assessment of CNS remyelination.

### Treatment of Demyelinating Diseases

The results of the experiments described herein have practical applications to multiple sclerosis (MS), EAE, and other related central nervous system demyelinating disorders. Rare examples of spontaneous CNS-type remyelination ("shadow plaques") are found in MS and occasional peripheral nervous system (PNS)-type remyelination is found in demyelinated spinal cord plaques near the root entry zone. Oligodendrocytes are infrequent at the center of the chronic plaques in MS but they appear to proliferate at the periphery of plaques, where they are associated with abortive remyelination. The process of remyelination may correlate with the spontaneous remission and improvements observed clinically in MS. These clinical observations indicate that new myelin formation is possible in MS. The remyelination that has been stimulated in mice with TMEV-induced demyelination by using a mAb holds promise for therapeutic applications in multiple sclerosis.

Of importance clinically is the question of whether morphologic regeneration of thin myelin sheaths contributes to functional recovery. Computer simulations indicate that new myelin formation even by inappropriately thin sheaths improves impulse conduction. Since the axon membrane of normally myelinated fibers is highly differentiated, it is necessary for sodium channels to be present at high density at the node of Ranvier to propagate saltatory conduction. Experimental evidence suggests that newly formed nodes do develop the required high sodium channel density as demonstrated by saxitoxin binding. Data to date suggest that remyelination even by inappropriately thin myelin improves conduction in a previously demyelinated axon. Therefore, any strategy to promote this morphologic phenomenon has the potential of producing functional recovery.

The isolation and testing of human autoantibodies as set forth herein provides human antibodies particularly suitable and desirable for use in humans. Importantly, the use of human antibodies avoids the potential for human immune response against the therapeutic antibody. Therapeutic antibodies derived from non-human animals have been shown to generate an immune response, which can be significant and detrimental to the individual. Polyclonal human IgM and polyclonal human IgG have been tested in two models of *in vivo* spinal cord demyelination; a chronic viral infection model, and an acute toxicity model. In both models polyclonal human IgM treated animals had a significantly higher density of newly myelinated axons than animals treated with polyclonal human IgG. A panel of human monoclonal IgM antibodies have also been identified, based on their reactivity with surface antigens specific to the central nervous system. These human antibodies promote significantly more central nervous system remyelination than polyclonal human IgG when given to mammals with demyelinating disease. The human monoclonal antibodies are antigenically polyreactive and recognize determinants on the surface of oligodendrocytes and specific populations of neurons. The light and heavy chain variable regions of one human antibody that promotes remyelination has been partially sequenced. This antibody can induce calcium fluxes in oligodendrocytes in culture, suggestive of direct binding and signaling through glial cells. These human antibodies bind to human white matter and may be effective in promoting remyelination in humans. The benefits of a monoclonal antibody for use as a therapeutic agent are 1) the antibody can be grown free of possible host infection and, 2) the antibody can be genetically altered *in vitro* to change its effectiveness.

Thus, as a result of the experiments described herein, the autoantibodies of the present invention can be used in methods to treat mammals, including humans and domestic animals, afflicted with demyelinating disorders, and to stimulate remyelination and regeneration of the CNS axons, as well as to offer neuroprotection. As described herein, an effective amount of the monoclonal antibody or a peptide fragment, hapten, or equivalent, can be administered by conventional routes of administration, and particularly by, intravenous (iv) or intraperitoneal (ip) injection. As described herein, therapeutic compositions and vaccines are contemplated and may be prepared and administered. An effective amount of the antibody can vary depending on the size of the mammal being treated, the severity of the disease, the route of administration, and the course of treatment. For example, each dose of mAb administered can range from approximately 0.5 mg/kg to approximately 400 mg/kg, with the preferred range from approximately 0.5 mg/kg to approximately 250 mg/kg. It is important to note that a dose as low as 10 µg (0.5 mg/kg) was effective in promoting remyelination of CNS axons in mice. The dose of mAb will also depend on the route of administration. For example, an iv dose administered to mice was 0.5 mg/kg, and an ip dose was 5.0 mg/kg. The course of treatment includes the frequency of administration of the mAb (e.g., daily, weekly, or bi-weekly) and the duration of the treatment (e.g., four weeks to four months). Thus, for example, a larger amount of mAb can be given daily for four to five weeks, as opposed to a smaller amount of mAb given for four months.

The effectiveness of the amount of the monoclonal antibody being administered can be assessed using any number of clinical criteria, for example, as described herein, including overall appearance of the mammal, the activity of the mammal and the extent of paralysis of the mammal. The effectiveness of the amount of monoclonal antibody necessary to induce remyelination in humans can also be assessed in a double blinded controlled trial. Patients with fixed neurological deficits from demyelinating disease can be treated with monoclonal antibody or controls. Improvement in isometric muscle strength as detected by quantitative biomechanics muscle testing could be used as the primary therapeutic end-point.

Ex *vivo* methods of stimulating remyelination in CNS axons are also encompassed by the present invention. For example, the monoclonal antibody may be used *in vitro* to stimulate the proliferation and/or differentiation of glial cells, such as oligodendrocytes. These exogenous glial cells can then be introduced into the CNS of mammals using known techniques. Remyelination of CNS axons would be increased by increasing the number of endogenous glial cells present (glial cells, such as oligodendrocytes play a critical role in the production of myelin).

*In vitro* methods of producing glial cells, or stimulating the proliferation of glial cells from mixed culture (e.g., rat optic nerve cell, or rat brain cell cultures) are also encompassed by this invention. For example, cells obtained from rat optic nerve, or rat brain, containing glial cells, are cultured as a mixed culture under conditions sufficient to promote growth of the cells. An effective amount of mAb capable of promoting remyelination of CNS axons, such as sHIgM22 (LIM 22), sHIgM46, ebvHIgM MSI19D10, and CB2bG8, is then added to the mixed culture of cells and maintained under conditions sufficient for growth and proliferation of cells. In addition, an effective amount of mAb such as AKJR4, CB2iE12, CB2iE7, or MSI19E5 may be added. In particular mixtures or combination of more than one of the antibodies provided herein are contemplated and provided for use in the methods. The mAb stimulates the proliferation of glial cells cultured in the presence of the mAb is increased, relative to the proliferation of glial cells grown in the absence of the mAb.

As stated above, the human monoclonal antibodies for use in the methods of the present invention can be, and are preferably, administered as medicaments, *i.e.,* pharmaceutical compositions. An effective amount of the Monoclonal antibody can thus be combined with, or diluted with, an appropriate pharmaceutically acceptable carrier, such as a physiological buffer, or saline solution. In particular, combinations or mixtures of more than one of the monoclonal antibodies presented herein may be combined with, or diluted with, an appropriate pharmaceutically acceptable carrier, such as a physiological buffer, or saline solution. In the instance where a vaccine is to be prepared, the monoclonal antibody or equivalent active of the invention may be prepared with a pharmaceutically effective and suitable carrier or adjuvant, and the protocol for administration may proceed in accordance with standard procedures for immunization known to the skilled practitioner.

The pharmaceutical compositions used in the methods of this invention for administration to animals and humans comprise the monoclonal antibodies in combination with a pharmaceutical carrier or excipient. In a preferred embodiment, the pharmaceutical composition may contain more than one, preferably two, monoclonal autoantibodies of the present invention. Such compositions are advantageous in that the presence of more than one monoclonal autoantibody will potentiate the activity of others in the same therapeutic composition or method.

The medicament can be in the form of tablets (including lozenges and granules), dragees, capsules, pills, ampoules or suppositories comprising the compound of the invention.

Advantageously, the compositions are formulated as dosage units, each unit being adapted to supply a fixed dose of active ingredients. Tablets, coated tablets, capsules, ampoules and suppositories are examples of preferred dosage forms according to the invention. It is only necessary that the active ingredient constitute an effective amount, *i*.*e*., such that a suitable effective dosage will be consistent with the dosage form employed in single or multiple unit doses. The exact individual dosages, as well as daily dosages, will, of course, be determined according to standard medical principles under the direction of a physician or veterinarian.

The monoclonal antibodies can also be administered as suspensions, solutions and emulsions of the active compound in aqueous or non-aqueous diluents, syrups, granulates or powders.

Diluents that can be used in pharmaceutical compositions (*e.g*., granulates) containing the active compound adapted to be formed into tablets, dragees, capsules and pills include the following: (a) fillers and extenders, *e.g.,* starch, sugars, mannitol and silicic acid; (b) binding agents, *e.g.,* carboxymethyl cellulose and other cellulose derivatives, alginates, gelatine and polyvinyl pyrrolidone; (c) moisturizing agents, *e.g.,* glycerol; (d) disintegrating agents, *e.g.,* agar-agar, calcium carbonate and sodium bicarbonate; (e) agents for retarding dissolution, *e.g.,* paraffin; (f) resorption accelerators, *e.g.,* quaternary ammonium compounds; (g) surface active agents, *e.g.,* cetyl alcohol, glycerol monostearate; (g) adsorptive carriers, *e.g.,* kaolin and bentonite; (i) lubricants, *e.g.,* talc, calcium and magnesium stearate and solid polyethylene glycols.

The tablets, dragees, capsules and pills comprising the active compound can have the customary coatings, envelopes and protective matrices, which may contain opacifiers. They can be so constituted that they release the active ingredient only or preferably in a particular part of the intestinal tract, possibly over a period of time. The coatings, envelopes and protective matrices may be made, for example, from polymeric substances or waxes.

The diluents to be used in pharmaceutical compositions adapted to be formed into suppositories can, for example, be the usual water-soluble diluents, such as polyethylene glycols and fats (*e.g.,* cocoa oil and high esters, [*e.g.,* C₁₄-alcohol with C₁₆-fatty acid]) or mixtures of these diluents.

The pharmaceutical compositions which are solutions and emulsions can, for example, contain the customary diluents (with, of course, the above-mentioned exclusion of solvents having a molecular weight below 200, except in the presence of a surface-active agent), such as solvents, dissolving agents and emulsifiers. Specific non-limiting examples of such diluents are water, ethyl alcohol, isopropyl alcohol, ethyl carbonate, ethyl acetate, benzyl alcohol, benzyl benzoate, propylene glycol, 1,3-butylene glycol, dimethylformamide, oils (for example, ground nut oil, glycerol, tetrahydrofurfuryl alcohol, polyethylene glycols and fatty acid esters of sorbitol or mixtures thereof.

For parental administration, solutions and suspensions should be sterile, e.g., water or arachis oil contained in ampoules and, if appropriate, blood-isotonic.

The pharmaceutical compositions which are suspensions can contain the usual diluents, such as liquid diluents, *e.g.,* water, ethyl alcohol, propylene glycol, surface active agents (*e.g*., ethoxylated isostearyl alcohols, polyoxyethylene sorbitols and sorbitan esters), microcrystalline cellulose, aluminum methahydroxide, bentonite, agar-agar and tragacanth, or mixtures thereof.

The pharmaceutical compositions can also contain coloring agents and preservatives, as well as perfumes and flavoring additions (*e.g*., peppermint oil and eucalyptus oil), and sweetening agents, (*e.g*., saccharin and aspartame).

The pharmaceutical compositions will generally contain from 0.5 to 90% of the active ingredient by weight of the total composition.

In addition to the monoclonal antibodies, the pharmaceutical compositions and medicaments can also contain other pharmaceutically active compounds, e.g. steroids, antiinflammatory agents or the like.

Any diluent in the medicaments of the present invention may be any of those mentioned above in relation to the pharmaceutical compositions. Such medicaments may include solvents of molecular weight less than 200 as the sole diluent.

It is envisaged that the monoclonal antibodies will be administered perorally, parenterally (for example, intramuscularly, intraperitoneally, subcutaneously, transdermally or intravenously), rectally or locally, preferably orally or parenterally, especially perlingually, or intravenously.

The administered dosage rate will be a function of the nature and body weight of the human or animal subject to be treated, the individual reaction of this subject to the treatment, type of formulation in which the active ingredient is administered, the mode in which the administration is carried out and the point in the progress of the disease or interval at which it is to be administered. Thus, it may in some case suffice to use less than a minimum dosage rate, while other cases an upper limit must be exceeded to achieve the desired results. Where larger amounts are administered, it may be advisable to divide these into several individual administrations over the course of the day.

The neuromodulatory agents of the invention may also be prepared for administration in the form of vaccines, which may comprise as the active, the herein recited autoantibodies, peptide analogs, or haptens, or possibly combinations thereof. Thus, the preparation of vaccines may proceed in accordance with known procedures, and monovalent as well as polyvalent vaccines are contemplated. Also, DNA sub unit vaccines, based upon the DNA molecules of the invention, may be prepared. All vaccines may be administered in accordance with standard practices of the physician or clinician, and such parameters are considered to be within the scope of the present invention.

Further, also described herein is treatment by gene therapy, where the appropriate neuromodulatory agent is correspondingly introduced to target cells for treatment, to cause or increase expression of the corresponding agent. Thus, in one embodiment, the DNA or a gene encoding the neuromodulatory agent, autoantibody, antibody peptide, etc., or a protein or polypeptide domain fragment thereof is introduced *in vivo, ex vivo,* or *in vitro* using a viral vector or through direct introduction of DNA. Expression in targeted tissues can be effected by targeting the transgenic vector to specific cells, such as with a viral vector or a receptor ligand, or by using a tissue-specific promoter, or both.

Viral vectors commonly used for *in vivo* or *ex vivo* targeting and therapy procedures are DNA-based vectors and retroviral vectors. Methods for constructing and using viral vectors are known in the art [*see*, *e.g.,* Miller and Rosman, BioTechniques 7:980-990 (1992)].

DNA viral vectors include an attenuated or defective DNA virus, such as but not limited to herpes simplex virus (HSV), papillomavirus, Epstein Barr virus (EBV), adenovirus, adeno-associated virus (AAV), and the like. Defective viruses, which entirely or almost entirely lack viral genes, are preferred. Defective virus is not infective after introduction into a cell. Use of defective viral vectors allows for administration to cells in a specific, localized area, without concern that the vector can infect other cells. Thus, adipose tissue can be specifically targeted. Examples of particular vectors include, but are not limited to, a defective herpes virus 1 (HSV1) vector [Kaplitt et al., Molec. Cell. Neurosci. 2:320-330 (1991)], defective herpes virus vector lacking a glyco-protein L gene [Patent Publication RD 371005 A], or other defective herpes virus vectors [International Patent Publication No. WO 94/21807, published September 29, 1994; International Patent Publication No. WO 92/05263, published April 2, 1994]; an attenuated adenovirus vector, such as the vector described by Stratford-Perricaudet et al. [J. Clin. Invest. 90:626-630 (1992); *see also* La Salle et al., Science 259:988-990 (1993)]; and a defective adeno-associated virus vector [Samulski et al., J. Virol. 61:3096-3101 (1987); Samulski et al., J. Virol. 63:3822-3828 (1989); Lebkowski et al., Mol. Cell. Biol. 8:3988-3996 (1988)].

Preferably, for *in vivo* administration, an appropriate immunosuppressive treatment is employed in conjunction with the viral vector, *e.g.,* adenovirus vector, to avoid immuno-deactivation of the viral vector and transfected cells. For example, immunosuppressive cytokines, such as interleukin-12 (IL-12), interferon-γ (IFN-γ), or anti-CD4 antibody, can be administered to block humoral or cellular immune responses to the viral vectors [*see, e.g.,* Wilson, Nature Medicine (1995)]. In addition, it is advantageous to employ a viral vector that is engineered to express a minimal number of antigens.

In another embodiment the DNA or gene can be introduced in a retroviral vector, *e.g.,* as described in Anderson et al., U.S. Patent No. 5,399,346; Mann et al., 1983, Cell 33:153; Temin et al., U.S. Patent No. 4,650,764; Temin et al., U.S. Patent No. 4,980,289; Markowitz et al., 1988, J. Virol. 62:1120; Temin et al., U.S. Patent No. 5,124,263; International Patent Publication No. WO 95/07358, published March 16, 1995, by Dougherty et al.; and Kuo et al., 1993, Blood 82:845. Retroviral vectors can be constructed to function as infections particles or to undergo a single round of transfection. In the former case, the virus is modified to retain all of its genes except for those responsible for oncogenic transformation properties, and to express the heterologous gene. Non-infectious viral vectors are prepared to destroy the viral packaging signal, but retain the structural genes required to package the co-introduced virus engineered to contain the heterologous gene and the packaging signals. Thus, the viral particles that are produced are not capable of producing additional virus.

Targeted gene delivery is described in International Patent Publication WO 95/28494, published October 1995.

Alternatively, the vector can be introduced *in vivo* by lipofection. For the past decade, there has been increasing use of liposomes for encapsulation and transfection of nucleic acids *in vitro.* Synthetic cationic lipids designed to limit the difficulties and dangers encountered with liposome mediated transfection can be used to prepare liposomes for *in vivo* transfection of a gene encoding a marker [Felgner, et. al., Proc. Natl. Acad. Sci. U.S.A. 84:7413-7417 (1987); *see* Mackey, et al., Proc. Natl. Acad. Sci. U.S.A. 85:8027-8031 (1988); Ulmer et al., Science 259:1745-1748 (1993)]. The use of cationic lipids may promote encapsulation of negatively charged nucleic acids, and also promote fusion with negatively charged cell membranes [Felgner and Ringold, Science 337:387-388 (1989)]. The use of lipofection to introduce exogenous genes into the specific organs *in vivo* has certain practical advantages. Molecular targeting of liposomes to specific cells represents one area of benefit. It is clear that directing transfection to particular cell types would be particularly advantageous in a tissue with cellular heterogeneity, such as pancreas, liver, kidney, and the brain. Lipids may be chemically coupled to other molecules for the purpose of targeting [*see* Mackey, et. al., *supra*]. Targeted peptides, *e.g.,* hormones or neurotransmitters, and proteins such as antibodies, or non-peptide molecules could be coupled to liposomes chemically.

It is also possible to introduce the vector *in vivo* as a naked DNA plasmid. Naked DNA vectors for gene therapy can be introduced into the desired host cells by methods known in the art, *e.g*., transfection, electroporation, microinjection, transduction, cell fusion, DEAE dextran, calcium phosphate precipitation, use of a gene gun, or use of a DNA vector transporter [*see, e.g.,* Wu et al., J. Biol. Chem. 267:963-967 (1992); Wu and Wu, J. Biol. Chem. 263:14621-14624 (1988); Hartmut et al., Canadian Patent Application No. 2,012,311, filed March 15, 1990; Williams et al., Proc. Natl. Acad. Sci. USA 88:2726-2730 (1991)]. Receptor-mediated DNA delivery approaches can also be sued [Curiel et al., Hum. Gene Ther. 3:147-154 (1992); Wu and Wu, J. Biol. Chem. 262:4429-4432 (1987)].

In a preferred embodiment of the present invention, a gene therapy vector as described above employs a transcription control sequence that comprises the DNA consensus sequence recognized by *e.g.* an autoantibody of the invention, *i.e.,* an antibody binding site, operably associated with a therapeutic heterologous gene inserted in the vector. That is, a specific expression vector of the invention can be used in gene therapy.

The present invention will be better understood from a consideration of the following non-limiting examples, which describe the preparation of materials, compounds and compositions and the development and practice of methods illustrative of the present invention. It will be apparent to those skilled in the art that many modifications, both of materials and methods, may be practiced without departing from the purpose and intent of this disclosure. The following examples are presented in order to more fully illustrate the preferred embodiments of the invention and serve also in fulfillment of applicants' duty to present the best mode known for the practice of the invention, and should in no way be construed as limiting the broad scope thereof.

### EXAMPLES

The Examples that follow, present the preparation and testing of human polyclonal and monoclonal antibodies. In particular, human polyclonal IgM antibodies were prepared, and tested whereby their ability to bind with high affinity to structures and cells within the CNS, for instance and including, oligodendrocytes, with a high specificity for neural tissue, and concomitant ability to enhance remyelination was demonstrated. As presented herein below remyelination was verified in a Theiler's virus and lysolecithin induced demyelination models.

### Introduction

Enhancement of remyelination is an important therapeutic goal in inflammatory demyelinating disorders of the CNS such as multiple sclerosis (MS). The identification of extensively remyelinated CNS lesions in some patients dying from acute MS, and in the applicants' recent data from cerebral biopsies suggests that full repair may be possible in the early stages of disease (Prineas and Connell 1979; Prineas, et al 1993; Rodriguez and Scheithauer 1994). However, as the disease progresses, remyelination is limited and occurs primarily at the periphery of the lesion. A number of reasons have been proposed for the failure to achieve complete remyelination in MS lesions (Ludwin 1981). Two important considerations include the depletion of cells capable of remyelination, and depletion of factors, which sustain their growth and differentiation. Thus early intervention to stimulate reparative cells or to remove inhibitory factors preventing myelin repair may be key to a therapeutic strategy.

A number of approaches have been tested as therapeutic strategies to promote remyelination in experimental animals. Transplantation of oligodendrocytes or progenitor glial cells into previously demyelinated lesions can result in remyelination of CNS axons, and to a smaller extent migration of myelinating precursor cells. It has been shown that central remyelination restores conduction. An alternative strategy proposed by the applicants is to enhance endogenous remyelination (Miller, D.J. et al. 1995b). This approach implies that the cells capable of remyelination and the factors which sustain their growth or differentiation are present in demyelinated lesions, but that there are mechanisms which inhibit this response and thus prevent full remyelination.

One of the first descriptions of enhancement of endogenous CNS remyelination came from the experimental autoimmune encephalomyelitis (EAE) model. Using incomplete Freund's adjuvant (IFA) as a vehicle and myelin components as the antigen, immunization after disease induction promoted structural and functional recovery in guinea pigs with EAE (Trautgott et al., 1982). Based on the promotion of endogenous remyelination in EAE, similar experiments were conducted in mice chronically infected with Theiler's murine encephalomyelitis virus (TMEV) (Lang, W. et al., 1984). TMEV infection of susceptible strains of mice results in chronic inflammatory demyelination in the context of virus persistence which serves as an excellent model of MS. Chronically infected mice treated with spinal cord homogenate (SCH) in IFA showed substantial CNS remyelination compared to control animals given adjuvant alone. This observation was followed by experiments demonstrating that passive transfer of either antiserum or purified Ig from uninfected syngeneic animals immunized with SCH/ IFA promotes remyelination in mice chronically infected with TMEV. (Rodriguez et al., 1987a; Rodriguez and Lennon 1990; Rodriguez 1991). These experiments were novel and contrasted the classical view that the humoral immune response plays a pathogenic role in CNS demyelinating disease. These experiments were the first to demonstrate that Igs, in particular autoantibodies, could play a beneficial role in promoting CNS remyelination.

Based on these observations, the generation was begun of mAbs which promoted CNS remyelination in the Theiler's model of demyelination. Spleens from SJL mice that had been injected with SCH/IFA were used as the source of B cells for fusion hybridoma production. Serum from these mice had been shown previously to promote remyelination in chronically demyelinated mice. Hybridomas were generated and screened by ELISA using SCH as the antigen. After initial fusion, two of 95 viable hybridomas secreted antibodies that bound significantly to SCH. Hybridoma cells from these wells (designated SCH79 and SCH94) were subcloned and screened for SCH immunoreactivity. In the SCH79 series, 14 of 49 clones were positive, and for the SCH94 series 17 of 32 were positive. The monoclonal antibodies produced by these hybridomas were then tested *in vivo* for their ability to promote remyelination in the Theiler's model system. Six to eight month chronically infected SJL mice were given either intraperitoneal or intravenous injection of mAbs twice weekly for 4 to 5 weeks for a total dose of 0.5 mg to 5.0 mg. Two mAbs, SCH94.03 and SCH94.32, showed the greatest enhancement of CNS remyelination (Miller et al., 1994). Sequence of variable heavy and light chains proved these two antibodies to be identical, thus later designated as SCH94.03 (Miller and Rodriguez 1995c).

SH94.03 treatment of SJL mice with chronic TMEV-induced demyelination generally results in 20-30% remyelination of total demyelinated area, as compared to less than 5 % in PBS treated control animals. This represents a 4-6 fold increase in remyelination over controls and it is estimated from axonal counts that this represents an average of 100,000 remyelinated axons in SCH94.03 treated animals. Electron microscopic analysis of fully remyelinated lesions reveals no remaining unremyelinated axons suggesting that the remyelination of available axons in these lesions is close to 100%.

SCH94.03 belongs to the IgM subclass and is highly polyreactive against known and unknown protein antigens including cytoskeletal proteins. Of interest, it is encoded by unmutated Ig germline genes confirming that SCH94.03 is a natural autoantibody. Of importance, SCH94.03 recognizes an unidentified surface antigen on oligodendrocytes, providing a potential target for the mechanisms of action of this antibody.

Central to this hypothesis are the differences in the biology between SCH94.03 and CH12. At the time of the identification of the Ig gene sequence of SCH94.03, it was discovered that there was another mouse, IgMk antibody with an identical germline sequence and gene combination. This IgM antibody, designated CH12, is from a CD5 + B cell lymphoma and has apparent specificity phosphatidyl choline. SCH94.03 and CH12 have 99.1 % amino acid identity in the V_{L} region and have identical V_{H} sequences. The only differences between SCH94.03 and CH12 are in the CDR3 region, due to N-region insertions. CH12 does not label the surface of oligodendrocytes and does not promote remyelination in the Theiler's model system, thus establishing that the only molecular difference to account for the mechanism of promotion of remyelination lies within the CDR3 region. This conclusion supports the hypothesis that binding of these mAbs to specific antigens, likely within the demyelinated lesion, is important for the induction of remyelination.

To date, six different mouse monoclonal antibodies have been identified which promote remyelination in the TMEV model for demyelinating disease (Asakura et al., 1998). All six antibodies are of the IgM isotype and retain germline sequences that are reminiscent of autoantibodies (Miller and Rodriguez 1995; Asakura et al., 1996). Each displays a broad antigen binding specificity but most importantly, each binds to antigens expressed on the surfaces of oligodendrocytes. IgM antibodies studies this far which did not bind oligodendrocytes did not promote remyelination. The prototypic member of this group, mAb SCH94.03, has been shown to promote remyelination in several mouse models for demyelinating disease. In mice with chronic virus-induced (TMEV) demyelination, treatment with SCH94.03 results in a 4-6 fold increase in remyelination. SCH94.03 treatment has also been shown to significantly increase the rate of spontaneous remyelination that occurs after chemically-induced demyelination following lysolecithin injection.

Given the success with the isolation and characterization of remyelination promoting mouse antibodies, the identification of human counterparts to the mouse monoclonals was begun using an antigen-independent strategy. The rationale was to identify human antibodies that react with mouse spinal cord homogenate by ELISA assay, and which bind with high affinity to structures and cells within the CNS. These antibodies were then tested in the mouse demyelination models for their ability to promote myelin repair. Pooled human IgM and IgG, sera from patients from the Mayo Hematology clinic, and monoclonal antibodies from EBV-immortalized human B cells (from various sources as described in the results) were characterized and tested in the mouse models. The results of these experiments are presented below.

### RESULTS

### Human polyclonal IgM, but not IgG, binds to rat brain slices and oligodendrocytes in culture.

By immunocytochemistry, polyclonal human IgM stains the surface of a subpopulation of oligodendrocytes (**Figure 6**) and is highly reactive to structures and cells in a slice of rat brain (**Figure 1B**)**.** No reactivity to oligodendrocyte surface antigens (**Figure 6**) or slices of rat brain (**Figure 1A**) was observed with polyclonal human IgG. Immunocytochemistry utilizing polyclonal human IgM or IgG on fixed and permeabilized mixed glial cells demonstrated minimal staining of intracellular structures (data not shown).

The specificity of polyclonal human IgM to CNS structures and cells and the binding to oligodendrocytes may drive the pronounced remyelinating potential. In contrast, polyclonal human IgG, while promoting remyelination over control levels **(See Table 1),** does not bind to CNS, and may function by a different mechanism than polyclonal human IgM.

### Human polyclonal IgG and IgM promote CNS remyelination in TMEV infected mice.

Even though the cause of MS is unknown, epidemiologic studies suggest that the disease may be triggered by an infectious agent (Franklin et al., 1991), although to date no conclusive evidence proves this theory. Most recently, herpes virus type 6 has received attention as a possible etiologic agent in a subset of patients (Groves et al., 1993). Of the multiple epidemiological factors studied to correlate with exacerbations, only recent virus infection has been associated consistently (Smith et al., 1981). In addition, the major established treatment for MS, IFN-β, is a cytokine central for control of virus replication (14).

One important murine model for the study of MS is Theiler's murine encephalomyelitis virus (TMEV). This positive-stranded picornavirus has a number of advantages: (1) The virus is a natural pathogen of mice, a species for which there is vast known regarding immunology and genetics; (2) Primary demyelination (destruction of myelin sheaths) is the main physical manifestation of chronic infection (Trautgott et al., 1982); (3) Host genetics play a critical role in determining susceptibility or resistance to chronic viral persistence, demyelination and neurologic disease (Lang et al., 1996; Rodriguez et al., 1987a, 1987b); (4) As in MS, pathology is immune-mediated (Rodriguez and Lennon, 1990; Rodriguez 1991; Miller et al., 1994; Miller and Rodriguez 1995c); (5) There is complete information regarding the molecular virology, including details of virus replication and virus assembly; (6) The majority of susceptible mice harboring chronic TMEV infection develop neurologic disease similar to MS - weakness of the extremities, spasticity, incontinence, decreased spontaneous activity and eventually paralysis (Asakura et al., 1996a; Askura et al., 1998).

Similar to MS, TMEV-infected mice demonstrate a wide spectrum of disease phenotypes (defined as both demyelination and neurologic deficits) (Ludwin, 1997). At one extreme are animals in which virus replicates rapidly in CNS neurons, is not cleared by the immune system, and results in severe encephalitis and death (Asakura et al., 1996). This pattern of pathology is observed in neonatal mice or mice with severe immunodeficiency. This fulminant disease contrasts with mice that mount a protective immune response, primarily mediated by class I-restricted T cells which clear virus from CNS. These animals develop acute encephalitis, after which virus is cleared from the CNS without subsequent demyelination (Blakemore et al., 1997; Jeffrey and Blakemore 1995). In between these extremes are animals which develop and resolve acute disease, but enter a chronic phase characterized by progressive demyelination, and virus persistence in oligodendrocytes, astrocytes, and microglia (Rivera-Quinones et al., 1998; Miller et al., 1995a). Although these mice mount an immune response which prevents death and overwhelming encephalitis, failure to clear virus from white matter results in chronic, persistent inflammation and immune-mediated demyelination. To avoid anti-human immune responses, chronically infected mice (5 to 6 months after TMEV infection) were treated with a single bolus injection of 1 mg of Ig intraperitoneally. The total dose of Ig was approximately 0.05 g/kg body weight, which corresponds to one eighth the total dose used for human IVIg treatment. There was no significant differences in the areas of myelin pathology between the treatment groups **(Table 1).** However, mice treated with polyclonal human IgM demonstrated prominent remyelination. Approximately one quarter of the total area of myelin pathology was repaired in mice treated with polyclonal human IgM. The extent of remyelination was significantly higher than the spontaneous remyelination observed in the PBS-treated control groups (p<0.01), but also higher than that observed in mice treated with polyclonal human IgG (p=0.05). Mice treated with polyclonal human IgG demonstrated more remyelination than mice treated with PBS. Individual lesions which were remyelinated showed almost complete repair with few inflammatory cells or macrophages (**Figure 12A****,B**). Frequently 500 to 1000 remyelinated axons were observed in each of lesion of this type. In contrast, most lesions in mice treated with PBS had few remyelinated axons and the lesions contained many inflammatory cells and macrophages, signs of active myelin destruction.

### Polyclonal human IgM enhances remyelination in lysolecithin-induced demyelination.

Lysolecithin injection into the spinal cord is a well established method for chemically induced spinal cord injury and demyelination. Injection results in reproducible demyelinated lesions which undergo complete spontaneous remyelination by 5-6 weeks. Previous work by the present applicants has shown that treatment with remyelination promoting antibodies increases the rate of endogenous remyelination such that lesions are substantially repaired by 3 weeks. The monophasic nature of lysolecithin lesions, their rapid spontaneous repair, and the lack of clinical deficits in lesioned animals, are in contrast to chronic TMEV induced demyelination and may model aspects of spinal cord injury better than MS.

Animals with lysolecithin-induced spinal cord lesions were treated with polyclonal human IgM and IgG. Photomicrgraphs of the lesion areas showed the animals that received polyclonal human IgM contained many remyelinated axons, while the animals treated with polyclonal human IgG or PBS contained few remyelinated axons (data not shown). In an effort to quantitate the number of remyelinated axons per area of lesion, remyelinated axons were counted under high magnification from lesions of the 3 treatment groups. There were significantly more myelinated axons in lysolecithin lesions treated with polyclonal human IgM than animals treated with polyclonal human IgG (p<0.05).

### Polyclonal human immunoglobulins react with multiple self antigens and chemical haptens.

Antigen specificities of Igs used in this study were studied by ELISA. Previous studies have indicated the antibodies which promote remyelination show wide reactivity to multiple protein and hapten antigens (Miller et al., 1995c). Both polyclonal human IgG and IgM bound multiple protein antigens and chemical haptens **(****Figures 15** **&** **16****).**

### Polyclonal human IgG and IgM does not react with TMEV antigens.

To exclude the possibility that enhanced remyelination by pooled human IgM was the result of specificity for TMEV antigens. Western blotting using purified TMEV was performed. None of the Abs used in this study reacted with any of the known TMEV capsid proteins (data not shown). In contrast, rabbit polyclonal antibody raised against TMEV showed strong reactivity to the VP1, VP2, VP3 capsid antigens of the virus.

### CNS -reactive monoclonal antibodies can be identified from human serum.

Since natural autoantibodies exist in the normal human population it should be possible to identify human natural monoclonal autoantibodies by screening a large number of human monoclonal IgM clones. An initial screen was designed for polyreactivity of serum-derived human monoclonal antibodies utilizing an unfixed brain slice binding assay system. Positive clones are those samples that bound to specific brain structures or anatomical layers or cell populations significantly above the background level of a fluorescently-conjugated secondary antibody alone.

Fifty two samples of human IgM, purified from the sera of patients obtained from the hematology department Mayo Clinic, under the direction of Dr. Robert A Kyle, were tested for CNS specificity in a brain slice binding assay. Thirty two antibodies were determined to bind above background. A variety of the reactivities are presented in **Figures 1** **and** **2****.** Fifty human serum-derived IgGs were also tested for CNS specificity in a brain slice binding assay and no distinct binding patterns above background were identified (data not shown). Thus, immediately, a major difference between human monoclonal IgMs and IgGs was identified.

The 32 positive antibodies were further tested for binding via a spinal cord homogenate ELISA system (**Figure 10**) and for binding to both live and fixed rat mixed primary glial cell cultures (**Figure 7**). A tabulation of the reactivities of those antibodies (sHIgm # antibodies) is shown in **Table 2.** The criteria for testing an antibody for biological activity *in vivo* was CNS specificity, binding to unfixed oligodendrocytes in culture and a significant reactivity to SCH via ELISA. Thus, sHIgM 22 and sHIgM46 were identified as promoting remyelination. Several other sHIgM candidates remain to be carefully studied. The ability of tested sHIgM antibodies to bind oligodendrocytes is shown in **Table 3.**

### CNS-reactive monoclonal antibodies were identified from the supernatant of EBV-immortalized human B-cell clones.

Supernatants from cell clones that had a total IgM concentration over 3 ug/ml were tested in a brain slice assay system for their ability to bind to CNS structures. One hundred forty clone supernatants were tested for brain slice binding. Fifteen antibodies were determined to bind above background. A tabulation of the reactivities of these antibodies (MSI #) are shown in **Table 2.** A representation of ebvHIgM reactivities are presented in **Figures 4** **and** **5****.** The ability of certain of these antibodies to bind oligodendrocytes was tested. These results are tabulated in **Table 3.**

### sHIgMs bind to human cortical white matter.

To confirm that sHIgMs bind to human CNS, human cortical white matter was immuno-labeled in system analogous to the rat brain slce assay. **Figure 3** presents several of the CNS specificities of sHIgMs. Four antibodies bind well to human CNS **(Figures 3B****,C****,D****,E****)** while **Figure 3A** shows a SHIgM that binds slightly above background level.

### Human monoclonal antibodies bind to surface antigens on cells in mixed primary glial cultures.

Several of the sHIgMs bind to cells in rat primary mixed glial cell cultures. sHIgM 12 binds to presumptive oligodendrocyte progenitor clusters (**Figure 7A**) in a field of 04 positive oligodendrocytes. Four other sHIgMs (**Figures 7B****,C,D,F**) bind to morphologically mature oligodendrocytes. sHIgM 30, binds to most cells (oligodendrocytes, asrocytes, microglial) in the culture (**Figure 7E**).

### sHIgMs and ebvHIgMs promote CNS remyelination in TMEV infected mice.

To avoid anti-human immune responses, chronically infected mice (5 to 6 months after TMEV infection) were treated with a single intraperitoneal bolus injection of 0.5 mg of human monoclonal antibody. Of the human monoclonal antibodies tested in vivo to date, sHIgM 22, sHIgM46 and ebvHIgM MSI19D10 significantly promoted remyelination over other tested human monoclonal IgMs (sHIgMs 1,2, and 14). There are no differences in the areas of myelin pathology between the treatment groups **(Table 1).** Mice treated with sHIgM 22 and sHIgM46 demonstrated prominent remyelination. Approximately one fifth of the total area of myelin pathology was repaired in mice treated with sHIgM 22 **(Table 1).** The extent of remyelination was significantly higher than the spontaneous remyelination observed in PBS-treated control groups (p<0.05, **Table 1**). Individual remyelinated lesions showed almost complete repair with few inflammatory cells or macrophages (**Figure 12**). Frequently 500 to 1000 remyelinated axons were observed in each of lesion of this type. In contrast, most lesions in mice treated with PBS had few remyelinated axons and the lesions contained many inflammatory cells and macrophages, signs of active myelin destruction. Consistent with previous work demonstrating that oligodendrocyte-reactive mouse Monoclonal antibodies can promote remyelination in vivo (Asukara et al., 1998); oligodendrocyte-reactive human antibodies with similar reactivities to the mouse counterparts can promote remyelination *in vivo* as well.

**Table 1. CNS Remyelination by Human Antibodies**

| Treatment | No. of Mice | Area of White Matter (mm2) | Area of Myelin Pathology (mm2) | Area of CNS-Type Remyelination (mm2) | Area of CNS-Type Remyelination (%) |
|---|---|---|---|---|---|
| Polyclonal Human IgG | 10 | 8.6 ± 0.52 | 0.86 ± 0.10 | 0.13 ± 0.02 | 14.15 ± 2.38* |
| Polyclonal Human IgM | 13 | 9.70 ± 0.43 | 1.21 ± 0.21 | 0.24 ± 0.04 | 23.2 ± 3.26** |
| sHIgM 1 | 4 | 9.34 ± 1.93 | 0.68 ± 0.07 | 0.03 ± 0.01 | 8.35 ± 3.73 |
| sHIgM 2 | 4 | 8.78 ± 0.70 | 0.87 ± 0.12 | 0.09 ± 0.01 | 11.37 ± 1.30 |
| sHIgM 14 | 7 | 10.68 ± 0.24 | 0.98 ± 0.09 | 0.08 ± 0.03 | 8.57 ± 2.51 |
| sHIgM 22 | 8 | 10.55 ± 0.41 | 1.16 ± 0.22 | 0.19 ± 0.05 | 17.06 ± 3.42* |
| sHIgM 46 | 5 | 9.44 ± 0.36 | 0.66 ± 0.06 | 0.18 ± 0.04 | 27.12 ± 4.01 |
| ebvHIgM MSI19D10 | 3 | 8.24 ± 0.40 | 0.90 ± 0.14 | 0.26 ± 0.07 | 26.47 ± 3.71*** |
| ebvHIgM AKJR4 | 4 | 8.70 ± 0.84 | 1.10 ± 0.15 | 0.05 ± 0.03 | 4.15 ± 1.98 |
| PBS | 7 | 9.78 ± 0.60 | 1.20 ± 0.22 | 0.06 ± 0.02 | 6.74 ± 1.80 |

Values represent the mean ± SEM. One way ANOVA and *t*-test were used to compare the percent area of CNS-type remyelination in mice treated with human antibodies to mice treated with PBS. Such analysis revealed *p<0.05; ^{†}p<0.01, ^{‡}p<0.001. Comparison of mice treated with polyclonal human IgG to other treatments revealed; polyclonal human IgM p=0.05, sHIgM 46 P<0.05. All other comparisons were not statistically significant. There was no difference in the CNS-type remyelination between polyclonal human IgM, sHIgM 22 and sHIgM 46. Area of PNS-type Schwann cell remyelination ranged from) to 0.08 mm². This corresponded to 0.0 to 6.92 percent area of PNS-type Schwann cell remyelination as a function of myelin pathology. There was no statistical difference in the area of myelin pathology in the various treatment groups or compared to PBS or in the PNS-type Schwann cell remyelination between groups.

**TABLE 2**

| CLONE NAME* | DESCRIPTION OF IMMUNOFLUORESCENT STAINING OF NEONATAL RAT CEREBELLUM |
|---|---|
| CB2b-G8 | Identified large neuronal cell bodies in granular layer; small round cell bodies in the molecular layer; fibrous astrocytes in the central white matter. |
| CB2e-C2 | Weak label of Purkinje cell bodies; small processed cells, of oligodendrocyte and microglial morphology, and astrocytes in central white matter and granular layer. |
| CB2i-E7 | Labels Purkinje cells, cells in granular layer; central while matter in the folia; oligodendrocyte cytoskeleton and microglial cells. |
| CB2i-E12 | Strong label of Purkinje cell bodies, dendritic arbors and small round cells in molecular layer; nearly confluent label of granular layer; fibrous astrocytes in central white matter. |
| CB2i-G2 | Strong fibrous astrocyte label in central white matter tracts; identifies Purkinje cell bodies; punctate label over granular layer; small cell bodies in molecular layer. |
| CB2L-H1 | Strong label of glial cell bodies in central white matter and Purkinje cell bodies; weaker label of dendritic arbors; very pronounced small cel bodies in molecular layer. |
| MSI 10-E10 | Strong label of fibrous central white matter tracts, similar to that observed using anti-glycolipid or anti-microglial antibodies. |
| MSI 16-E6 | Strong label of Purkinje cell bodies, weaker dendritic arbors; nearly confluent label of small cells in granular layer, central white matter nearly unlabeled. |
| MSI 17-A2 | Strong label of small round cells in molecular layer and Purkinje cell bodies; diffuse label of granular layer; unlabeled central white matter. |
| MSI 19-C11 | Fibrous appearance to central white matter with many cells of oligodendrocyte morphology; punctate surface label over most of tissue, but concentrated over presumptive cell bodies. |
| MSI 19-E5 | Extra cellular matrix-like label of molecular layer; strong fibrous label of central white matter, identifying many individual glial cells. |
| AKJR4 | Identifies all neuronal cell bodies in granular layer; small round cell bodies in central white matter and molecular layer. |
| MSI 19D10 | Binds strongly to cells of the granular layer and to Purkinje cells and their dendritic arbors, in addition to weakly identifying white matter and astrocytes. |
| MSI 20H10 | Binds to central white matter, the granular and molecular layer and Purkinje cells with varying degrees of intensity. |
| MSI 17E11 | Binds in a punctate manner to only a few glial-appearing cells at the surface of the brain slice. |
| sHIgM1 | Binds the cytoskeleton of astrocytes overlying the central white matter of the folia. |
| sHIgM2 | Binds to cells of the granular layer and to fibers traversing the central white matter of the folia. |
| sHIgM12 | Binds to lend a spongy appearance to the central white matter of the folia, and a uniform label over the molecular layer, reminiscent of an extracellular matrix molecule. |
| sHIgM14 | Binds well to cells of the granular layer and Purkinje cells located at the surface of the slice, while the central white matter of the folia is largely devoid of label. |
| sHIgM22 | Binds well to the cytoskeleton of damaged astrocytes overlying the central white matter of the folia, Purkinje cells and their dendritic arborizations, and to small round cells in the molecular layer, weakly labels surface of granular cells. |
| sHIgM26 | Binds to oligodendrocyte-appearing cells and fibrous white matter. |
| sHIgM29 | Binds weakly to many structures in the cerebellum with intensity just above background except for a small population of neurons in the granular and molecular layer. Axon extensions over 100µm are clearly delineated. |
| sHIgM30 | no binding to unfixed cerebellum |
| sHIgM31 | Binds predominantly to the granular layer, with little binding to the white matter, Purkinje cells or astrocytes. |
| sHIgM32 | Binds to type 2 astrocyte-appearing cells. |
| sHIgM42 | Binds in a fibrous pattern to the entire folia, molecular and granular layers and white matter. |
| sHIgM46 | Binds in a fibrous pattern to the granular layer and white matter. The Purkinje cells are well defined. |
| sHIgM50 | Binds predominantly to the granular layer with little binding to the white matter, Purkinje cells or astrocytes. |
| sHIgM51 | Binds to small cells similar to microglia in molecular and granular layers |

| | |
|---|---|
| Of the 96 human EBV B-cell clones generated, 60 produced IgM antibody t a concentration of 2µg/ml or greater. Of these, 11 were found to strongly bind to murine cerebellum on a cnsistant basis. Another 10 bound to murine cerebellum weakly or inconsistantly, while 39 did not bind at all. Pictures of the immunofluorescent staining of the consistently strongly staining antibodies listed above as well as four representative negative clones (CB2g-E10, CB2g-F11, CB2I-F10, MSI24-D6) are included. *CB: clones generated from human umbilical cord blood; MSI: clones generated from the peripheral blood of multiple schlerosis patients; AKJR: clones generated from the peripheral blood of rheumatoid arthritis patients. | |

**TABLE 3**

| Oligodendrocyte Binding | |
|---|---|
| Antibody | Binding to Oligodendrocyte |
| MSI 17 A.2 | negative |
| MSI 19C11 | negative |
| MSI 19E5 | labels oligodendrocyte of mature morphology |
| AKJR 4 | negative |
| MSI 19010 | labels some oligodendrocyte of mature morphology |
| MSI 20H10 | not tested |
| MSI 17E11 | not tested |
| SH1gm 1 | no reactivity to surface antigens |
| SH1gm 2 | no reactivity to surface antigens |
| SH1gm 12 | presumptive oligodendrocyte progenitors prior to sulfortide expression |
| SH1gm 14 | label of mature multi-processed oligodendrocyte |
| SH1gm 22 | labels mature stages of oligodendrocyte and process extensions |
| SH1gm 26 | no reactivity to surface antigens |
| SH1gm 29 | no reactivity to surface antigens |
| SH1gm 30 | no reactivity to surface antigens |
| poly hIgG | negative on surface |
| poly hIgm | subset of mature oligodendrocyte |
| CB2b G8 | labels oligodendrocytes (human) of mature morphology |

| Antibody | Binding to Oligodendrocyte |
|---|---|
| CB2e C2 | negative |
| CB2iE7 | labels oligodendrocytes of mature morphology |
| CB2i E12 | negative |
| CB2i G2 | negative |
| CB2L H1 | negative |
| MSI10E10 | negative |
| MSI 16E6 | negative |
| sHIgM31 | No binding to Oligodendrocyte |
| sHIgM32 | No binding to Oligodendrocyte |
| sHIgM42 | Binds to mature stages of oligodendrocyte and faintly to underlying astrocytes. |
| sHIgM46 | Strongly binds to both mature and immature stages of oligodendrocytes with punctate label. |
| sHIgM50 | Weak punctate label of subset of mature stages of oligodendrocyte |
| sHIgM51 | Binds to mature stages of oligodendrocytes and faintly to underlying astrocytes. |

### Monoclonal Antibodies that promote remyelination cause Ca²⁺ flux in glial cells in culture.

The response of cultured glial cells to physiological concentrations of remyelination promoting antibodies suggests that these antibodies may have direct effects on the biochemistry of glial cells through the regulation of cellular calcium flux. This effect may represent an important aspect of the molecular mechanism of antibody induced remyelination. **Figure 21** demonstrates glial Ca²⁺ responses to four different antibodies. Two of these antibodies, sHIgM 22 and SCH94.03, promote remyelination *in vivo,* and two, sHIgM 14 and CH12, do not promote remyelination. Cells which responded to antibody, exhibited one of two different types of calcium spikes. Some cells responded with a rapid onset spike of short duration (fast response) as shown by the red traces in panels A and B. A separate subset of cells responded with a slower onset, longer duration spike (slow response) as demonstrated by the black traces in Panels A and B. The antibodies sHIgM 22 and SCH94.03 each elicited both types of responses but always from different individual glial cells. These qualitatively different responses clearly suggest two distinct molecular modes of action on distinct subsets of cells. A response to antibody (either the fast or the slow response) was observed in 30 of 251 cells after treatment with sHMab22 and in 36 of 251 cells treated with SCH 94.03. Antibodies which do not promote remyelination in vivo (sHIgM 14 & CH12) were not observed to cause calcium flux in cultured glia (panel C). A total of 203 cells were examined for each of these antibodies.

### Human monoclonal antibodies bind to primary neurons.

Many of the sHIgMs and ebvHIgMs bind to neuronal populations in brain slices. However, many of the neurons bound are at the surface of the slice, presenting the likelihood that the antibodies may bind internal epitopes inside damaged neurons. Positive binding HIgMs were tested for binding to live rat granule cells in culture. **Figure 22** demonstrates the binding of two sHIgMs to live neurons. sHIgM 12 binds to both axonal and dendritic extensions of the neurons (**Fig. 22A**), while ebvHIgm CB2iE12 binds exclusively to the exterior of the granule cells membrane and proximal axon extensions (**Figure 22B**). These reactivities were verified by double label immunocytochemistry by c-labeling human antibodies positive neurons with anti-neurofilament and anti-microtublue associated protein 2 antibodies (data not shown).

Of particular interest to our laboratory is the possibility that demyelination predisposes axons to immune-mediated injury and corresponding neurologic deficits. In our recent analysis of β2 microglobulin deficient mice (β2m -/-), we demonstrated that in the absence of class I MHC, TMEV infected mice develop large demyelinating lesions but fail to develop clinical deficits. The mice demonstrated a relative preservation of axons with increased sodium channel densities and remyelination of spinal cord white matter. Axonal preservation appears to be essential for the maintenance of neurologic function. The observation that human antibodies can bind specifically to neurons presents another potential avenue for antibodies mediate repair of the CNS. Certain antibodies may be able to potentiate remyelination through action on the neuron. The repair of CNS lesions may be potentiated by monoclonal antibodies by many possible scenerios. 1) increasing the adhesive bonds between neurons and oligodendrocytes. 2) direct cell stimulation of neuron to upregulate trophic factors and attract oligodendrocyte progenitors the area of bare axons. 3) neuroprotection of axons by antibody blockade of leaky ion channels on bare axons. 4) protection of bare axons from recognition by activated and destructive immune cells.

### MATERIALS AND METHODS

### A. Monoclonal Antibody Production, Characterization, Screening and Purification:

### Sources of Abs and Ab purification.

Normal human IgM was purified from pooled plasma of over 2,500 healthy donors by modified Deutsch-Kistler-Nitschmann's ethanol fractionation procedure followed by octanoic acid precipitation and two successive ion-exchange chromatography steps as previously described (Hurez et al., 1997). The purity of IgM was over 90% as confirmed by ELISA and SDS-polyacrylamide gel electrophoresis (PAGE). Pooled human IgG from healthy donors used clinically as IVIg was purchased from Miles Inc. (Elkhart, IN). Samples were obtained from the dysproteinase clinic under the directin of Dr. Robert A. Kyle, Mayo Clinic. The samples of sera came from patients with a wide variety of conditions characterized by a monoclonal IgG or IgM spike in the serum, including Waldenstrom's macroglobulinemia, multiple myeloma, lymphoma, benign monoclonal gammopathy.

### Generation of Epstein-Barr Virus (EBV) Immortalized B Cell lines

The B95-8 marmoset cell line was obtained from ATCC (#CRL 1612) for the growth and isolation of EBV. Cells are seeded at 1x10⁶ cells/ml in complete RPMI-10 medium followed by 3 days of incubation in a humidified, 37°C, 5% CO₂ incubator. The cells are harvested and the supernatant is cleared by centrifugation for 10 minutes at 300 *x g* and 4°C. The EBV-containing supernatant is passed through a 0.45µm filter and the flow through is collected and stored at -130°C (liquid nitrogen). This EBV-supernatant generally contains 10²-10³ transforming units/ml.

Peripheral B cells for immortalization were collected from the blood of normal adults (NA), adults with rheumatiod arthritis (AKJR), adults with multiple sclerosis (MS), and from fetal cord blood (CB). Heparinized blood (15 ml) is diluted 1:2 in phosphate buffered saline (PBS) and 12 ml of this dilution is underlayered with 12 ml of Ficoll-Hypaque in a 50 ml centrifuge tube. The tube is centrifuged for 8 minutes at 1500 x g, at room temperature, and the buffy coat interface is removed and transferred to a new 50 ml centrifuge tube. The cells are washed by centrifugation (15 minutes, 300 x g, room temperature), once in PBS and then twice in Hank's balanced saline solution (HBSS). The cells are then resuspended in 2-5 ml of complete RPMI-10 medium and counted.

The cells are diluted to 4x10⁶ cells/ml in complete RPMI-10 medium, 2.5 ml (1x10⁷ cells) are transferred to a 50 ml centrifuge tube and 2.5 ml of EBV-supernatant is added. The tube is incubated for two hours in a 37°C water bath followed by the addition of 5 ml of complete RPMI-10 medium containing 1 *µ*g/ml cyclosporin A. The 10 ml of cell suspension is then transferred to a 25 cm² tissue culture flask and cultured for 3 weeks in a humidified, 37°C, 5% CO₂ incubator. After 3 weeks, an aliquot of the culture is cryopreserved and the remainder is expanded and clonal cell lines are isolated by limiting dilution.

### Purification of IgM Antibodies -

Human serum samples used for study were chosen solely by the presence of a high IgM peak in the Ig chromatogram. Samples were obtained from the dysproteinase clinic under the directin of Dr. Robert A. Kyle, Mayo Clinic. The samples of sera came from patients with a wide variety of conditions characterized by a monoclonal IgG or IgM spike in the serum, including Waldenstrom's macroglobulinemia, multiple myeloma, lymphoma, benign monoclonal gammopathy. Patients sera were dialyzed against deionized water during three days. Euglobulinic precipitates were collected by centrifugation (14000 rpm / 30 min.) and dissolved in PBS. Solutions were cleared by centrifugation (14000 rpm / 30 min.) and chromatographed on Superose 6 column (Pharmacia, Upsalla) equilibrated with PBS. Fractions corresponding to IgM were pooled and analyzed by reducing SDS PAGE (12 % gel). IgM concentrations were determined by staining the SDS gels with Cypro Orange (Molecular Probes, Eugene) and subsequent scanning on Storm 840 (Molecular Dynamics). Monoclonal IgM (Sigma, St Louis) were used as a standard for concentration measuring. IgM solutions were sterilized by filtration through 0.22 *µ*m filters.

### Characterization of Antigen Binding Specificity

ELISA against mouse spinal cord homogenate (SCH) was used as an assay for the preliminary screening of antibodies prior to in vivo testing for ability to promote remyelination. To further characterize the polyreactivity and antigenic specificity of selected antibodies, an ELISA against a standard panel of protein and chemical antigens is used, as well as analysis of the antibody staining patterns in sectioned neural tissues and on cultured oligodendrocytes.

### ELISA Assay

Antibodies were screened for their reactivity to mouse spinal cord homogenate (SCH). SCH at 0.01 mg/ml coated onto polystyrene microtiter plates in 0.1 M carbonate buffer, pH 9.5, for 18 hours at 4°C, and then washed 3x with PBS. Coated plates were blocked with phosphate buffered saline (PBS) containing 1 % BSA for 1 hour at room temperature, and then incubated with antibody diluted to 10 *µ*g/ml in blocking buffer for 2-24 hours at room temperature. Plates are washed three times with PBS/0.05% Tween 20 and bound antibody is then detected with biotinylated goat anti-IgM or IgG followed by alkaline phosphatase conjugated to streptavidin, with p-nitrophenylphosphate as chromogenic substrate. Absorbance of the reaction is measured at 405 nm.

Antibodies are also tested for their reactivity to a panel of protein antigens (human erythrocyte spectrin, bovine myosin heavy chain, mouse hemoglobin, bovine transferrin, bovine vimentin, chicken egg lysozyme, rabbit actin, rabbit myelin basic protein, keyhole limpet hemocyanin) and bovine serum albumin (BSA)-coupled chemical haptens (4-hydroxy-3-nitrophenyl acetyl (NP), phenyloxazolone (PhoX), axophenyltrimethylammonium (TMA), fluorescein (FL), azophenylphosphoryl-choline (PC), azophenylarsonate (Ars), trinitrophenyl acetyl (TNP)). Proteins are used at 5 *µ*g/ml and BSA-coupled haptens are used at 2 *µ*M hapten concentration. Antigens are coated onto polystyrene microtiter plates, reacted with antibody, and the bound antibodies are detected as described for SCH ELISA.

### Tissue Section Staining

Rat pup cerebellum is used as a source of neural tissue for the comparison of antibody staining patterns. Fresh, unfixed, tissue is embedded in 2% low melting point agarose and cut into 300 *µ*M saggital sections on a McIlwain Tissue Chopper. Sections are not fixed and are kept at 4°C or on ice throughout the rest of the procedure. Slices are transferred into 48-well tissue culture plates in HEPES buffered Earles balanced salts (E/H) and blocked for 30 minutes in E/H with 5% BSA. Sections are stained with primary antibody at 10 *µ*g/ml in E/H with 1% BSA for 2-12 hours at 4°C. Sections are washed 3x in E/H and incubated with an appropriate fluorescent secondary antibody in E/H with 1% BSA for 2 hours. Sections are washed 3x in E/H, 1x in PBS and then post-fixed with 4% paraformaldehyde for 30 minutes. Sections are washed 3x with PBS and mounted in 90% glycerin with 2.5% 1,4-diazabicyclo[2.2.2]octane to prevent photobleaching.

### Cultured Oligodendrocyte Staining

Cerebral hemispheres are dissected from P0-P3 Sprague-Dawley rats and the meninges and blood vessels are removed. The tissue is minced and transferred to a 0.25% trypsin solution in calcium and magnesium free HEPES buffered Earles salts (E/H), 10 ml final volume per brain. The tissue is shaken at low rpm at 37°C for 30 minutes and then heat inactivated fetal calf serum is then added to final a final concentration of 10% to inactivate trypsin. MgSO₄ and DNAse I are added (to 0.1 % and 20 *µ*g/ml respectively) and the tissue is shaken for an additional 5 minutes. The cells are washed by centrfugation and resuspended in E/H with DNase I and dissociated by trituration through a glass pipette. Large debris is allowed to settle and the overlying cellular supernatant is washed by centrifugation through a 4% BSA cushion in E/H. The cell pellet is resuspended in culture medium and the cells are plated at 2.5x10⁵ cells per cm² on poly-D-lysine culture plates. Plates are shaken to isolate for oligodendrocyte progenitors at day 9-12. A complete phenotypic spread of oligodendrocytes is present in the culture at this time with progenitors present as a top layer in clusters of recently divided cells. Oligodendrocyte progenitors are isolated by gently shaking th4e cultures, replated on poly-lysine coated cover slips and stimulated to differentiate by removal of growth factors from the culture medium.

Live surface staining is performed at 4°C for 15 minutes on unfixed cells after blocking with PBS and 5 % BSA. Intracellular staining is performed after fixation with 4% paraformaldehyde and permeabilization with 0.1 % Triton X-100. Primary antibodies are detected with fluorescein-conjugated second antibodies. Coverslips are mounted in 90% glycerin with 2.5% 1,4-diazabicyclo[2.2.2]octane to prevent photobleaching and viewed on an epifluorescent microscope.

### Western blotting

Purified TMEV (Njenga et al., 1996) was separated by SDS-PAGE on 15% acrylamide gels. Proteins were transferred to a nitrocellulose membrane by electroblotting. The membrane was blocked with Tris buffered saline containing 5 % non-fat dry milk and 0.05% Tween 20 for 2 hours at room temperature. The membrane was incubated with pooled human IgM, pooled human IgG, IgMs from two patients with Waldenstrom's macroglobulinemia, and rabbit polyclonal anti-TMEV Ab (1:2000) (Njenga et al., 1996) for 4 hours at room temperature. All human Igs were used at the same concentration (10 ug/ml). Bound Igs were detected with biotinylated goat anti-human abs or biotinylated goat anti-rabbit abs (both from Jackson ImmunoResearch Laboratories, Inc., West Grove, PA) and alkaline phosphatase-conjugated streptavidin using 5-bromo-4-chloro-3-indolyl phosphate and nitro blue tetrazorium (BCIP/NBT).

### B. Promotion of Remyelination Using Human Monoclonal Antibodies:

TMEV Induced Demyelination - For the production of TMEV induced demyelination, intracerebral virus injection is performed on 4-6 week old animals that are lightly anesthetized with metofane. Virus is injected using a 27 gauge needle with a Hamilton syringe that delivers a 10 *µ*l volume which contains 2x10⁵ PFU of the Daniel's strain of TMEV. Intracerebral injection results in greater than 98 % incidence of chronic viral infection with demyelination. Chronically infected animals for remyelination experiments are generally 6-8 months post-infection.

### Antibody Treatment Protocol

Animals with chronic demyelination receive intraperitoneal (IP) injections of purified antibodies in phosphate buffered saline. For TMEV infected animals the injection schedule consists of twice weekly injections of 50 *µ*g in 100 ml. The duration of antibody treatment is five weeks (500 *µ*g total dose). Animals are then sacrificed and spinal cord tissue is processed for morphological evaluation as described below. For each different antibody treatment, nine chronically infected, female SJL/J mice are injected with antibody. At the end of the treatment period, six of the animals are perfused and processed for morphometric quantitation of demyelination / remyelination and three are sacrificed for frozen tissue that is used for assessment of axonal integrity. Three separate treatment trials, with reproducible and consistent results, are required for any given antibody before the data is considered significant. PBS and isotype control groups are included as negative controls for each new antibody treatment experiment.

### Morphological Evaluation of Demyelination / Remyelination

At the end of each experiment the spinal cord of each animal will be assessed histologically. Mice are anesthetized with pentobarbital and perfused by intracardiac administration of fixative (phosphate buffered 4% formaldehyde with 1 % glutaraldehyde, pH 7.4). Spinal cords are removed and sectioned coronally into 1 mm blocks, postfixed with osmium, and embedded in araldite. One micron-thick cross-sections are cut from each block and stained with 4% paraphenyldiamine.

This technique is reproducible and allows consistent visualization of myelin sheaths in spinal cord white matter.

Demyelination and remyelination are quantified using a Zeiss digital analysis system (ZIDAS) and camera lucida. For each mouse, ten spinal cord cross sections are examined which span the entire cord from the cervical to the proximal coccygeal spinal column regions. Total area of white matter, area of demyelination, and area of remyelination are determined for each section, and the areas from all ten sections analyzed for a specific mouse are added to provide total areas for each mouse. Areas of demyelination are characterized by large amounts of myelin debris, macrophages engulfing debris, cellular infiltration and naked axons. Oligodendrocyte remyelination is is characterized by areas of axons with abnormally thin myelin sheaths and the absence of Schwann cells. Statistical comparison of the extent of demyelination and remyelination is performed using the Student's t test.

### Lysolecithin Induced Demyelination

For these experiments, 12 weeks old SJL/J mice are anesthetized with sodium pentobarbitol and a dorsal laminectomy is performed in the upper thoracic region of the spinal cord. A 34 guage needle attached to a Hamilton syringe is used to inject 1 *µ*ml of a 1 % solution of lysolecithin directly into the dorsolateral aspect of the cord. Animals are killed on day 21 post injection and the injected region of the spinal cord is removed and processed for morphological evaluation.

As a second model of demyelination, intraspinal injection of lysolecithin was used. Twelve-week-old SJL/J mice were anesthetized by intraperitoneal injection of sodium pentobarbitol (0.08 mg/g). Dorsal laminectomies were performed on the upper thoracic region of the spinal cord and lysolecithin (L-a-lysophosphatidylcholine) (Sigma, St. Louis, MO) was injected as described previously (Pavelko et al., 1998). Briefly, a 34 gauge needle attached to a Hamilton syringe mounted on a stereotactic micromanipulator was used to inject 1 % solution of lysolecithin in sterile PBS (pH 7.4) with Evan's blue added as a marker. The needle was inserted into the dorsolateral part of the spinal cord, 1 ul of lysolecithin solution was injected, and then the needle was slowly withdrawn. The wound was sutured in two layers, and mice were allowed to recover. The day of lysolecithin injection was designated day 0.

Seven days after lysolecithin injection, mice were treated with bolus intraperitoneal injection of human IgM or human IgG (1 mg/injection each). Control mice were treated with bolus intraperitoneal injection of PBS. Three weeks and five weeks after the lysolecithin injection, mice were sacrificed and one (m thick sections were prepared as described in the previous section. The araldite block showing the largest lysolecithin-induced demyelination lesion was used for quantitative analysis. The total area of the lesion was quantitated using a Zeiss interactive digital analysis system. The total number of remyelinated fibers was quantitated using a Nikon microscope/computer analysis system. The data was expressed as number of remyelinated axons/mm² of lesion.

Lysolecithin treated mice are given 50 *µ*g IP injections of antibody on days 0, 3, 7, 10, 14, and 17 after lysolecithin injection. Animals are killed on day 21 after lysolecithin injection. We routinely find statistically significant treatment effects with experimental treatment groups of ten animals. PBS and isotype control groups serve as negative controls.

### C. Mechanism of Action of Remyelination Promoting Human Monoclonal Antibodies:

### Ca2+ Ratiometric Fluorescent Analysis

Mixed primary glial cultures, from day 2-4 postnatal rat pups, are seeded onto poly-D-lysine coated coverslips and cultured for 5-7 days prior to analysis. Fura-2-AM and Pluronic F-127 are mixed 1:1 and added to DMEM (serum free) to yeild 4 mM Fura-2 in solution (Fura-2 loading media).

Coverslips with cells are washed once with DMEM and then incubated in Fura-2 loading media for 60 minutes at 37°C. The cells are then washed 4 times in DMEM. The coverslip is mounted in a recording chamber on an inverted fluorescence microscope connected to a computer controlled data acquisition system which captures digital images of 510 nm fluorescence emission at two different wavelengths of excitation: 340 nm and 380 nm. For each recording, digital images are captured from an individual cell, at 10 second intervals over 600-800 seconds. Relative internal Ca²⁺ concentration is calculated as the ratio of 340 nm/380 nm fluorescence. All recordings are made at 37°C in 1 ml DMEM.

Test antibody is introduced by adding 50 ml of concentrated (60 mg/ml in PBS) antibody stock solution to the recording chamber to yield a final concentration of 3 mg/ml. After recording the effects of the addition of test antibodies, 50 ml of of a calcium ionophore stock (200 mM Br-A23187 in PBS) is added to the recording chamber to yield a final concentration of 10 mM.

### DISCUSSION

Normal human immunoglobulin (Ig), especially IgG, administered intravenously (IVIg) has been shown to be effective in treating various autoimmune neurological diseases including Guillain-Barré syndrome (van der Mech et al., 1992), chronic idiopathic demyelinating neuropathies (van Doom et al., 1991), multifocal motor neuropathy (Chaudhry et al., 1993), polymyositis (Cherin et al., 1991), and myasthenia gravis (Edan and Landgraf, 1994). The mechanisms by which administered Ig acts is unclear. Some investigators have also suggested that this therapy may be effective in T cell-mediated autoimmune CNS diseases such as multiple sclerosis (MS)(van Engelen et al., 1992; Achiron et al., 1992; Fazekas et al., 1997; Achiron et al., 1998; Sorensen et al., 1998).

We have used Theiler's murine encephalomyelitis virus (TMEV)-induced demyelination as a model to develop novel treatments for MS. When this picornavirus is inoculated intracerebrally in susceptible strains of mice, TMEV induces immune-mediated progressive CNS demyelination which is clinically and pathologically similar to MS. We showed previously that multiple mouse IgM( monoclonal antibodies (mAbs) directed against normal CNS antigens promote CNS remyelination following TMEV-induced demyelination (Miller et al., 1994; Asakura et al., 1998). The prototypic antibody, designated SCH94.03, was also shown to enhance the rate of spontaneous CNS remyelination following lysolecithin-induced demyelination (Pavelko, et al., 1998) and decrease the severity and frequency of relapses in a relapsing model of experimental autoimmune encephalomyelitis (EAE). The common features of these remyelinating-promoting IgM mAbs are that they react to surface antigens on oligodendrocytes and have phenotypic and genotypic features of natural autoantibodies (Miller and Rodriguez, 1995; Asakura et al., 1998). Natural autoantibodies have wide spectrum of reactivities with self and non-self antigens. These antibodies represent a major fraction of the normal circulating IgM repertoire. Though.their physiological function is unknown, the beneficial effects of natural autoantibodies have been reported in various autoimmune disease models including myasthenia gravis, systemic lupus erythmatosus, and non-obese diabetes (Sundblad et al., 1989; Hentati et al., 1994; Andersson et al., 1991, 1994).

IVIg is purified from human plasma pools of 3000 to 10000 healthy donors and contains more than 95% IgG and a negligible amounts of IgM (Dalakas, 1997). Based on our previous observations we hypothesized that human IgM from healthy donors, which is enriched in natural autoantibodies, would be a more effective treatment for demyelinating disease than conventional IVIg. To test this hypothesis we treated chronically TMEV-infected mice with pooled human IgM obtained from over 2,500 healthy donors and examined for CNS remyelination.

In this study we demonstrated that treatment with pooled human IgM from healthy donors resulted in significantly enhanced remyelination by oligodendrocytes in TMEV-infected mice as compared to the treatment with pooled human IgG, or PBS. We confirmed by ELISA and immunocytochemistry that pooled human IgM contains a population of polyreactive natural autoantibodies to proteins and haptens. This is the first demonstration that polyclonal human IgM promotes CNS remyelination in models of demyelinating disease, thus raising the possibility that IgM from healthy donors may be more effective to treat human inflammatory demyelinating diseases than conventional pooled human IgG.

To our knowledge pooled human IgM has never been tested in MS, even though it has been shown to be safe and effective in severe infections and immunodeficiency.

Natural autoantibodies are a major fraction of the IgM repertoire. In mice natural autoantibodies are exclusively IgM, whereas in humans natural autoantibodies are also of the IgG isotypes although with much less frequency. To date, the only mAbs which have been shown to enhance remyelination have been oligodendrocyte-reactive IgM( mAbs, which have genotypic and phenotypic features of natural autoantibodies, (Asakura et al. 1998).

In conclusion, we have demonstrated that a logical screening technique can be used to identify human monoclonal antibodies that have the potential to promote remyelination in model systems of demyelination. Properties of the antibody, such as CNS specificity, the ability to recognize antigens present on oligodendrocytes and a strong binding to spinal cord homogenate, combined, can predict which antibodies are the best candidates to test for remyelination *in vivo.* Many of these monoclonal antibodies bind well to human CNS, giving reason to hope that some may be useful as a therapy to successfully treat human disease.

The following is an alphabetical list of the references referred to in this Example.
Asakura, K., D.J. Miller, K. Murray, R. Bansal, S.E. Pfeiffer, and M. Rodriguez. 1996a. Monoclonal autoantibody SCH94.03, which promotes central nervous system remyelination, recognizes an antigen on the surface of oligodendrocytes. J Neurosci Res 43:273-281.
Asakura, K., D.J. Miller, R.J. Pogulis, L.R. Pease, and M. Rodriguez. 1996b. Oligodendrocyte-reactive O1, O4, and HNK1 monoclonal antibodies are encoded by germline immunoglobulin genes. Mol.Brain Res. 34:282-293.
Asakura, K., D.J. Miller, L.R. Pease, and M. Rodriguez. 1998. Targeting of IgMkappa antibodies to oligodendrocytes promotes CNS remyelination. Journal of Neuroscience 18:7700-7708.
Blakemore, W.F., R.A. Eames, K.J. Smith, and W.I. McDonald. 1977. Remyelination in the spinal cord of the cat following intraspinal injections of lysolecithin. J.Neurol.Sci. 33:31-43.
Crang, A.J. and W.F. Blakemore. 1991. Remyelination of demyelinated rat axons by transplanted mouse oligodendrocytes. GLIA. 4:305-313.
Dubois-Dalcq, M. and R. Armstrong. 1990. The cellular and molecular events of central nervous system remyelination. Bioessays 12:569-576.
Franklin, R.J., A.J. Crang, and W.F. Blakemore. 1991. Transplanted type-1 astrocytes facilitate repair of demyelinating lesions by host oligodendrocytes in adult rat spinal cord. J.Neurocytol. 20:420-430.
Groves, A.K., S.C. Barnett, R.J. Franklin, A.J. Crang, M. Mayer, W.F. Blakemore, and M. Noble. 1993. Repair of demyelinated lesions by transplantation of purified O- 2A progenitor cells Nature. 362:453-455.
Jeffery, N.D. and W.F. Blakemore. 1995. Remyelination of mouse spinal cord axons demyelinated by local injection of lysolecithin. Journal of Neurocytology 24:775-781.
Lang, W., M. Rodriguez, V.A. Lennon and P.W. Lampert, 1984. Demyeliantion and remyelination in murine viral encephalitis. Ann N.Y. Acad. Sci. B436: 98-102.
Ludwin, S.K. 1981. Pathology of demyelination and remyelination Adv.Neurol. 31: 123-168.
Ludwin, S.K. 1987. Remyelination in demyelinating diseases of the central nervous system. Crit Rev Neurobiol. 3: 1-28.
Ludwin, S.K. 1989. Evolving concepts and issues in remyelination. Dev.Neurosci. 11:140-148.
Miller, D.J., K.S. Sanborn, J.A. Katzman and M. Rodriguez, 1994. Monoclonal antibodies promote central nervous system repair in an anaimal model of multiple sclerosis J. Neurosci 14: 6230-6238.
Miller, D.J. and M. Rodriguez. 1995. Spontaneous and induced remyelination in multiple sclerosis and the Theiler's virus model of central nervous system demyelination. [Review] [119 refs]. Microscopy Research & Technique 32:230-245.
Miller, D.J., K. Asakura, and M. Rodriguez. 1995. Experimental strategies to promote central nervous system remyelination in multiple sclerosis: insights gained from the Theiler's virus model system. J Neurosci Res. 41:291-296.
Miller, D.J. and M. Rodriguez. 1995. A monoclonal autoantibody that promotes central nervous system remyelination in a model of multiple sclerosis is a natural autoantibody encoded by germline immunoglobulin genes. J Immunol 154:2460-2469.
Miller, D.J., C. Rivera-Quinones, M.K. Njenga, J. Leibowitz, and M. Rodriguez. 1995a. Spontaneous CNS remyelination in beta(2) macroglobulin-deficient mice following virus-induced demyelination. J Neurosci 1545:8345-8352.
Miller, D.J., K. Asakura and M. Rodriguez, 1995b. Experimental strategies to promote central nervous system remyelination in multiple sclerosis: insights gained from the Theiler's virus model system J. Neurosci. Res. 56: 65-73.
Miller, D.J., J.J. Bright, S. Sriram, and M. Rodriguez. 1997. Successful treatment of established relapsing experimental autoimmune encephalomyelitis in mice with a monclonal natural autoantibody. Journal of Neuroimmunology 75:204-209.
Pavelko, K.D., B.G. van Ehgelen and M. Rodriguez 1998. Acceleration in the rate of CNS remyelination in lyso lecithin-induced demyelination. J. Neurosci. 18: 2498-2505.
Prineas, J.W. and F. Connell. 1979. Remyelination in multiple sclerosis. Ann. Neurol. 5:22-31.
Prineas, J.W., R.O. Barnard, E.E. Kwon, L.R. Sharer, and E.S. Cho. 1993. Multiple sclerosis: remyelination of nascent lesions. Ann Neurol 33:137-151.
Raine, C.S. and E. Wu. 1993. Multiple sclerosis: remyelination in acute lesions. J. Neuropathol.Exp.Neurol. 52:199-204.
Rivera-Quinones, C., D.B. McGavern, J.D. Schmelzer, S.F. Hunter, P.A. Low, and M. Rodriguez. 1998. Absence of neurological deficits following extensive demyelination in a class I-deficient murine model of multiple sclerosis. Nature Med 4:187-193.
Rodriguez, M., V.A. Lennon, E.N. Benveniste, and J.E. Merrill. 1987. Remyelination by oligodendrocytes stimulated by antiserum to spinal cord. J. Neuropathol. Exp. Neurol. 46:84-95.
Rodriguez, M., E. Oleszak, and J. Leibowitz. 1987a. Theiler's murine encephalomyelitis: a model of demyelination and persistence of virus. Crit.Rev.Immunol. 7:325-365.
Rodriguez, M. and V.A. Lennon. 1990. Immunoglobulins promote remyelination in the central nervous system. Ann.Neurol. 27:12-17.
Rodriguez, M. 1991. Immunoglobulins stimulate central nervous system remyelination: electron microscopic and morphometric analysis of proliferating cells. Lab Invest. 64:358-370.
Rodriguez, M. and B. Scheithauer. 1994. Ultrastructure of multiple sclerosis. Ultrastruct Pathol 18:3-13.
Smith, K.J., W.F. Blakemore, and W.I. McDonald. 1981. The restoration of conduction by central remyelination. Brain. 104:383-404.
Traugott, U., S.H. Stone, and C.S. Raine. 1982. Chronic relapsing experimental autoimmune encephalomyelitis. treatment with combinations of myelin components promotes clinical and structural recovery. J.Neurol.Sci. 56:65-73.

### Example 2

### SCREENING FOR EPITOPE MIMIC PEPTIDES WITH AN AUTOANTIBODY

In this example, the identification and preparation of peptides which mimic the recognized antigens, or portions thereof, corresponding to the autoantibodies of the invention is described. As described earlier herein, such peptides could serve as vaccines to elicit enhanced immune response to conditions indicated to be favorably responsive to increased circulating levels of antibodies.

An exemplary strategy for the identification of peptide mimics would be to search for peptides specifically binding, for example to the HNK-1 antibody, a mouse autoantibody demonstrated to be capable of inducing remyelination. The HNK-1 epitope antigen is a carbohydrate. The HNK-1 epitope is expressed predominantly on glycolipids and glycoproteins from nervous tissue (McGarry et al., (1983) Nature 306:376-378; Ilyas et al., (1984) Biochem. Biophys. Res. Comm. 122:1206-1211; Kruse et al., (1984) Nature 311:153-155; Yuen et al., (1997) J. Biol. Chem. 272:8924-8931). The structure which reacts with HNK-1 antibody was first described by Chou and Jungalwala for the major antigenic glycolipid present in human peripheral nerve. The compostion, sugar linkage, configuration and position of the sulfate group, were characterised as sulfate-3 GlcAβ (1-3) Galβ (1-4) GlcNAcβ (1-3) GalNAcβ (1-3) Galβ (1-4) Glcβ(1-1)-ceramide for SGGL-1 and as sulfate-3 GlcAβ (1-3) Galβ (1-4) GlcNAcβ (1-3) Galβ (1-4) GlcNAcβ (1-3) Galβ (1-4) Glcβ (1-1)-ceramide for SGGL-2. (Chou et al, 1986).

Screening phage-displayed random peptide libraries offers a rich source of molecular diversity and represents a powerful means of identifying peptide ligands that bind a receptor molecule of interest. Phage expressing binding peptides are selected by affinity purification with the target of interest. This system allows a large number of phage to be screened at one time. Since each infectious phage encodes a random sequence expressed on its surface, a particular phage, when recovered from an affinity matrix, can be amplified by another round of infection. Thus, selector molecules immobilized on a solid support can be used to select peptides that bind to them. This procedure reveals a number of peptides that bind to the selector and that often display a common consensus amino acid sequence. Biological amplification of selected ibrary members and sequencing allows the determination of the primary structure of the peptide(s).

Peptide ligand identified by phage display frequently interact with natural binding site(s) on the target molecule, and often resemble the target's natural ligand(s). Although this system has often been used to identify peptide epitopes recognized by antibodies, it has also been successfully used to find peptide mimics of carbohydrate molecules. Work directed towards using peptide mimics in place of carbohydrate antigens has been reviewed by Kieber-Emmons et al, 1998). The demonstrated ability of a peptide to mimic a carbohydrate determinant indicates that, although mimicry is accomplished using amino acids in place of sugars, the specificity pattern can be reproduced.

A first screening was done with the amplified starting library of 15 mer peptides. Several clones positive in binding to the HNK-1 antibody were found. In the initial screening with HNK-1, bound phage were eluted by pH shift, so that there was no differentiation between specifically and non-specifically bound phage. Therefore a screening was carried out wherein HNK-1 antibody is biotinylated with a coupling agent incorporating a disulfide bridge. The biotinylated antibody is pre-reacted with the streptavidin-coated tube, unbound antibody is washed off, and the immunotube is used for screening. Alternatively, phage are reacted with the biotinylated antibody in solution, and then the biotinylated complex is allowed to react with an immunotube coated with streptavidin. In either case, after washing away unbound phage, the bound phage are eluted by addition of dithiothreitol, which releases the antibody and the attached phage (Griffiths et al, 1994). Furthermore, these screenings were done in the presence of mouse serum (12.5%). This provides a large excess of mouse IgM over the HNK-1 antibody, so that non-specific binding to the HNK-1 antibody should be suppressed.

In some cases, when phage in solution were allowed to react with "pre-immobilized" antibody, a rise was obtained in the number of phage bound after the third or the fourth round of selection. The clones tested bound to total mouse IgM as well. In a final experiment various procedures were compared in parallel: Phage were allowed to bind either to HNK-1 coated immunotube or to biotinylated HNK-1 in solution, and in the presence or absence of mouse serum. An enrichment was observed using the pre-coated antibody, but the selected clones again bound to total mouse IgM, although they also bound HNK-1. It is interesting to note that the selected phage were also reactive to L2-412 antibody, which recognizes the same carbohydrate as HNK-1, although HNK-1 requires a terminal sulfate group, while L2-412 antibody recognizes the carbohydrate with or without a sulfate group.

### MATERIALS AND METHODS

### Materials

A 15-mer peptide library and E.coli K91 Kan cells may be used. The 15-mer library was constructed in the vector fUSE5, a derivative of the filamentous phage fd-tet (Scott et al, 1990). This vector carries a tetracycline resistance gene allowing for selection. The filamentous phage do not kill their host; thus the infected cells become tetracycline resistant, continue to grow and secrete progeny particles. The *E.coli* strain K91Kan is a lambda⁻ derivative of K38 (Lyons et al, 1972), has a chromosomal genotype *thi* and carries a kanamycin-resistance gene (*mkh*) (Smith et al, 1993; Yu et al, 1996). Peptides and peptide (10 mg) coupled to SPDP-activated BSA (60 mg) via C-terminal cysteine, may be obtained e.g. from ANAWA AG, 8602 Wangen, Switzerland. Tetracycline and Kanamycin may be purchased from Sigma. L2/HNK-1 glycolipids were purified from beef cauda equina by B. Becker in our laboratory. Sulfated sugars, SO₃-GlcA-Gal-allyl, were kindly provided by N. Nifant'ev, Zelinsky Institutre of Organic Chemistry, Russian Academy of Sciences, Moscow.

### Antibodies

Characterization and purification of the monoclonal antibody (mAb L2-412), raised in rats and recognizing the HNK-1 carbohydrate has been described by Noronha, A. et al., Brain Res. 385, 237-244 (1986)). The L2-412 antibody has been deposited with the DSMZ - Deutsche Sammlung Von Mikroorganismen und Zellkulturen GmbH, Mascheroder Weg 1b, D-38124 Braunschweig, Germany, under the Budapest Treaty, and is designated. HNK-1 antibody is available as TIB200 from the American Type Culture Collection (ATCC). Polyclonal rat IgG and HRP-Streptavidin were obtained from Sigma (USA). HRP/anti-M13 polyclonal antibody was purchased from Pharmacia Biotech. Horseradish peroxidase (HRP)-conjugated secondary antibody directed against rat IgG was obtained from Jackson Immunoresearch.

### Amplifying the starting library

The primary library encoding the 15mer peptides was amplified based on the Smith procedure (Smith et al, 1992) as follows:

The night before the cells were needed, 2 ml of LB medium (g/L Bacto-Tryptone, 5 g/L NAcl, 5 g/L yeast extract), containing 100 *µ*g/ml kanamycin, were inoculated with K91Kan cells and shaken overnight at 37°C. A 1L flask containing 100 ml of Terrific Broth was prepared (12 g Bacto-Tryptone, 24 g yeast extract, 5.04 g glycerol (4ml) added to 900 ml of water and autoclaved in 90 ml portions; 10 ml of potassium phosphate buffer (0.17M KH₂PO₄, 0.72M K₂HPO₄, no pH adjustment required) were added to each 90 ml portion bef ore use).

The 100 ml Terrific Broth were inoculated with 1 ml of the overnight culture of K91kan cells and shaken vigorously until the OD₆₀₀ of a 1:10 dilution reached 0.2. Shaking was then slowed down for 10 min to allow F-pili to regenerate and 10 *µ*l of the starting library was added to the flask; slow shaking was continued to allow for adsorption. The culture was then transferred to 1 L of LB containing 0.22 *µ*g/ml tetracycline and allowed to shake vigorously for 35 minutes at 37°C. The tetracycline concentration was adjusted to 20 *µ*g/ml, and an aliquot was taken for determination of the titer. The phage were titered (recovered titer) by plating infected cells on tetracycline medium and counting the number of tetracycline resistant colonies. An infectious unit defined in this way is called a transforming unit (TU) and the infectivity is the ratio of number of TU's to number of physical particles. Typically, an aliquot of 50 *µ*l of the culture was removed and diluted with LB containing 0.2 *µ*g/ml tetracycline (dilution range was 10³-10⁵). An aliquot of 200 *µ*l of each dilution were spread on an agar-plate containing 40 *µ*g/ml tetracycline and 100 *µ*g/ml kanamycin, incubated overnight at 37°C. The colonies were counted on the next day. At this stage, the titer of tetracycline resistant colonies should be about 10⁷/ml. The remainder of the culture was shaken vigorously overnight.

The next morning the doubly cleared supernatant obtained after 2 steps of centrifugation (4000 x g, 10 min, 4°C and 10'500 x g, GSA, 10 min, 4°C) was precipitated overnight at 4°C by adding 0.15 volume of PEG/NaCl solution (16.7% polyethylene glycol in 3.3 M NaCl solution). The precipitated phages collected after centrifugation (10'500 x g, GSA, 40 min, 4°C) were dissolved in 10 ml of TBS (50 mM Tris-HCl pH7.5, 150 mM NaCl) and a second precipitation was carried out by adding 0.15 volume of the PEG/NaCl solution to the phage suspension and incubating for 1 hr on ice. At this stage, a heavy precipitate should be evident.

The pellet obtained after centrifugation (14'500 x g, SA600, 10 min, 4°C) was redissolved in 10 ml TBS and transferred into a tared vessel containing 4.83 g CsCl. The vessel was retared and TBS was added to a net weight of 10.75 g. This should give 12 ml of a 31 % w/v solution of CsCl (density 1.30 g/ml); the solution was centrifuged 48 hrs at 150'000 x g at 5°C in a SW41 rotor (Beckman). With the help of a strong visible light source, a faint bluish non-flocculent band (containing the amplified phages) was visible above a narrow flocculent opaque white band (probably deriving from PEG). The phage band was collected by first aspirating slowly the fluid overlying the phage band and then, using a pipette, the phage band was withdrawn avoiding as much as possible the flocculent band underneath. The phage band was then delivered to a 26 ml polycarbonate centrifuge bottle, which was filled to the shoulder with TBS and centrifuged in a Ti70 rotor (279'000 x g, 4h, 5°C) and resuspended in 2 ml TBS per 1 L of culture. Phages can be stably stored in this form in a refrigerator.

The amplified library was then titered (final titer) as follows: several dilutions of phage were prepared in TBS/gelatine (0.1 g gelatin in 100 ml TBS) covering the dilution range from 10⁷ to 10¹⁰. Then 10 *µ*l of each of these dilutions were used to infect 10 *µ*l of K91kan cells prepared as described at the beginning of this section and each dilution mixture was incubated 15 min at room temperature (RT) to allow phage to infect the concentrated cells. One ml of LB containing 0.2 *µ*g/ml tetracycline was added and incubated 30 min at 37°C in a shaker-incubator. The infected cells were then spread (200 *µ*l) on an agar plate containing 40 *µ*g/ml tetracycline and 100 *µ*g/ml kanamycin as described above (recovered titer).

### Screening Procedure

### A. Direct Binding

The phage library was panned using Immunotubes (Nunc., Maxisorb) coated with mAbL₂-412. The tubes were coated by incubating overnight at 4°C with antibodyL2-412 at 10 *µ*g/ml protein in PBS (1 ml total volume) for the first round and 1 *µ*g/ml for the second and third round of screening. After blocking 2 hours with Blotto (5 % non-fat dry milk, 0.05%(v/v) Tween 20 in PBS) at 4° C, 10¹¹ transforming units (in 250 *µ*l volume) of the phage library per immunotube were allowed to bind 1 hour at 37° C in a rotating chamber. For the second and third rounds, the phages were preincubated 1 hour with 100 *µ*g/ml of rat IgG before being added to the immunotube, in order to decrease the number of non-specific binders. After recovery of the unbound phages (from which the negative control phage was chosen), the tubes were washed 10 times with PBS-0.05% (v/v) Tween 20 and eluted with 0.1 M Glycine pH 2.2 (0.5-1 ml total volume), 10 min. at 4° C. Eluted phages were neutralized with 1.5M Tris pH9 and then used to infect 0.5-1 ml of log phase *E. coli* K91 Kan cells 15 min at room temperature. The infected bacteria were transferred to 20 ml of LB containing 0.2 *µ*g/ml tetracycline, and after removing an aliquot for determination of the titer (recovered titer), allowed to grow overnight as described in the previous section. The amplified eluate was then twice centrifuged (10 min, 3600 x g and 10 min, 14'500 x g, SA600) and the final supernatant was precipitated with 0.15 volume of PEG/NaCl overnight at 4°C. The phage was pelleted (15 min. 14'500 x g, SA600) and dissolved in 1 ml PBS by pipetting and vortexing, microcentrifuged 1 min. to pellet insoluble matter, and PEG-precipitated again for at least 1 hr at 4°C. A heavy precipitate should be visible at this stage. The pellet obtained after 10 min. microcentrifugation was finally dissolved in 200 *µ*l of PBS containing 0.02% azide. This amplified eluate can be stored and kept at 4°C. The library was subjected to three rounds of amplification and selection.

The same procedure was used for the HNK-1 screening with HNK-1 antibody, except that a 100-fold excess of mouse lgM was included to decrease non-specific binding.

The phage were titered (final titer) as described. The colonies were counted on the next day and the yield of the screening was calculated by dividing the recovered titer by the titer (input) of the previous round.

### B. Screening with biotinylated antibody

Two procedures were used to accomplish this screening, both following protocols of G. Smith (unpublished protocols). The HNK-1 antibody was biotinylated as described below using NHS-SS-biotin. NHS-SS-Biotin links the biotin to the protein via a disulfide bridge, in order to allow the biotin group to be subsequently removed by incubation with dithiothreitol (DTT). TheL2-412 antibody was similarly biotinylated as described below. In procedure A, the biotinylated antibody is first allowed to bind to a streptavidin coated immunotube, which is then subsequently used to pan the phage input. In procedure B, the biotinylated antibody is preincubated with the phage in solution, and the reaction mixture is allowed to bind (a few minutes) to the streptavidin-coated immunotube.

In procedure A, the immunotubes were coated with 10 *µ*g/ml streptavidin in PBS, 1 ml total volume (wet the entire surface of the tube), overnight at 4°C on a rotator. Streptavidin was discarded and the tube was filled with blocking solution, PBS containing 0.5 % (w/v) BSA, for 2 hrs at 4°C. After washing 6 times with PBS-0.05% (v/v) Tween 20 (PBS-T), the biotinylated antibody was added. Typically, 3 *µ*g of the biotinylated HNK-1, or 5 *µ*g of the biotinylatedL2-412 antibody were added in 400 *µ*l of the blocking solution. The antibody was allowed to bind for at least 2 hrs (or overnight) at 4°C on the rotator. After washing 6 times with PBS-T, 10¹⁰ phages from the 15-mer starting library, in 400 *µ*l of blocking solution, were allowed to bind to the respective antibody-coated immunotube for 4 hr at 4°C on the rotator. In procedure B, during coating of the immunotubes 10¹⁰ phage were preincubated overnight with 3 or 5 *µ*g of the biotinylated HNK-1 orL2-412 antibody, respectively. The biotinylated antibody was then allowed to bind to the coated immunotube for 10 minutes at 4°C on the rotator. In both procedures, the tubes were then washed 10 times, then phage-antibody complexes were eluted with 20 mM DTT (0.5 ml volume) in PBS 1-5 min. at room temperature. Amplification and titering were performed as described above. The library was subjected to four rounds of amplification and selection.

### ELISA Screening

### A. Direct Binding for Detection of Positive Clones

Individual colonies resistant to tetracycline and kanamycin.were grown in LB containing 20 *µ*g/ml tetracycline in 96-wells plates (Nunc) overnight at 37° C (300*µ*l/well), then centrifuged 10 minutes at 3000 rpm in Jouan centrifuge and the supernatant (100 *µ*l) was incubated for 2 hr in another 96-well plate previously coated with mAb_{L2}-412 (100*µ*l, *µ*g/ml overnight at 4° C) and blocked by incubation for 2 hours with PBS-0.5% (w/v) BSA. After washing 5 times, the binding of the phages was detected by incubation with HRP-conjugated anti-M13 antibody (Pharmacia, Biotech.) for 1 hour at a dilution of 1:2000. The peroxidase reaction was started by the addition of 100*µ*l developer containing 0.01 % hydrogen peroxide and 0.1 % (w/v) 2,2'-azino-bis(3-ethylbenzthiazoline-6-sulfonic acid)-diammonium salt (ABTS, Boehringer Mannheim) in HRP buffer (0.1M sodium acetate, 0.05M NaH₂PO₄, pH adjusted to 4.2 with acetic acid). The absorbance of the colored reaction product was determined at 405 nm in a Multiscan TitertekPlus (Flow, Switzerland). In parallel, each clone was also tested on 96-well plates coated with rat IgG, (100 *µ*l, 1*µ*g/ml in PBS and identically blocked for 2 hours). Bacteria producing the selected binding clones (named positive phage), that were positive binders for the mAb_{L2}-412 but did not bind to rat IgG were streaked on an agar plate containing LB medium with 40 *µ*g/ml tetracycline and 100*µ*g/ml kanamycin. Two individual colonies were picked and re-assayed for positivity towards mAb L2-412. Positive single colonies were stored in 40% glycerol at -80° C.

### B. Competition Binding

Microtiter plates (Nunc) were coated with theL2/HNK-1 glycolipids (50*µ*l, 1 *µ*g/ml, dissolved in EtOH) and allowed to dry overnight. While blocking the wells for 2 hours with 0.5% (w/v) fatty-acid-free BSA in PBS, a limiting concentration of_{L2}-412, previously determined, was pre-incubated with successive 2-fold dilutions of the inhibitor, starting at a concentration of 2.2 mM for the free peptide, 5mM for the SO₃ sugar and 10¹² positive and negative phages (the negative phages were cloned from the unbound fraction of the first round of screening). The pre-incubated mixture was then added to the well in 100*µ*l and incubated for 1 hour at RT. After washing 5 times with PBS-0/05% (v/v) Tween 20, the binding of mAb L2-412 was detected by incubation with HRP-conjugated goat anti-rat IgG for 1 hour, followed by the color reaction described earlier. The percentage of inhibition of the binding of mAb L2-412 to the substrate in the presence of the inhibitor was calculated with reference to the control value obtained in the absence of inhibitor (0% of inhibition).

### C. Inhibition of Binding

Microtiterplates were coated overnight at 4° C with laminin (Gibco/BRL), (10 *µ*g/ml, 100*µ*l), or mAb_{L2}-412 (1 *µ*g/ml, 100*µ*l) in PBS. All the following reaction steps were carried out at room temperature. After blocking with PBS + 0.5% (w/v) BSA, 50 *µ*l of successive 2-fold dilutions of peptide coupled to BSA (ANAWA Ag, Switzerland) starting at a concentration of 30*µ*M was added for 1-2 hours at RT. Then a limiting number of phages bearing the peptide of interest, previously determined, was added and incubated for another hour. The bound phages were detected with HRP/anti-M13 antibody as described in the ELISA screening section. The analogous experiment was done with immobilized L2-412 instead of laminin, the peptide coupled to BSA competing with the binding of positive phages to the antibody L2-412.

### D. Direct Binding to Laminin

Microtiter plates were coated with 100*µ*l of mAb L2-412 or laminin as described above and 100*µ*l of biotinylated peptide coupled to BSA was added starting at a concentration of 30 *µ*M, incubated 2 hours at room temperature, and detected with HRP-streptavidin.

### DNA Sequencing

Positive clones, toothpicked from frozen glycerol stocks, were grown overnight at 37° C in LB containing 20 *µ*g/ml tetracycline. Single stranded DNA was purified as described by G. Smith (1992) using the double-spin method, sequenced with the Thermo Sequenase cycle sequencing kit (Amersham), and loaded on an automated sequencer (B10 Genetic Analyzer, Applied Biosystems Inc.).

### Biotinylation

Biotinylation of the HNK-1 antibody, BSA and the peptide coupled to BSA was done using Sulfo-NHS-biotin (Pierce) according to the manufacturer's instructions. A molar ratio of 10 to 1 was used for the antibody and 5 to 1 for BSA or the peptides coupled to BSA. The biotinylated product was dialysed overnight against PBS at 4°C.

### Neurite Outgrowth Experiments and Culture

### Preparation of Motor Neurons

Cover slips were sterilized by baking them overnight at 160°C and coated by an overnight incubation with polyornithine (Sigma, 1.5 *µ*g/ml in water) at 4°C. The cover slips were then washed 3 times with water and further coated with test substances as follows: 1) The BSA-peptide conjugates were dissolved at 100 *µ*g/ml in PBS, sonicated 1 min with a table sonicator and centrifuges in a microfuge for 20 min at maximum speed. The protein concentration of the supernatant was determined each time by the method of Bradford (Bradford et al, 1976). Then 120 *µ*l complex was mixed with 280 *µ*l of collagen solution (20 *µ*g/ml collagen in PBS) and 100 *µ*l were applied on each cover slip overnight at 4°C; 2) As a negative control, untreated BSA was used in place of the peptide-BSA complex; 3) The glycolipids carrying the L2/HNK-1 carbohydrate were dissolved in ethanol at a concentration of 10 *µ*g/ml, and 80 *µ*l were added to 1 ml of the collagen solution described above. A volume of 100 *µ*l was used for coating. Cover slips were placed in quadruplicate in a 24-well plate (NUNC), and finally washed 3 times before the cells were plated (the cover slips were never allowed to dry).

Motor neuronal cells were prepared as described by Arakawa (1990) from spinal cord of 6-day old chick embryos dissociated in 1 ml of ice cold solution containing 0.05% DNAse 1 (Sigma), 0.1% BSA in L-15 medium (Life Technologies). Cells were layered on 2 ml of 6.8% Metrizamide (Fluka) in L-15 and centrifuged 15 minutes at 500 x g, 4° C. Cells collected from the Metrizamide/medium interface were diluted in 5 ml L-15 and loaded on a 4 ml cushion of BSA (4% BSA in L-15) and centrifuged 10 minutes at 300 x g, 4° C. The pellet was resuspended in 0.5-1 ml of complete medium ((22 mM NaHCO₃, 22 mM glucose, 1% of penicillin and streptomycin (Gibco) in L-15 supplemented with 1% N2 supplement (Gibco) and 15 µg/ml chicken muscle extract (3.5 mg/ml). 30,000 cells were plated on poly-ornithine/collagen coated cover slips in the presence or absence of the peptide coupled to BSA and incubated in a humidified chamber at 37° C and 5% CO₂, The length and number of neurites were measured and counted for isolated neurons that were not in contact with other cells and with at least one process that was as long as the diameter of the cell body after 24 hours of culture.

### Preparation and culture of dorsal root ganglion neurons

The cover slips were prepared identically as for the experiments with motor neurons.

Dorsal root ganglia neurons were isolated from embryonic-day 11 chicken eggs. The ganglia were transferred into 1 ml of digestion solution (0.05% Trypsin, 0.01% DNAse 1 in HBSS medium) and incubated 15 min. at 37°C with resuspending every 2-5 min. The ganglia were then dissociated in 1 ml of ice cold dissociation solution (0.05% DNAse 1, 0.1% BSA, in L15 medium), loaded on 3 ml of a 4% BSA cushion in a 15 ml Falcon tube and centrifuged at 4°C, 600 x g for 20 min. The cells were resuspended in 0.5 ml of the complete medium described in the previous section. 20,000 cells were added to wells containing one cover slip, and allowed to grow for 18 hrs in a humidified chamber at 37°C and 5% CO₂. Fixing and analysis of neurite outgrowth was performed as described in the preceding section.

### Immunohistology and Immunocytology

### Immunohistology

Cryosections of femoral nerve from a 4-month-old mouse were used to look for binding of peptide-BSA complex. The sections were treated for 1 hr with 1% H₂O₂, 0.5% bovine serum albumin (BSA), and 10% goat serum in PBS, in order to reduce the endogenous peroxidase activity. The sections were then incubated overnight at 4°C with peptide-BSA complex or BSA (1 mg/ml in PBS, 150 µl/cover slips), and then washed 4 times with PBS-0.01% Tween 20. For detection, anti-BSA antibody (Sigma, 1:16 dilution, 150 µl/cover slips) was added and incubated overnight at 4°C. HRP-coupled goat anti rabbit serum was added (1:2000), for 1 hr in a volume of 150 µl per cover slip. The color reaction was developed using a 5% dilution of a 4 mg/ml stock solution of 9-amino-3-ethylcarbazol (AEC, Fluka) in N,N'-dimethylformamide in 0.1 M sodium acetate buffer, pH 4.8, containing 0.1% H₂O₂.L2-412 antibody and HRP-coupled goat anti-rat antibody were used for the positive control. A similar experiment was performed using biotinylated BSA-peptide conjugate. A concentration of 50 µg/ml was used for the overnight incubation and HRP-coupled streptavidin (1:2000) was added for 1 hr. The color reaction was developed as described above.

### Immunocytology

Cover slips were coated with polyornithine (1.5 µg/ml) then with collagen (20 µg/ml,) and 40,000 cells were allowed to grow for 40 hrs at 37°C under 5% CO₂ as described above. The fixed cover slips were then blocked in 5% non-fat dry milk powder in PBS for 2 hrs. After extensive washing with PBS-0.05% Tween-20, biotinylated BSA-peptide conjugate was added at a concentration of 50 µg/ml for 4 hrs. After another six times wash steps, detection was done using HRP-coupled streptavidin, 1:500, for 1 hr. Color detection was as described above for immunohistology. The fixed neurons were photographed at 40 X magnification. The images presented were processed for enhanced color rendition using Adobe Photoshop.

The following is an alphabetical list of the references referred to in this Example.
Chou, D., and Jungalwala, F. J. Biol. Chem. 268, 21727-21733 (1993).
Chou, D.K., et al., J. Biol. Chem. 261, 11717-25 (1986).
Griffiths, A. et al. (1994) EMBO J. 13:3245-3260.
Kieber-Emmons, T. Immunologic Research 17, 95-108 (1998).
Lyons, L. and Zinder, N. (1972) Virology 49:45-60.
Scott, J.K. and Smith, G.P. (1990) Science 249:386-390.
Smith G.P. and Smith, J.K. (1993) Methods Enzymol. 217:228-257.
Yu, J. and Smith, G. (1996) Methods Enzymol. 267:3-27.

### EXAMPLE 3

### HUMAN MONOCLONAL ANTIBODIES REACTIVE TO OLIGODENDROCYTES PROMOTE REMYELINATION IN A MODEL OF MULTIPLE SCLEROSIS

Promoting remyelination, a major goal of an effective treatment for demyelinating diseases, has the potential to protect vulnerable axons, increase conduction velocity and improve neurologic deficits. Strategies to promote remyelination have focused on transplanting oligodendrocytes (OLs) or recruiting endogenous myelinating cells with trophic factors. Immunoglobulin (Ig) based therapies, routinely used to treat a variety of neurological and autoimmune diseases, underlies our approach to enhance remyelination. We isolated two human monoclonal antibodies (mAbs) directed against OL surface antigens that promoted significant remyelination in a virus-mediated model of multiple sclerosis (MS). Four additional OL-binding human mAbs did not promote remyelination. Both human mAbs were as effective as human intravenous immunoglobulin (IVIg), a treatment shown to have efficacy in MS, and bound to the surface of human OLs suggesting a direct effect of the mAbs on the cells responsible for myelination. Alternatively, targeting human mAbs to areas of central nervous system (CNS) pathology may facilitate the opsonization of myelin debris allowing repair to proceed. Human mAbs were isolated from the sera of individuals with a form of monoclonal gammopathy. These individuals carry a high level of monoclonal protein in their blood without detriment, lending support to the belief that administration of these mAbs as a therapy would be safe. Our results are 1) consistent with the hypothesis that CNS-reactive mAbs, part of the normal Ig repertoire in humans, may help repair and protect the CNS from pathogenic immune injury and 2) further challenge the premise that Abs that bind OLs are necessarily pathogenic.

### INTRODUCTION

Enhancement of remyelination and protection from axonal injury are important therapeutic goals in the treatment of inflammatory demyelinating CNS disorders such as MS. Remyelination in MS plaques can occur, but is limited (1,2) even though OL progenitors are present in the adult (3,4). A number of therapeutic strategies to promote remyelination have been tested in experimental animals. Transplantation of OLs (5) or their progenitors (6) into demyelinated tissue produces new myelin. Transplanted OL progenitors can also remyelinate demyelinated lesions in the adult CNS (7) and migrate toward an area of damage when placed in close proximity to the lesion (8). Unresolved issues remain concerning the survival of transplanted OL progenitors in the intact adult CNS and their ability to target to areas of myelin pathology (9). However, if CNS lesions are surgically approachable and axons are still intact, transplantation of glial cells maybe a viable therapy for improving functional performance (10).

The in vitro administration of growth or trophic factors induces the expansion of OL progenitors (11,12) or promotes mature OLs to dedifferentiate and subsequently reinitiate a program of myelination (13,14). The in vivo administration of trophic factors via genetically engineered fibroblasts to the injured CNS promotes axonal sprouting and OL proliferation (15). Obstacles to in vivo trophic factor therapy remain, specifically determining the biologically relevant local factor concentration and the potential pleiotropic roles of most trophic factors administered in high concentrations.

As an alternative, our laboratory proposes to repair CNS pathology and enhance endogenous remyelination by using CNS-binding Igs (16), building on a natural reparative response that may already be upregulated following demyelination. Ig therapy can be rapidly adapted and tested as a treatment for human demyelinating disease (17, 18). The premise of our approach is that cells capable of remyelination-and the factors necessary to sustain their growth and differentiation-are present in the demyelinated CNS, but their capacity to produce myelin is limited. The emerging heterogeneity of pathology and OL sparing within the MS population (19) suggests that in practice, the treatment of human demyelinating disease may require combinations of several therapeutic approaches based on an individual's requirements.

We have used a virus-mediated model of demyelination to develop Ig-based therapy. When Theiler's murine encephalomyelitis virus (TMEV) is inoculated intracerebrally into susceptible strains of mice, TMEV induces immune-mediated progressive CNS demyelination clinically and pathologically similar to MS (20). The efficacy of therapies in human MS closely parallel those observed in the TMEV model (21) making this an important platform for the design of clinical trials. A mouse mAb raised against spinal cord homogenate, designated SCH94.03, enhances remyelination in the TMEV model (22). SCH94.03 is a polyreactive, mouse IgMk mAb that binds to the surface of OLs (23). SCH94.03 also enhances the rate of spontaneous CNS remyelination following lysolecithin-induced demyelination (24) and decreases relapse in experimental autoimmune encephalomyelitis (EAE) (25). Additional OL-binding mouse IgMk mAbs, several of which are routine markers for the OL lineage, also promote CNS remyelination (26).

Since mouse IgM mAbs promote remyelination, we hypothesized that polyclonal human IgM would be a more effective treatment of demyelinating disease than IVIg, an established therapy for immune-mediated disorders (27). Treatment of chronically TMEV-infected mice with polyclonal human IgM resulted in enhanced remyelination when compared to IVIg. Two human IgM mAbs were also identified, using an antigen-independent strategy, which promote remyelination to an equivalent or greater degree than polyclonal human IgM. We suggest that human remyelination-promoting mAbs may be an easily implemented, effective therapy for human demyelinating disease. Human mAbs are readily applicable to clinical trials, can be produced free of infectious agents and may alleviate the national shortage and high cost of IVIg. An effective human mAb that promotes remyelination may also simplify the investigation for the mechanism of action of immunomodulatory therapies.

### MATERIALS AND METHODS

### Human Antibodies and Their Isolation

Normal human IgM purified from the pooled plasma of more than 2500 healthy donors was obtained from S. V. Kaveri (28). The purity of IgM was more than 90% as confirmed by SDS-PAGE. Pooled human IgG from healthy donors designated clinically as IVIg was from Miles Inc (Elkhart, IN).

Human serum samples were obtained from the dysproteinemia clinic under the direction of Dr. Robert A. Kyle, Mayo Clinic, and chosen solely by the presence of an Ig clonal peak of greater than 20 mg/ml. Sera were from 102 patients with a wide variety of conditions characterized by a monoclonal IgG or IgM spike in the serum, including Waldenstrom's macroglobulinemia, multiple myeloma, lymphoma, and monoclonal gammopathy of undetermined significance. Sera were dialyzed against water, the precipitates collected by centrifugation (14,000 rpm / 30 min) and dissolved in PBS. Solutions were centrifuged and chromatographed on Superose-6 column (Pharmacia, Upsalla, Sweden). IgM fractions were pooled and analyzed by SDS PAGE. Concentrations were determined by gel staining with Sypro Orange (Molecular Probes, Eugene, OR) densitometry. IgM solutions were sterile filtered and cryopreserved.

### OL Cell Culture and Immunocytochemistry

Cerebral hemispheres from PO-P2 Holtzman Sprague-Dawley rats were prepared for mixed primary glial cell culture as described (29) and grown for 9 days in vitro. Rat OL progenitors were isolated as described (30). Adult human OLs were prepared from temporal lobe biopsies obtained from patients undergoing therapeutic resection for intractable epilepsy. Tissue did not contain the epileptic focus and was of normal cytoarchitecture when examined by the Department of Surgical Pathology. Adult glial cells isolated as described (31) and seeded onto poly-ornithine (Sigma) and laminin (Life Technologies) coated plastic multi-wells (Becton Dickenson) or glass coverslips (Fisher Scientific) in a defined media of DMEM/F12 supplemented with biotin (0.01 mg/ml), tri-iodotyronine (15 nM), 0.5 % BSA (all from Sigma), N2, 1% pen/strep (both from Life Technologies) and recombinant human PDGF AA (R & D Systems, Minneapolis, MN). Cell surface staining was done at 4°C for 12 min on unfixed cells after blocking with HEPES-buffered EBSS (E/H) with 5% BSA. All human Abs were used at 10 mg/ml. Intracellular staining for myelin basic protein using polyclonal mouse antisera (Boehringer Mannheim) was done at room temperature after fixation with 4% paraformaldehyde and permeabilization for 5 min with 0.05% saponin. Primary Abs were detected using fluorescently-conjugated secondary Abs (Jackson ImmunoResearch Laboratories, West Grove, PA). Cell monolayers were mounted in 90% glycerin/PBS with 2.5% 1,4-diazabicyclo[2.2.2]octane to prevent fading (37) and 0.1 µg/ml bisbenzimide (both from Sigma) and viewed with an Olympus Provis epifluorescent microscope equipped with a SPOT digital camera (Diagnostic Instruments Inc, Sterling Heights, MI).

### Virus and Animals

The Daniel's strain of TMEV was used for these experiments and was prepared as described (32). Female SJL/J mice from the Jackson Laboratories were used after 1-week acclimation. Mice 4- to 6-weeks of age were injected intracerebrally with 2 x 105 plaque forming units of TMEV in 10 ml volume resulting in greater than 98% incidence of chronic viral infection. Animals used in this study were 5 to 8 months post-infection and received a single intraperitoneal injection of Ig or PBS. Dosages were 1.0 mg of IVIg or human polyclonal IgM or 0.5 mg of the human mAbs. Animals were killed 5 weeks following Ab treatment for morphologic assessment; chosen because studies in toxic models of demyelination indicate that CNS remyelination is almost complete by this time (33). Spinal cord sections embedded in plastic were cut by a centralized microscopy facility and returned to the laboratory marked with a numerical code. In this way slides are graded for remyelination in a blinded manner.

### Western Blotting

Purified TMEV (34) was separated by SDS-PAGE and proteins transferred to nitrocellulose. After blocking with Tris buffered saline containing 5% non-fat dry milk and 0.05% Tween 20 for 2 hours at room temperature the membrane was incubated with human Igs (10 µg/ml) or rabbit polyclonal anti-TMEV Ab (1:2000) for 4 hours. Bound Igs were detected with biotinylated goat anti-human mAbs or biotinylated goat anti-rabbit mAbs (both from Jackson ImmunoResearch) and alkaline phosphatase-conjugated streptavidin using 5-bromo-4-chloro-3-indolyl phosphate and nitro blue tetrazorium (BCIP/NBT, KPL, Gaithersburg, MD).

### Quantitation of Spinal Cord Demyelination/Remyelination

We have developed methods to quantify the amount of spinal cord demyelination, remyelination and atrophy in susceptible mice using plastic-embedded cross sections stained with 4% paraphenylenediamine (PPD) to visualize myelin (35, **Figure 24A****).** To obtain a representative sampling of the entire spinal cord, 1 mm thick cross sections were cut from every third serial 1 mm block, generating 10 to 12 cross sections that represent the whole spinal cord. From each cross section the area of white matter, white matter pathology, OL remyelination, and Schwann cell (SC) remyelination were calculated using a Zeiss interactive digital analysis system (ZIDAS) and camera lucida attached to a Zeiss photomicroscope (Carl Zeiss Inc., Thornwood, NY). White matter was outlined at a magnification of 40x. The areas of white matter pathology, defined as regions of white matter with demyelination or remyelination, were then traced at a magnification of 100x. Regions of white matter pathology often contained macrophage infiltration, inflammation, and little or no PPD stain (**Figure 25** **C, D, H**). The sum of the areas of pathology containing primary demyelination with or without remyelination was determined as a measure of total demyelination.

The areas of remyelination, either OL or SC, were traced at a magnification of 250x. OLs can remyelinate multiple axon fibers, and thus, OL remyelination results in densely packed, yet thin, myelin sheaths compared to spared normally myelinated axons. SCs can remyelinate only a single axon fiber, resulting in thicker myelin sheaths and increased space between axon fibers compared OL remyelination. SC bodies and nuclei can be observed adjacent to the axons they have remyelinated. Total areas were calculated for each mouse by summing all the areas traced from each of 10 to 12 spinal cord sections per mouse.

The percent area of spinal cord white matter pathology per mouse was obtained by dividing the total area of white matter pathology by the total area of white matter sampled. The percent area of remyelination per mouse was obtained by dividing the area of OL or SC remyelination by the total area of white matter pathology. Repeated measures of white matter pathology and extensive myelin repair revealed comparable values differing only by 1.5%. To determine the validity of using 10 cross sections as a representation of the remyelination throughout the spinal cord, a comparison was performed using 10 cross sections versus all 32 cross sections of a single chronically infected mouse. Assaying 10 cross sections resulted in a percent area remyelination value of 47.7%, whereas the data from all 32 cross sections resulted in a value of 40.0%. Either value would have indicated significant remyelination in our assay.

### RESULTS

### Human IVIg and Polyclonal Human IgM Promote CNS Remyelination in TMEV-Infected Mice

Clinical studies in MS indicate that IVIg may be partially effective in stabilizing the disease course (18,36,37). To determine if human IVIg could promote remyelination in the TMEV model of MS, chronically infected mice were treated with a single intraperitoneal injection of 1 mg of IVIg. A single dose was administered to avoid evoking an immune response to the foreign Ig. The total dose of human Ig was approximately 0.05 g/kg body weight, one-quarter the total dose used for human IVIg treatment (18). Additional mice were treated with a single 1 mg bolus of polyclonal human IgM. Upon examination of the spinal cords, the percent area of OL remyelination in mice receiving either IVIg or polyclonal human IgM **(Table 4,** 14.15% and 23.19%, respectively) was significantly higher than the spontaneous OL remyelination observed in the PBS-treated group (6.74%, p<0.05 for IgG, p<0.01 for IgM). There were no statistically significant differences in the areas of white matter or the areas of white matter pathology between either treatment group or the PBS control group. The data describes two independent experiments treating groups of 7 and 9 mice with IVIg and groups of 7 and 10 mice treated with polyclonal human IgM. The final values in **Table 4** include only those animals that contained at least 5% white matter pathology.

**Table 4. CNS remyelination in mice after treatment with human Abs**

| Treatment | No. of Mice | Area of white matter, mm² | Area of myelin pathology, mm² | Area of CNS type remyelination, mm² | Area of CNS-type remyelination, % |
|---|---|---|---|---|---|
| IVIg | 10 | 8.60 ± 0.52 | 0.86 ± 0.10 | 0.13 ± 0.02 | 14.15 ± 2.38* |
| Human Igm | 14 | 9.70 ± 0.43 | 1.21 ± 0.21 | 0.24 ± 0.04 | 23.19 ± 3.26† |
| sHIgM 1 | 4 | 9.34 ± 1.93 | 0.68 ± 0.07 | 0.03 ± 0.01 | 8.35 ± 3.73 |
| sHIgM 2 | 4 | 8.78 ± 0.70 | 0.87 ± 0.12 | 0.10 ± 0.01 | 11.37 ± 1.30 |
| sHIgM 14 | 7 | 11.01 ± 0.60 | 1.13 ± 0.18 | 0.08 ± 0.03 | 8.41 ± 2.59 |
| sHIgM22 | 8 | 10.55 ± 0.41 | 1.16 ± 0.22 | 0.19 ± 0.05 | 17.06 ± 3.42* |
| sHIgM 46 | 5 | 9.44 ± 0.36 | 0.66 ± 0.06 | 0.18 ± 0.04 | 27.12 ± 4.01‡ |
| PBS | 7 | 9.78 ± 0.60 | 1.20 ± 0.22 | 0.06 ± 0.02 | 6.74 ± 1.80 |

Values represent the mean ± SEM. One-way ANOVA and t test were used to compare the percent area of CNS -type remyelination in mice treated with human antibodies to mice treated with PBS. Such analysis revealed **P* < 0.05; †*P* < 0.01, ‡*P* < 0.001. Comparison of mice treated with other treatments revealed polyclonal human IgM *P* = 0.05, sHIgm 46 P < 0.05. All other comparisons were not statistically significant. There was no difference in the CNS-type remyelination between polyclonal human IgM, sHIgM22, and sHIgM 46. Area of peripheral nervous system-type SC remyelination ranged from 0 to 0.08 mm². This corresponded to 0.0 to 6.92 percent area of peripheral nervous system type SC remyelination as a function of myelin pathology. There was no statistical difference in the area of myelin pathology in the various treatment groups or compared to PBS or in other peripheral nervous system-type SC remyelination between groups.

Treatment with polyclonal human IgM resulted in more OL remyelination than that observed in mice treated with IVIg (p=0.05, **Figure 25** **A, B).** Approximately one quarter of the total area of myelin pathology was remyelinated in mice treated with polyclonal human IgM, representing thousands of ensheathed axons. On average, 1 mm2 within confluently remyelinated areas of pathology **(****Figure 25B****)** corresponded to 46,000 to 125,000 remyelinated axons. Therefore, the CNS remyelination following human Ig treatment was extensive. Few inflammatory cells or macrophages were present. In contrast, in mice treated with PB S, areas of myelin pathology contained few remyelinated axons **(****Figure 25H****).** Signs of active myelin destruction, such as myelin whirls, inflammatory cells and macrophages were present.

As an additional, faster, method to judge the effectiveness of a treatment to promote remyelination the 10 spinal cord sections representative of an animal were examined for the presence of areas of white matter pathology that demonstrated nearly complete repair. We defined complete repair as an area of white matter pathology with nearly confluent remyelinated axons and no inflammatory cells or macrophages present (as in **Figure 25** **B, F, G),** a very rare event in spontaneous remyelination. At least one area of complete repair was observed in four of ten animals treated with IVIg and in ten of fourteen animals treated with polyclonal human IgM. We concluded that both IVIg and polyclonal human IgM promote remyelination compared to PBS treatment and that polyclonal human IgM is superior to IVIg in the ability to promote CNS remyelination.

Human mAbs That Bind to OLs Promote CNS remyelination in TMEV-infected mice All of the previously identified mouse mAbs that promote CNS remyelination bind to OLs (23,26). To screen human mAbs for testing in the TMEV model, human mAbs were tested for the ability to bind to the surface of rat OLs in unfixed mixed primary glial culture. Primary cultures established from neonatal rat brain contain OLs at varying stages of differentiation at 9 days in vitro (38). Our sources of human mAbs were serum-derived human monoclonal IgMs (sHIgMs) and sera-derived human monoclonal IgGs (sHIgGs). None of 50 sHIgGs bound to unfixed rat OLs, but six of 52 sHIgMs bound to the surface of rat OLs co-labeled with the anti-sulfatide mAb, 04 (39).

The six OL-binding sHIgMs were used to treat TMEV-infected mice. Groups of five animals each received a single injection of 0.5 mg of human mAb. The average percent area of OL remyelination following treatment with sHIgM22 and sHIgM46 **(****Figure 25** **F, G)** were both significantly above the background levels attributable to spontaneous remyelination. The other four OL-binding sHIgMs promoted remyelination at levels comparable to or below the level observed following treatment with PBS. A second set of animals were treated with sHIgM22, sHIgM46 or PBS to confirm the initial observations. SHIgM14 was also repeated as an example of a human mAb that bound to OLs, but did not promote remyelination. The combined data are presented in Table 1. Only animals that contained at least 5% total white matter pathology were included in statistical analysis.

The highest percent area of OL remyelination was observed in animals treated with sHIgM46 (27.1%), followed by animals treated with sHIgM22 (17.1%). The percent area of remyelination following treatment with sHIgM14 (8.41%) was similar to that observed following treatment with PBS (6.74%). To test if any sHIgM, irrespective of antigen specificity, could promote remyelination we studied two mAbs in vivo which demonstrated no immunoreactivity to OLs in mixed primary culture, sHIgM1 and sHIgM2 **(****Figure 25** **C, D).** The percent area of remyelination following treatment with sHIgM1 (8.3%), sHIgM2 (11.4%) were not significantly different from the sHIgM14 or PBS treatment groups. In all groups the areas of white matter and areas of white matter pathology were not statistically different. Compared to the remyelination observed in the PBS-treated group, the percent area of remyelination following treatment with sHIgM46 or sHIgM22 resulted in p values of <0.001 and <0.05, respectively. The area of peripheral nervous system-type SC remyelination ranged within treatment groups from 0 to 0.08 mm2. This corresponded to values of 0.0 to 6.92 percent area of SC remyelination as a function of white matter pathology. There were no statistical differences in the percent area of SC remyelination between any treatment group.

Comparing the percent area of OL remyelination observed following treatment with either human polyclonal or monoclonal preparations revealed that sHIgM46 was statistically superior to IVIg (p<0.05), but not to polyclonal human IgM. The percent area of OL remyelination observed following treatment with sHIgM22 was no different than that following treatment with IVIg, polyclonal human IgM or sHIgM46.

When examined for areas of white matter pathology with complete repair at least one area was observed in four out of eight animals treated with sHIgM22 and in five out of five animals treated with sHIgM46. In contrast, none of the animals treated with sHIgM1, sHIgM2, sHIgM14 or PBS contained a single area of complete repair.

### Human mAbs, but not Polyclonal Human Igs, Bind to Rat Human OLs

If human mAbs are to be a potential therapy to promote remyelination in humans, a reactivity to surface antigens on human OLs may prove important in targeting to areas of human CNS pathology. Therefore, we determined whether human remyelination-promoting mAbs could bind to OLs obtained from the adult human brain. Human glial cell cultures were established from adult temporal lobe biopsies and immuno-labeled with the human mAbs at several time points in culture.

Three of the six sHIgMs that bound to the surface of OLs in our initial screen, also bound to human OLs. At one week in culture morphologically immature sulfatide positive human OLs labeled with sHIgM14 and sHIgM46, but not with sHIgM22. By 3 weeks in culture, morphologically complex sulfatide positive human OLs co-labeled with sHIgM14, sHIgM22 and sHIgM46 (**Figure 26** **A**, **B, C**). By 4 weeks in culture, virtually all MBP-positive human OLs also bound sHIgM22 and sHIgM46, but the binding of sHIgMs4 was greatly reduced (data not shown).

Neither IVIg nor polyclonal human IgM bound to the surface of human OLs in culture at any time tested. However, polyclonal human IgM bound strongly to white matter tracts and a variety of neuronal populations when incubated with fresh unfixed slices of rodent CNS. IVIg was completely negative in this binding assay (data not shown). SHIgM22 and sHIgM46, both of which promoted remyelination, and sHIgM14, which did not promote remyelination, also bound to the surface of purified myelin basic protein-positive rat OLs (data not shown).

We concluded that an affinity for OL antigens may be necessary, but is not sufficient for a human mAb to promote remyelination. The fact that both human mAbs that promote significant remyelination bind to mature differentiated human OLs underscores the possible requirement of mAbs to be directed against surviving adult OLs for in vivo function.

To exclude the possibility that human Igs or mAbs promoted remyelination by neutralizing virus, each preparation was tested for reactivity to purified TMEV antigens by Western blotting (34). None of the human Ab preparations reacted with TMEV proteins; however, rabbit polyclonal Ig raised against TMEV reacted strongly to four virus capsid proteins (data not shown).

Peripheral B-cells were obtained from the individual from which sHIgM22 was identified. The light and heavy chain variable domain sequences of sHIgM22 were determined. The sHIgM22 light chain variable region (GenBank accession AF212992) belongs to the λ subgroup I of the human light chain variable regions. The sHIgM22 heavy chain variable region (GenBank accession AF212993) belongs to subgroup III of the human heavy chain variable regions. There were significant differences between the sHIgM22 variable domains and the closest known human germline variable domain sequence (40).

### DISCUSSION

In this series of experiments we have demonstrated that human Abs can promote CNS remyelination. More extensive remyelination was observed in the spinal cords of TMEV-infected mice following treatment with polyclonal human IgM than treatment with human IVIg. In addition, we identified two human monoclonal IgMs that consistently enhanced remyelination. Both mAbs were isolated from the sera of patients with Waldenstrom's macroglobulinemia (WM), a class of lymphoma characterized by the malignant clonal expansion of a single B-cell at the late stage of maturation which floods the serum with a monoclonal IgM (41). The high level of these mAbs do not appear to be deleterious. In patients with WM the dominant IgM normally recognizes antigens that are recognized by the IgM repertoire present in healthy individuals (42). Our ability to readily identify and isolate OL antigen-binding, remyelination promoting mAbs from the human population lends support to the concept that these Abs are common among the B-cell repertoire and may function as modifiers in response to CNS injury.

### Remyelination-promoting mAbs may be produced in the sera of individuals when confronted with CNS damage.

Although both IVIg and polyclonal human IgM promoted remyelination neither bound to rat or human OLs in culture. In contrast, both human mAbs that promoted remyelination bound to both rat and human OL surface antigens. The increased efficacy of human mAbs to promote remyelination may be due to the effective targeting to adult OLs in the area of damage. Stangel reported that IVIg had no affect on the differentiation, migration or proliferation of OL progenitors in culture; however, the binding of IVIg to OL progenitors was not assessed (43). The lack of affinity of IVIg to OLs likely explains the lack of any discernible affect on OL progenitors. Nevertheless, the fact that IVIg does not bind to OLs implies that the mechanism of action in promoting remyelination may be distinct from that of the human mAbs.

The very same preparation of polyclonal human IgM used in this study has been demonstrated to neutralize autoantibodies (28) and alter cytokine expression in EAE (44) and to be beneficial in a mouse model of myasthenia gravis (45). Polyclonal human IgM, but not IVIg, binds to myelinated tracts in unfixed slices of rodent brain. Neither polyclonal preparation bound to fresh human white matter. Polyclonal human IgM may promote significant remyelination in the mouse via a combination of general immunoregulation, binding to pathogenic antibodies and opsonization of myelin debris.

The mechanism by which Igs promote remyelination remains to be elucidated. Since many of the remyelination-promoting mAbs bind to OLs and/or myelin, it is reasonable to hypothesize a direct effect on the recognized cells. There are examples of mAbs binding to and altering the biology of OLs in culture (46-48). However, since the mAbs that promote remyelination have varying specificities (23, 26) it is unlikely that each mAb functions directly through a common antigen or receptor. A polyvalent molecule like an IgM could bring normally disparate signaling molecules into close proximity within the plasma membrane with subsequent activation (49). Since most of the remyelination-promoting mAbs appear to bind to lipids (26), the binding of these IgMs to the cell surface could reorganize the plasma membrane and facilitate a signaling pathway. When SCH94.03 is added to mixed primary glial cultures a 2-3 fold increase the uptake of tritiated thymidine is observed (Rodriguez, unpublished observations).

Another potential mechanism by which remyelination-promoting mAbs may function is by targeting to myelin debris or damaged OLs. Binding to OLs or myelin may enhance the clearance of cellular debris from areas of damage, allowing the normal process of spontaneous CNS repair to progress. Perhaps the mechanism of action of polyclonal human Igs is primarily through immunomodulation-via an inhibition of B-cell differentiation or an alteration of cytokine expression and the anti-idiotypic network (27, 50)-whereas the action of the human mAbs is via a direct targeting to OL antigens and/or myelin. No characteristic was completely predictive of an Ab's ability to promote remyelination. In fact, one human mAb tested in chronically TMEV-infected mice appears to suppress remyelination below the level of spontaneous remyelination, suggesting that certain OL-binding human mAbs can inhibit remyelination in vivo or may exacerbate demyelination. This is consistent with the observation that specific mAbs reactive to OL antigens (i.e., myelin oligodendrocyte glycoprotein, 51) enhance demyelination in EAE (52). Ultimately, proof of an Ab's remyelinating potential and lack of pathogenicity requires in vivo testing.

Several double-blind, placebo-controlled trials with human IVIg have shown some efficacy in MS (18,36,37). Polyclonal human IgM, sHIgM22 and sHIgM46 all enhanced CNS remyelination in the TMEV model as well as IVIg, suggesting that these Abs may be as effective in MS. Human mAbs that bind to OLs may have the additional benefit of direct OL stimulation. Human mAbs can be produced free from potential pathogen infection and can be structurally altered to augment their effectiveness and immunogenicity. In contrast to mouse mAbs or "humanized" mouse mAbs, human mAbs should result in minimal immune response and are readily applicable to human trials. Given that human mAbs promoted remyelination in chronically paralyzed animals provides hope that successful therapies can be developed for patients with long-standing disabilities.

### REFERENCES

1. Perier, O. & Gregoire, A. (1965) Brain 88, 937-952.
2. Prineas, J.W. & Connel, F. (1979) Ann. Neurol. 5, 22-31.
3. Wolswijk, G. & Nobel, M. (1989) Development 105, 387-400.
4. Armstrong, R.C., Dorn, H.H., Kufta, C.V., Friedman, E. & Dubois-Dalcq, M.E. (1992) J. Neurosci. 12, 1538-1547.
5. Gumpel, M., Baumann, N., Raoul, M. & Jaques, C. (1983) Neurosci. Lett. 37, 307-312.
6. Warrington, A.E., Barbarese, E. & Pfeiffer, S.E. (1993) J. Neurosci. Res. 34, 1-13.
7. Groves, A.K., Barnett, S.C., Franklin, R.J., Crang, A.J., Mayer, M., Blakemore, W.F. & Noble, M. (1993) Nature 362, 453-455.
8. Franklin, R.J.M., Bayley, S.A. & Blakemore, W.F. (1996) Exp. Neurol. 137, 263-276.
9. O'Leary, M.T. & Blakemore, W.F. (1997) J. Neurosci. Res. 48, 159-167.
10. Jeffery, N.D., Crang, A.J., O'Leary, M.T., Hodge, S.J. & Blakemore WF. (1999) Eur. J. Neurosci. 11, 1508-1514.
11. McKinnon, R.D., Matsui, T., Dubois-Dalcq, M., & Aaronson, S.A. (1990) Neuron 5, 603-614.
12. Bögler, O., Wren, D., Barnett, S.C., Land, H. & Noble, M. (1990) Proc Natl Acad Sci USA 87, 6368-6372.
13. Rosano, C., Felipe-Cuervo, E., & Wood, P.M. (1999) Glia 27, 189-202.
14. Bansal, R. & Pfeiffer, S.E. (1997) J. Neurosci. Res. 50, 215-228.
15. McTigue, D.M., Horner, P.J., Stokes, B.T. & Gage, F.H. (1998) J. Neurosci. 18, 5354-5365.
16. Miller, D.J., Asakura, K. & Rodriguez, M. (1995) J. Neurosci. Res. 41, 291-296.
17. Noseworthy, J.H., O'Brien, P.C., van Engelen, B.G. & Rodriguez, M. (1994) J. Neurol. Neurosurg. Psychiatry 57 Suppl: 11-14.
18. Fazekas, F., Deisenhammer, F., Strasser-Fuchs, S., Nahler, G. & Mamoli, B. (1997) Lancet 349, 589-593.
19. Lucchinetti, C.F., Bruck, W., Rodriguez, M. & Lassmann, H. (1996) Brain Pathol. 6, 259-274.
20. Dal Canto, M.C. & Lipton, H.L. (1997) Am. J. Pathol. 88, 497-500.
21. Drescher, K.M., Rivera-Quinones, C., Lucchinetti, C. & Rodriguez M. (1998) J. Neuroimmunol. 88, 111-119.
22. Miller, D.J., Sanborn, K.S., Katzmann, J.A. & Rodriguez, M. (1994) J. Neurosci. 14, 6230-6238.
23. Asakura, K., Miller, D.J., Murray, K., Bansal, R., Pfeiffer, S.E. & Rodriguez, M. (1996) J. Neurosci. Res. 43, 273-281.
24. Pavelko, K.D., van Engelen, B.G. & Rodriguez, M. (1998) J. Neurosci. 18, 2498-2505.
25. Miller, D.J., Bright, J.J., Sriram, S. & Rodriguez, M. (1997) J. Neuroimmunol. 75, 204-209.
26. Asakura, K., Miller, D.J., Pease, L.R.& Rodriguez, M. (1998) J. Neurosci. 18, 7700-7708.
27. Dwyer, J.M. (1992) N. Engl. J. Med. 326, 107-116.
28. Hurez, V., Kazatchkine, M.D., Vassilev, T., Ramanathan, S., Pashov, A., Basuyaux, B., de Kozak, Y., Bellon, B. & Kaveri, S.V. (1997) Blood 90, 4004-4013.
29. Gard, A.L. & Pfeiffer, S.E. (1989) Development 106, 119-132.
30. Asakura, K., Hunter, S.F. & Rodriguez, M. (1997) J. Neurochem. 68, 2281-229035.
31. Hunter, S.F. & Bottenstein, J.E. (1991) J. Neurosci. Res. 28, 574-583.
32. Rodriguez, M., Leibowitz, J.L. & Lampet, P.W. (1983) Ann. Neurol. 13, 426-433.
33. Blakemore, W.F., Eames, R.A., Smith, K.J.& McDonald, W.I. (1977). J. Neurol. Sci. 33,
34. Njenga, M.K., Pavelko, K.D., Baisch, J., Lin, X., David, C., Leibowitz, J. & Rodriguez, M. (1996) J. Virol. 70, 1729-1737.
35. McGavern, D.B., Murray, P.D. & Rodriguez, M. (1999) J. Neurosci. Res. 58, 492-504.
36. Achiron, A., Pras, E., Gilad, R., Ziv, I., Mandel, M., Gordon, C.R., Noy, S., Sarova-Pinhas, I. & Melamed, E. (1992) Arch. Neurol. 49, 1233-1236.
37. Sorensen, P.S., Wanscher, B., Jensen, C.V., Schreiber, K., Blinkenberg, M., Ravnborg, M., Kirsmeier, H., Larsen, V.A. & Lee, M.L. (1998) Neurology 50, 1273-1281.
38. Pfeiffer, S.E. (1984) in Oligodendroglia, ed. Norton, W.T. (Plenum, New York), pp. 233-298.
39. Sommer, I. & Schachner, M. (1981) Devel. Biol. 83, 311-327.
40. Kabat, E.A., Wu, T.T., Perry, H.M., Gottesman, K.S. & Foeller, C. (1991) Sequences of proteins of immunological interest, 5th ed. (NIH Publication, Bethesda, MD).
41. Kyle, R.A. & Garton, J.P. (1987) Mayo Clin. Proc. 62, 719-731.
42. Lacroix-Desmazes, S., Mouthon, L., Pashov, A., Barreau, C., Kaveri, S.V. & Kazatchkine, M.D. (1997) Intl. Immunol. 8, 1175-1183.
43. Stangel, M., Compston, A. & Scolding, N.J.(1999) J. Neuroimmunol. 96, 228-233.
44. Pashov, A., Bellon, B., Kaveri, S.V. & Kazatchkine, M.D. (1997) Mult. Scler. 3, 153-156.
45. Vassilev, T., Yamamoto, M., Aissaoui, A., Bonnin, E., Berrih-Aknin, S., Kazatchkine, M.D. & Kaveri, S.V. (1999) Eur. J. Immunol. 29, 2436-2442.
46. Bansal, R. & Pfeiffer, S.E.. (1989) Proc. Natl. Acad. Sci. USA 86, 6181-6185.
47. Dyer, C.A. & Benjamins, J.A. (1990) J. Cell Biol. 111, 625-633.
48. Cohen, J.A., Williams, W.V., Geller, H.M. & Greene, M.I. (1991) Proc. Natl. Acad. Sci. USA 88, 1266-1270.
49. Bansal, R., Winkler, S. & Bheddah, S. (1999) J. Neurosci. 19, 7913-7924.
50. Stangel, M. Toyka, K.V. & Godl, R. (1999) Arch. Neurol. 56, 661-663.
51. Lebar, R., Lubetzki, C., Vincent, C., Lombrail, P. & Boutry, J-M. (1986) Clin. Exp. Immunol. 66, 423-443.
52.
   Genain, C.P., Nguyen, M.H., Letvin, N.L., Pearl, R., Davis, R.L., Adelman, M., Lees, M.B., Linington, C. & Hauser, S.L. (1995) J. Clin. Invest. 6, 2966-2974.

### EXAMPLE 4

As described in the prior Examples, we have used two approaches to identify human monoclonal antibodies that induce a similar pattern of remyelination in the Theiler's virus model of demyelinating disease. The first approach was to transform human B cells with Epstein Barr Virus (EBV) to generate immunoglobulin secreting B cell clones. The resulting cell lines were screened to identify cultures that expressed high levels of antibody and the ability of the produced antibodies to bind CNS antigens, with particular emphasis on those that bound oligodendrocytes. The second approach was to perform a similar screen CNS-binding of serum from patients diagnosed with a monoclonalgammopathy such as MUGUS, lymphoma, or Waldenström's syndrome. In the case of the EBV transformed cells, the cells themselves might provide a source of antibody for generating sufficient quantities of GMP grade antibodies for clinical trials. In addition, antibodies identified from either source could be produced more optimally in an artificial antibody producing system using synthetic antibody genes encoding the antibodies of interest. We anticipate that hybridoma cell lines transfected with an antibody expression cassette encoding the antibody of interest will provide sufficient antibody of interest for in vivo analysis and clinical trials.

In the course of the screening studies, we have identified a set of human monoclonal IgM antibodies that induce statistically significant remyelination in our in vivo Theiler's virus model of demyelinating disease **(TABLE 5).** Each of these antibodies mimicked the remyelination response originally described with the prototypical murine monoclonal antibody SCH 94.03. Among these human antibodies are two derived from EBV-transformed B cell lines, designated MSI 19D10 and CB2bG8, and two antibodies, designated sHIgM 22 and sHIgM 46, identified among a panel of antibodies from more than 50 patients expressing high levels of monoclonal IgM in their sera.

**TABLE 5. Remyelination induced by human monoclonal antibodies in SJL/J mice chronically infected with Theiler's virus.**

| Treatment | % Remyelination | Statistical Evaluation |
|---|---|---|
| | Comparison 1 | |
| PBS (n=7) | 6.74 (+/-1.80) | |
| sHIgM 22 (n=8) | 17.6 (+/-3.42) | P<0.05 |
| sHIgM 46 (n=5) | 27.12 (+/-4.01) | P<0.001 |
| | Comparison 2 | |
| PBS (n=12) | 8.25 (+/-1.44) | |
| MSI 19-D10 (n+13) | 24.38 (+/-2.91) | P<0.001 |
| CB2b-G8 (n=12) | 23.51 (+/-3.13) | P<0.001 |

Animals were chronically infected with DA strain of Theiler's virus for greater than 9 months prior to treatment with a single ip injection of 0.5 mg IgM antibody isolated from patient serum (sHIgM22 and sHIgM 46) or EBV transformed cell lines (MSI 19-D10 or CB2b-G8). Five weeks later, the mice were perfused with fixative and spinal cords were isolated for histological analysis. Areas of demyelination and remyelination were assessed directly by microscopy. Percent area remyelination was determined by the formula area remyelinated/area demyelinated X 100. Treatment effects were evaluated by statistical comparison to groups of animals that received PBS injections instead of antibody.

The structures of the IgM heavy and light chains for both the antibodies derived from the EBV-transformants have been determined by analysis of cDNA generated from immunoglobulin mRNA isolated from the cells. The sequences of the heavy and light chain variable regions of MSI 19D10 and sHIgM 22 are provided in **Figures 19** and **20** (SEQ ID NOS: 9 and 11) and **Figures 17** and **18** (SEQ ID NOS: 1, 49, 5 and 50), respectively. The sequences of the heavy and light chain variable regions of CB2bG8 are provided in **Figures 27** and **28** (SEQ ID NOS: 13 and 15). The sequences themselves are not remarkable other than they differ somewhat from known germline immunoglobulin sequences. Thus, they may be the products of somatic diversification during the course of immune responses against unidentified antigens. The value of the sequences is that they provide a blue print for the construction of expression vectors for the production of the immunoglobulin under controlled conditions.

Similarly, the structures of the heavy and light chains from the serum of one of the IgM-producing patients were determined by protein sequence analysis, followed by cloning and sequence analysis of cDNA from peripheral blood mononuclear cells isolated from the patient. Two closely related heavy and light chains were identified in the patient's serum, designated sHIgM22 **(****Figures 17** and **18****).** The two heavy and two light chains were both present in the isolated cDNA populations at a ration of 60:40. Both antibodies share a common µ-VDJ rearrangement and λ-VJ rearrangement, indicting that they are derived from a common B cell precursor. They have subsequently diverged, as a result of the accumulation of mutations that have altered the structures of their variable regions. We conclude that both antibodies are expressed in the serum of the patient because peptides from both antibodies were characterized from the protein isolated from the serum. However, the two distinct combinations of variable and light chains were not observed directly, leaving open the possibility that other combinations of the identified heavy and light chains may actually be present. Based on the positions of the observed amino acid substitutions, we suspect that the antibodies have very similar reactivity patterns.

### EXAMPLE 5

### DEVELOPMENT OF A TRANSFECTION SYSTEM FOR THE EXPRESSION OF ANTIBODY GENES IN CELL CULTURE

In order to generate a renewable supply of high titer antibody from the human antibodies, we have developed a transfection-based expression system. Hybridoma cells which have been selected for the loss of endogenous immunoglobulin mRNA production can be transfected with recombinant antibody genes to generate cells expressing the antibodies of interest.

We have explored the use of a series of genomic and cDNA based vector systems to express cloned antibody genes in cell culture. We have successfully expressed light chain protein using either genomic or cDNA -based genes. We have achieved heavy chain expression using a genomic-based heavy chain vector PAG 4026 (kindly provided by Dr. Sherie Morison at UCLA). However, the yield of antibody with this vector system is too low for practical use in vivo. Our focus has been to develop a new vector that will routinely yield transfected hybridoma clones that produce high titer antibody. Our current strategy is to assemble the vector from components that individually have been shown to work well in our hands.

We have shown that a vector expressing dHfR and an immunoglobulin light chain cDNA under CMV-promoter control expresses light chain and that this expression can be amplified by growing the transfected immunoglobulin producing cells in increasing concentrations of methotrexate **(****Figure 29****).** We then clone this functional unit, expressing immunoglobulin light chain and dHfR, into the vector expressing the genomic heavy chain gene encoding the complementing µ chain. The first vector we have created along this line encodes mouse/human chimeric 94.03 and is shown in the top panel of **Figure 30****.** The vector has been introduced into antibody-negative hybridoma cells and clones expressing small amounts of functional antibody have been isolated. The antibody stains CNS tissue in a manner identical to the native mouse antibody 94.03 **(****Figure 31****).** These antibody-producing clones are now undergoing selection with increasing amounts of methotrexate to expand the amount of antibody being produced. Because we have identified two heavy and two light chains in the human serum sHIgM22, all four permutations of heavy and light chain need to be evaluated. Vectors expressing three of the four possible combinations of sHIgM22 identified in our sequence study have been prepared and are now being introduced into the immunoglobulin-negative hybridoma cells. The prototype vector is shown in the bottom panel of **Figure 30****.**

### METHODS

### Construction of expression vectors for expressing mouse/human chimeric 94.03 (M1) and sHIgM-22 antibody:

### Assembly of expression system for mouse/human chimeric 94.03

The assembled vector consists of two units. The first encodes the heavy chain of the immunoglobulin which is encoded by a genomic DNA-derived immunoglobulin gene. The vector is in part a derivative of a backbone vector (PAG 4026) obtained from the laboratory of Dr. Sherrie Morrison from UCLA. The PAG 4026 vector encoded an IgM heavy chain expressing an irrelevant variable region. There were no convenient cloning sites available for the substitution of variable regions of interest. We therefore engineered sites by deletion of the irrelevant heavy chain sequences and reconstitution of the regions flanking the variable region with unique restriction sites (Rsr II at the 5' end and Pac I at the 3' end). As the sequence of the PAG vector was not known, we determined which restriction enzyme sites would be unique by trial and error using enzymes that recognize sequences infrequently present in mammalian DNA.

The heavy chain variable region of the mouse IgM monoclonal antibody 94.03 was isolated from cDNA by PCR using the RsrII primer ACTCCCAAGTCGGCTCGCTTTCTCTTCAGTGACAAACACAGACATAGAACA TTCACCATGGGATGGAGCTGTATCACT (SEQ ID NO: 39) to introduce the RsrII site upstream of the leader sequence and the PacI primer ACTGACTCTCTTAATTAAGACTCACCTGAGGAGACTGTGAGAGTGGT (SEQ ID NO: 40) to introduce the PacI site while maintaining the correct splice junction at the 3' end of the variable region coding block.

The second part of the expression vector is a derived from multiple plasmids. The finished construct contains EagI sites at both termini, the DHFR (dihydrofolate reductase) coding sequence under regulatory control of the SV40 promoter and a chimeric mouse/human kappa light chain cDNA coding block under regulatory control of the CMV promoter. This portion of the vector was assembled in a step wise fashion starting with three plasmids (pCIneo (Promega Corporation), pUC18 (New England Biolabs), and pFR400 (Simonsen and Levinson, Proc Natl Acad Sci USA 80:2495:1983)) that provided appropriate cloning sites, promoter regions, polyadenylation signals, and the DHFR coding block. After a series of modifications which included the introduction of synthetic linker regions and deletions of undesirable restriction endonuclease recognition sites, the methotrexate selectable light chain cassette was assembled. The cassette includes unique restriction endonuclease sites (Nhe I and Xho I) that flank the cDNA coding block of the light chain gene.

The chimeric light chain gene was assembled from two cDNA sequences using the PCR splicing by overlap extension technique (Horton et al. Gene 77:61:1989). The primers flanking the fused regions of the chimeric cDNA (contained the enzyme recognition sequences for the endonuclease Xho I and Nhe I. The 5' primer used to amplify the fused gene product was TTGGCGCGCCAAAGACTCAGCCTGGACATGATGTCCTCTGCTCAGTTC (SEQ ID NO: 41) ; the 3' primer was ATAGTTTAGCGGCCGCATTCTTATCTAACACTCTCCCCTGTTG (SEQ ID NO: 42). The cDNA coding block was inserted into the light chain cassette vector using these sites.

Once assembled, the cassette was excised using the endonuclease Eag I and inserted into the unique Eag I site in the vector containing the heavy chain gene. The resulting construct contains the coding sequences for both the heavy and light chain components of the mouse/human chimeric antibody for "humanized" 94.03. The heavy chain is expressed by the human IgH promoter and the light chain is expressed by the CMV promoter. The dHFR gene provides an amplification marker and is expressed by the SV40 promoter. Each of these genes contains polyadenylation signals at the 3' ends. Other important features of the vector include a bacterial origin of replication and a gene expressed in bacteria encoding resistance to ampicillin. The heavy variable and light chain cDNA coding blocks are flanked by unique restriction endonuclease sites that can be use to substitute new immunoglobulin sequences isolated from mRNA of any antibody producing cell or synthetic immunoglobulin genes.

### Insertion of sHIgM.22 sequences into the expression vector system

The cDNA of mRNA encoding the heavy and light chains of sHIgM.22 were prepared by PCR amplification of peripheral blood RNA using 5' primers deduced from amino acid sequence information and sequences in the constant regions of the heavy and light chain respectively. The heavy chain variable region coding block, leader sequence and donor splice junction along with the flanking RsrII and Pac I sites were assembled by using PCR to add the 5' region GACTCGGTCCGCCCAGCCACTGGAAGTCGCCGGTGTTTCCATTCGGTGATC ATCACTGAACACAGAGGACTCACCATGGAGTTTGGGCTGAGCTGGGTTTTC CTCGTTGCTCTTTTAAGAGGTGTCCAGTGTCAGGTGCAGCTGGTGGAGTCT GG (SEQ ID NO: 43) and the 3' sequences CCTTAATTAAGACCTGGAGAGGCCATTCTTACCTGAGGAGACGGTGACCAG GGTTC (SEQ ID NO: 44). The resulting DNA molecule was digested with Rsr II and Pac I and subsequently cloned into the expression vector, substituting the desired variable region sequence for the irrelevant sequence in the vector.

The light chain sequence was assembled in two steps. The lambda constant region was isolated from mRNA by RT-PCR using the 5' primer CTAGCTAGCGTCCTAGGTCAGCCCAAGGCTGCCCCC (SEQ ID NO: 45) and 3' primer ATAGTTTAGCGGCCGCACCTATGAACATTCTGTAGG (SEQ ID NO: 46). This fragment was cloned using a unique AvrII site and a 3' Not I site into the pCIneo vector.

The variable region of sHIgM.22 was generated by RT-PCR using the 5' primer CTAGCTAGCCCGAATTTCGGGACAATCTTCATCATGACCTGCTCCCCTCTC CTCCTCACCCTTCTCATTCACTGCACAGGGTCCTGGGCCCAGTCTGTGTTG ACGCAGCCG (SEQ ID NO: 47) in order to introduce the needed Nhe I site and leader sequence onto the cDNA. The 3' primer, GGGCAGCCTTGGGCTGAGCTAGGACGGTCAGC (SEQ ID NO: 48), was used to introduce an AvrII site so that this fragment could be joined with the constant region piece. The resulting coding block containing a functional leader signal was flanked by the necessary NheI and Xho I sites for cloning into the dHFR/light chain cassette, which was subsequently assembled with the heavy chain plasmid to generate the final product containing both the heavy and light chain coding sequences and promoters needed for expression in mammalian cells.

### EXAMPLE 6

### A 94.03 IgG ISOTYPE ANTIBODY

One strategy for determining the importance of isotype in the ability of the mouse antibody 94.03 to induce remyelination is to generate a recombinant antibody that expresses the variable region of 94.03 with an IgG isotype. As an alternative strategy, we sought to identify a natural isotype switch variant within the population of 94.03-producing cells in culture. Spontaneous switch variants have been known to appear upon occasion in cultures of this type. After successive FACS sorts of cells stained for cell surface IgG, we were able to isolate a clonal cell line secreting 94.03 bearing the IgG₁ isotype **(****Figure 32****).** The structure of the antibody produced by these cells was confirmed by ELIZA, by characterization of the produced protein on SDS gels, and by cDNA cloning. Direct sequence analysis as outlined in **Figure 33** produced definitive data indicated that we have isolated an IgG₁ variant of the 94.03 antibody.

### EXAMPLE 7

### IgG₃ ISOTYPE ANTI-OLIGODENDROCYTE MOUSE ANTIBODY 09

The mouse 09 antibody was isolated as an anti-oligodendrocyte antibody and is of the IgG₃ subtype (Kuhlmann-Krieg, S., Sammer, I. and Shachner M. (1988) Devel Brain Res 39:269-280). The 09 antibody binds strongly and specifically to white matter in the CNS. We examined and demonstrated the ability of the 09 antibody to stimulate remyelination in the TMEV model (data not shown). The 09 antibody heavy chain variable region sequence is provided in **Figure 34** (SEQ ID NO: 17). The sequence of the kappa light chain 1 variable region of 09 is provided in **Figure 35** (SEQ ID NO: 19). The sequence of the kappa light chain 1 variable region of 09 is provided in **Figure 36** (SEQ ID NO: 21).

### EXAMPLE 8

### IgM MONOMERS INDUCE REMYELINATION

Another approach to deciphering the importance of structural features of the IgM antibodies for the induction of remyelination is to fractionate the antibody biochemically and to evaluate the ability of the antibody fragments to induce remyelination in vivo. One possibility is that the identified antibodies have low affinity for CNS structures, and therefore, the decavalency of IgM may be critical for remyelinating activity because the multiple binding sites provide enough avidity for the antibodies to interact with the target structures in the CNS. To address this question, we have generated IgM monomers by reduction of the disulfied bonds that hold the pentameric immunoglobulin molecules together. The resulting monomers are divalent. The monomers fail to bind oligodendrocytes in vitro and do not stain brain sections in the pattern observed with the intact native antibody. However, the monomeric antibodies retain the ability to induce remyelination in vivo **(TABLE 6).** This was a surprising result in light of the absence of observed staining in our in vitro assays. One possibility is that in vitro assays monitoring binding are not as sensitive as the bioassay for remyelination. Because there is such a strong correlation between binding and the induction of remyelination, we believe that the specificity of these antibodies for CNS structures is important despite our inability to observe binding with the monomers.

**Table 6. Remyelination induced by monomeric fragments of murine IgM mAb 94.03.**

| Treatment | % Remyelination | Statistical Evaluation |
|---|---|---|
| PBS (n=7) | 6.74 (+/-1.80) | |
| Monomeric 94.03 (n=8) | 17.32 (+/-2.67) | P<0.01 |
| Pentameric 94.03 (N=5) | 18.1 (+/-5.76) | P<0.01 |

IgM antibodies were reduced in mild conditions and alkylated. This treatment disrupted the pentameric structure of the antibodies and allowed divalent monomers to be isolated by column chromatography. Chronically infected SJL/J mice received a total of 0.5 mg of antibody administered ip twice a week over the five week treatment period. After five weeks, the animals were perfused with fixative and their spinal cords removed for histological analysis. Percent remyelination was determined microscopically by comparing the area of remyelinated lesions to total demyelinated area as indicated in Table 5. Individual treatment groups were compared to animals which received PBS injections instead of antibody.

We have further fractionated the antibody by generating (Fab')₂ Fab, and Fv fragments of the antibody. SJL mice chronically infected with Theiler's virus have been treated with these antibody fragments to determine whether divalent fragments missing the Fc portion of the antibody or monovalent antibody fragments comprised primarily of a single antigen binding site can induce remyelination.

A parallel analysis of the human monoclonal antibody sHIgM 22 is performed to determine whether antibody fragments of this human IgM behave in a similar manner. If it can be determined that a single small binding domain can induce remyelination, this information may prove important for determining the mechanism of repair as well as provide an avenue for the development of an pharmacological analogue.

### EXAMPLE 9

### sHIgM 46 ANTIBODY INDUCES MYELIN REPAIR

Our initial studies suggests that the induction of myelin repair by sHIgM 46 may be qualitatively superior to the repair observed with sHIgM 22. Upon histological examination of sections of SJL mice that were chronically infected with TMEV, smaller areas of demyelination were observed following treatment with sHIgM 46 than with other monoclonal antibodies **(Table 7).** This observation was highly statistically significant. This result is notable because treatment with the antibody does not begin until demyelinated lesions are well established and have reached maximum size in chronically infected animals. Our interpretation of this result is that myelin repair is so complete in some areas of the spinal cord that they are not being distinguished from normal areas of the cord during our standard histological examination. The lesions in sHIgM 46 treated mice are examined by electron microscopy to confirm whether the repaired lesions contain higher numbers of myelin wraps than in other treatment groups.

**Table 7. Qualitative differences in myelin repair by human antibody sHIgM 46**

| Treatment | % White Matter Demyelinated | Statistical Evaluation |
|---|---|---|
| sHIgM 46 (n=15) | 4.07 (+/-2.52) | |
| All other antibodies (n=70) | 10.41 (+/-6.26) | P<0.001 |

SJL/J mice chronically infected with Theiler's virus for more than 9 months were divided into groups and individual groups received a single 0.5 mg ip injection of one of a battery of monoclonal antibodies. After five weeks animals were perfused with fixative and their spinal cords were isolated for histological analysis. The area of demyelination was determined by measuring the total area of the cord occupied by white matter and the area of demyelination visualized by light microscope using 25X optics. The data are comprised of mice from three independent experiments. Antibodies used to treat animals in the pooled treatment group ("all other antibodies") were human monoclonal IgM sHIgM 12, 14, 22, 47, 50, AKJR8, MSI 10E10, 2B2GE7, NA8FE4, and mouse antibodies 06, 09, RIP, and MOG. The data set passed normality tests and were analyzed by ANOVA.

### EXAMPLE 10

The sequences of the heavy and light chain variable regions of human antibodies AKJR4, CB2iE12 and CB2iE7, and the light chain variable region of MSI19E5 were determined. The sequences of the heavy and light chain variable region of AKJR4 are shown in **Figures 37** and **38** **,** respectively (SEQ ID NOS: 23 and 25). The sequences of the heavy and light chain variable region of CB2iE12 are shown in **Figures 39** and **40****,** respectively (SEQ ID NOS: 27 and 29). The sequences of the heavy and light chain variable region of CB2iE7 are shown in **Figures 41** and **42****,** respectively (SEQ ID NOS: 31 and 33. The sequence of the light chain variable region of MSI19E5 is shown in **Figure 43****,** respectively (SEQ ID NO: 35).

## Claims

1. A human antibody, or a mixture, monomer or active fragment thereof which has:
(i) a heavy chain variable domain comprising the CDR1, CDR2 and CDR3 region sequences as set forth in Figure 39, and a light chain variable domain comprising the CDR1, CDR2 and CDR3 region sequences as set forth in Figure 40; or,
(ii) a heavy chain variable domain comprising the CDR1, CDR2 and CDR3 region sequences as set forth in Figure 41, and a light chain variable domain comprising the CDR1, CDR2 and CDR3 region sequences as set forth in Figure 42.

2. A human antibody, or a mixture, monomer or active fragment thereof which has:
(i) a heavy chain variable domain having the amino acid sequence of CB2iE12 as set forth in Figure 39, and a light chain variable domain having the amino acid sequence of CB2iE12 as set forth in Figure 40; or
(ii) a heavy chain variable domain having the amino acid sequence of CB2iE7 as set forth in Figure 41, and a light chain variable domain having the amino acid sequence of CB2iE7 as set forth in Figure 42.

3. A pharmaceutical composition comprising, as the active agent, a human antibody, or a mixture, monomer or active fragment thereof, as defined in any one of claims 1 or 2.

4. A neuromodulatory agent comprising an antibody according to any one of claims 1 or 2 that functions to promote neurite outgrowth, regeneration or remyelination.

5. An isolated nucleic acid sequence or a degenerate variant thereof, which encodes a human antibody, an active fragment thereof, or a monomer, or a synthetic antibody derived therefrom, as defined in any one of claims 1 or 2.

6. A vector which comprises the nucleic acid molecule of claim 5.

7. A host vector system for the production of a polypeptide which comprises the vector of claim 6 in a suitable host cell.

8. A method of obtaining a polypeptide in purified form which comprises:
(i) culturing a host vector system of claim 7 so as to produce the polypeptide;
(ii) recovering the polypeptide produced in step (i); and
(iii) purifying the polypeptide recovered in step (ii).

9. The antibody of any one of claims 1 or 2, for use in preventing and/or treating cellular debilitations, derangements, dysfunctions and/or disease states in mammals including humans, optionally wherein the cellular debilitations, derangements, dysfunctions and/or disease states are CNS conditions where nerves are damaged such as by trauma or a viral demyelinating disease, or a post-neural disease of the central nervous system.

10. An antibody of any one of claims 1 or 2 which is monoclonal.

## Patentansprüche

1. Menschlicher Antikörper oder ein Gemisch, Monomer oder aktives Fragmente davon, der Folgendes aufweist:
(i) eine variable Domäne der schweren Kette, die die CDR1-, CDR2- und CDR3-Region-Sequenzen umfasst, wie in Figur 39 dargestellt, und eine variable Domäne der leichten Kette, die die CDR1-, CDR2- und CDR3-Region-Sequenzen umfasst, wie in Figur 40 dargestellt; oder
(ii) eine variable Domäne der schweren Kette, die die CDR1-, CDR2- und CDR3-Region-Sequenzen umfasst, wie in Figur 41 dargestellt, und eine variable Domäne der leichten Kette, die die CDR1-, CDR2- und CDR3-Region-Sequenzen umfasst, wie in Figur 42 dargestellt.

2. Menschlicher Antikörper oder ein Gemisch, Monomer oder aktives Fragment davon, das Folgendes aufweist:
(i) eine variable Domäne der schweren Kette mit der Aminosäuresequenz von CB2iE12, wie in Figur 39 dargestellt, und eine variable Domäne der leichten Kette mit der Aminosäuresequenz von CB2iE12, wie in Figur 40 dargestellt; oder
(ii) eine variable Domäne der schweren Kette mit der Aminosäuresequenz von CB2iE7, wie in Figur 41 dargestellt, und eine variable Domäne der leichten Kette mit der Aminosäuresequenz von CB2iE7, wie in Figur 42 dargestellt.

3. Pharmazeutische Zusammensetzung, umfassend als Wirkstoff einen menschlichen Antikörper oder ein Gemisch, Monomer oder aktives Fragment davon nach einem der Ansprüche 1 oder 2.

4. Neuromodulatorisches Mittel, umfassend einen Antikörper nach einem der Ansprüche 1 oder 2, das die Förderung von Neuritenwachstum, -regeneration oder -remyelinisierung bewirkt.

5. Isolierte Nukleinsäuresequenz oder eine degenerierte Variante davon, die einen menschlichen Antikörper, ein aktives Fragment davon oder ein Monomer oder einen davon stammenden synthetischen Antikörper nach einem der Ansprüche 1 oder 2 codiert.

6. Vektor, der das Nukleinsäuremolekül nach Anspruch 5 umfasst.

7. Wirt-Vektor-System zur Herstellung eines Polypeptids, das den Vektor nach Anspruch 6 in einer geeigneten Wirtszelle umfasst.

8. Verfahren zum Erhalten eines Polypeptids in gereinigter Form, das Folgendes umfasst:
(i) Kultivieren eines Wirt-Vektor-Systems nach Anspruch 7, um das Polypeptid herzustellen;
(ii) Gewinnen des in Schritt (i) hergestellten Polypeptids; und
(iii) Reinigen des in Schritt (ii) gewonnenen Polypeptids.

9. Antikörper nach einem der Ansprüche 1 oder 2 für die Verwendung zur Vorbeugung und/oder Behandlung von zellulären Schwächungen, Störungen, Dysfunktionen und/oder Krankheitszuständen bei Säugetieren, einschließlich Menschen, wobei es sich gegebenenfalls bei den zellulären Schwächungen, Störungen, Dysfunktionen und/oder Krankheitszuständen um ZNS-Zustände, wobei Nerven geschädigt sind, wie durch Trauma oder virale demyelinisierende Krankheit, oder eine post-neurale Krankheit des zentralen Nervensystems handelt.

10. Antikörper nach einem der Ansprüche 1 oder 2, der monoklonal ist.

## Revendications

1. Anticorps humain, ou un mélange, monomère ou fragment actif de celui-ci, qui possède :
(i) un domaine variable de chaine lourde comprenant les séquences de région CDR1, CDR2 et CDR3 telles qu'exposées à la Figure 39, et un domaine variable de chaine légère comprenant les séquences de région CDR1, CDR2 et CDR3 telles qu'exposées à la Figure 40 ; ou
(ii) un domaine variable de chaine lourde comprenant les séquences de région CDR1, CDR2 et CDR3 telles qu'exposées à la Figure 41, et un domaine variable de chaine légère comprenant les séquences de région CDR1, CDR2 et CDR3 telles qu'exposées à la Figure 42.

2. Anticorps humain, ou un mélange, monomère ou fragment actif de celui-ci, qui possède :
(i) un domaine variable de chaine lourde ayant la séquence d'acides aminés de CB2iE12 telle qu'exposée à la Figure 39, et un domaine variable de chaine légère ayant la séquence d'acides aminés de CB2iE12 telle qu'exposée à la Figure 40 ; ou
(ii) un domaine variable de chaine lourde ayant la séquence d'acides aminés de CB2iE7 telle qu'exposée à la Figure 41, et un domaine variable de chaine légère ayant la séquence d'acides aminés de CB2iE7 telle qu'exposée à la Figure 42.

3. Composition pharmaceutique comprenant, comme agent actif, un anticorps humain, ou un mélange, monomère ou fragment actif de celui-ci, tel que défini selon l'une quelconque des revendications 1 ou 2.

4. Agent de neuro-modulation comprenant un anticorps selon l'une quelconque des revendications 1 ou 2 qui fonctionne afin de promouvoir l'excroissance, la régénération ou la remyélination des neurites.

5. Séquence d'acide nucléique isolée ou un variant dégénéré de celle-ci, qui code pour un anticorps humain, un fragment actif de celui-ci ou un monomère, ou un anticorps synthétique en étant dérivé, tel que défini selon l'une quelconque des revendications 1 ou 2.

6. Vecteur comprenant la molécule d'acide nucléique selon la revendication 5.

7. Système de vecteur hôte pour la production d'un polypeptide qui comprend le vecteur selon la revendication 6 dans une cellule hôte convenable.

8. Méthode d'obtention d'un polypeptide sous forme purifiée, comprenant :
(i) la culture d'un système de vecteur hôte selon la revendication 7, de façon à produire le polypeptide ;
(ii) la récupération du polypeptide produit dans l'étape (i) ; et
(iii) la purification du polypeptide récupéré dans l'étape (ii).

9. Anticorps selon l'une quelconque des revendications 1 ou 2, pour une utilisation dans la prévention et/ou le traitement de débilitations, dérangements, dysfonctionnements cellulaires et/ou états pathologiques chez les mammifères y compris l'homme, éventuellement où les débilitations, dérangements, dysfonctionnements cellulaires et/ou états pathologiques sont des conditions du SNC où les nerfs sont endommagés tel que par un traumatisme ou une maladie de démyélinisation virale, ou une maladie post-neurale du système nerveux central.

10. Anticorps selon l'une quelconque des revendications 1 ou 2, qui est monoclonal.


### SEQUENCE LISTING - CORRECTION

<110> MAYO FOUNDATION FOR MEDICAL EDUCATION AND RESEARCH
<120> HUMAN IGM ANTIBODIES AND DIAGNOSTIC AND THERAPEUTIC USES THEREOF PARTICULARLY IN THE CENTRAL NERVOUS SYSTEM
<130> P055698EP
<140> EP 00948498.1
   <141> 2000-05-30
<160> 50
<170> PatentIn Ver. 2.0
<210> 1
   <211> 119
   <212> PRT
   <213> Homo sapiens
<400> 1
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Ser
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Arg Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Thr Ala Asp Asp Thr Ala Val Tyr Tyr Cys
                   85                  90                  95
   Ala Lys Gly Val Thr Gly Ser Pro Thr Leu Asp Tyr Trp Gly Gln Gly
               100                 105                 110
   Thr Leu Val Thr Val Ser Ser
           115
<210> 2
   <211> 357
   <212> DNA
   <213> Homo sapiens
<400> 2
   caggtgcagc tggtggagtc tgggggaggc gtggtccagc ctgggaggtc cctgagactc 60
   tcctgtgcag cctctggatt caccttcagt agctatggca tgcactgggt ccgccaggct 120
   ccaggcaagg ggctggagtg ggtggcagtt atatcatatg atggaagtaa taaatactat 180
   gcagactccg tgaagggccg attcaccatc tccagagaca attccaagaa cacgctgtat 240
   ctgcaaatga acagcctgag agctgaggac acggctgtgt attactgtgc gaaagaggtg 300
   actgctattc cctactttga ctactggggc cagggaaccc tggtcaccgt ctcctca    357
<210> 3
   <211> 357
   <212> DNA
   <213> Homo sapiens
<400> 3
   caggtgcagc tggtggagtc tggggggggc gtggtccagc ctgggaggtc cctgagactc 60
   tcctgtgcag cctctggatt caccttcagt agctctggca tgcactgggt ccgccaagct 120
   ccaggcaagg ggctggagtg ggtggcagtt atttcatatg atggaagtag gaaatactat 180
   gcagactccg tgaagggccg attcaccatc tccagagaca actccaagaa cactctgtat 240
   ctgcaaatga acagcctgac ggctgacgac acggctgtgt attattgtgc gaaaggagtg 300
   actggtagtc cgacgcttga ctactggggc cagggagccc tggtcaccgt ctcctca    357
<210> 4
   <211> 357
   <212> DNA
   <213> Homo sapiens
<400> 4
   caggtgcagc tggtggagtc tgggggaggc gtggtccagc ctgggaggtc cctgagactc 60
   tcctgtgcag cctctggatt caccttcagt agctctggca tgcactgggt ccgccaggct 120
   ccaggcaagg ggctggagtg ggtggcaatc atttcatatg atggaagtac gaaatactat 180
   gcagactccg tgaagggccg attcaccatc tccagagaca actccaagaa cactctctat 240
   ctgcaaatga acagcctgac agctgaggac acggctgtgt attactgtgc gaaaggagtg 300
   actggtagtc cgacgcttga ctactggggc cagggaaccc tggtcaccgt ctcctcg    357
<210> 5
   <211> 114
   <212> PRT
   <213> Homo sapiens
<400> 5
   Gln Ser Val Leu Thr Gln Pro Pro Ser Val Ser Ala Ala Pro Gly Gln
   1               5                   10                  15
   Lys Val Thr Ile Ser Cys Ser Gly Ser Ser Ser Asn Ile Gly Asn Asn
               20                  25                  30
   Phe Val Ser Trp Tyr Gln Gln Leu Pro Gly Thr Ala Pro Arg Leu Leu
           35                  40                  45
   Ile Tyr Asp Ile Thr Lys Arg Pro Ser Gly Ile Pro Asp Arg Phe Ser
       50                  55                  60
   Gly Ser Lys Ser Gly Thr Ser Ala Thr Leu Gly Ile Thr Gly Leu Gln
   65                  70                  75                  80
   Thr Gly Asp Glu Ala Asp Tyr Tyr Cys Gly Thr Trp Asp Ser Ser Leu
                   85                  90                  95
   Ser Ala Val Val Phe Gly Gly Gly Thr Lys Leu Thr Val Leu Gly Gln
               100                 105                 110
   Pro Lys
<210> 6
   <211> 337
   <212> DNA
   <213> Homo sapiens
<400> 6
   cagtctgtgt tgacgcagcc gccctcagtg tctgcggccc caggacagaa ggtcaccatc 60
   tcctgctctg gaagcagctc caacattggg aataattatg tatcctggta ccagcagctc 120
   ccaggaacag cccccaaact cctcatttat gacaataata agcgaccctc agggattcct 180
   gaccgattct ctggctccaa gtctggcacg tcagccaccc tgggcatcac cggactccag 240
   actggggacg aggccgatta ttactgcgga acatgggata gcagcctgtg tggtattcgg 300
   cggagggacc aagctgaccg tcctaggtca gcccaag                          337
<210> 7
   <211> 342
   <212> DNA
   <213> Homo sapiens
<400> 7
   cagtctgtgt tgacggagcc gccttcagtg tctgctgccc caggacagaa ggtcaccatc 60
   tcctgctctg gaagcagctc caacattggc aataattttg tatcctggta ccagcaactc 120
   ccaggaacag cccccagact cctcatttat gacattacta agcgaccctc agggattcct 180
   gaccgattct ctggctccaa gtctggcacg tcagccaccc tgggcatcac cggactccag 240
   actggggacg aggccgatta ttactgcgga acatgggata gcagcctgag tgctgtggta 300
   ttcggcgggg ggaccaagct gaccgtccta ggtcagccca ag                    342
<210> 8
   <211> 342
   <212> DNA
   <213> Homo sapiens
<400> 8
   cagtctgtgt tgacggagcc gccttcagtg tctgctgccc caggacagaa ggtcaccatc 60
   tcctgctctg gaagcagctc caacattggc aataattttg tatcctggta ccagcaactc 120
   ccaggaacag cccccaaact cctcatttat gacattacta agcgaccctc agggattcct 180
   gaccgattct ctggctccaa gtctggcacg tcagccaccc tgggcatcac cggactccag 240
   actggggacg aggccgatta ttactgcgaa acatgggata gcagcctgag tgctgtggta 300
   ttcggcgggg ggaccaagct gaccgtccta ggtcagccca ag                    342
<210> 9
   <211> 121
   <212> PRT
   <213> Homo sapiens
<400> 9
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Tyr Tyr Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Ser Ala Gln Gln Gln Leu Val Tyr Tyr Phe Asp Tyr Trp Gly Gln
               100                 105                 110
   Gly Thr Leu Val Thr Val Ser Ser Gly
           115                 120
<210> 10
   <211> 370
   <212> DNA
   <213> Homo sapiens
<400> 10
   caggtgcagc tgcaggagtc gggcccagga ctggtgaagc cttcggagac cctgtccctc 60
   acctgcactg tctctggtgg ctccatcagt agttactact ggagctggat ccggcagccc 120
   ccagggaagg gactggagtg gattgggtat atctattaca gtgggagcac caactacaac 180
   ccctccctca agagtcgagt caccatatca gtagacacgt ccaagaacab ccagttctcc 240
   ctgaagctga gctctgtgac cgctgcggac acggccabcg tgtattactg tgcgaggtcg 300
   gcacagcagc agctggtata ctacdtttga ctactggggc cagggaaccc tggtcaccgt 360
   ctcctcaggg                                                        370
<210> 11
   <211> 119
   <212> PRT
   <213> Homo sapiens
<400> 11
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Ser Thr Pro Leu Thr Phe Gly Pro Gly Thr Lys Val Asp Ile
               100                 105                 110
   Lys Arg Thr Val Ala Ala Pro
           115
<210> 12
   <211> 357
   <212> DNA
   <213> Homo sapiens
<400> 12
   gacatcgtga tgacccagtc tccagactcc ctggctgtgt ctctgggcga gagggccacc 60
   atcaactgca agtccagcca gagtgtttta tacagctcca acaataagaa ctacttagct 120
   tggtaccagc agaaaccagg acagcctcct aagctgctca tttactgggc atctacccgg 180
   gaatccgggg tccctgaccg attcagtggc agcgggtctg ggacagattt cactctcacc 240
   atcagcagcc tgcaggctga agatgtggca gtttattact gtcagcaata ttatagtact 300
   cctctcactt tcggccctgg gaccaaagtg gatatcaaac gaactgtggc tgcacca    357
<210> 13
   <211> 122
   <212> PRT
   <213> Homo sapiens
<220>
   <221> unsure
   <222> 1-10
   <223> "Xaa"s in the sequence represent unknown amino acids.
<400> 13
   Xaa Xaa Xaa Xaa Xaa Xaa Xaa Xaa Xaa Xaa Ala Val Val Gln Pro Gly
   1               5                   10                  15
   Arg Ser Leu Arg Leu Ser Cys Ala Ala Ser Gly Phe Ile Phe Ser Ser
               20                  25                  30
   Tyr Gly Met His Trp Val Arg Gln Val Pro Gly Lys Gly Leu Glu Trp
           35                  40                  45
   Val Ala Val Ile Trp Tyr Asp Gly Ser Asp Lys Tyr Tyr Val Asp Ser
       50                  55                  60
   Val Lys Gly Arg Phe Thr Ile Ser Arg Asp Asn Ser Lys Asn Thr Leu
   65                  70                  75                  80
   Tyr Leu Gln Met Asn Ser Leu Arg Ala Glu Asp Thr Ala Val Tyr Tyr
                   85                  90                  95
   Cys Ala Arg Asp Arg Ser Ser Gly Trp Tyr Trp Ser Cys Asp Ser Trp
               100                 105                 110
   Gly Gln Gly Thr Leu Val Ile Val Ser Ser
           115                 120
<210> 14
   <211> 366
   <212> DNA
   <213> Homo sapiens
<220>
   <221> unsure
   <222> 1-25
   <223> All "n"s in the sequence represent unknown nucleotide.
<220>
   <221> unsure
   <222> 28-30
   <223> All "n"s in the sequence represent unknown nucleotide.
<400> 14
   nnnnnnnnnn nnnnnnnnnn nnnnnagnnn gccgtggtcc agcctgggag gtccctgaga  60
   ctctcctgtg cagcgtctgg attcattttc agtagctatg gcatgcactg ggtccgccag  120
   gttccaggca aggggctgga gtgggtggca gttatatggt atgatggaag tgataaatac  180
   tatgtagact ccgtgaaggg ccgattcacc atctccagag acaattctaa aaacacgctc  240
   tatctgcaaa tgaacagcct gagagccgag gacacggctg tgtattactg tgcgagagat  300
   cgcagcagtg gctggtactg gtcctgcgac tcctggggcc agggaaccct ggtcattgtc  360
   tcctca                                                              366
<210> 15
   <211> 127
   <212> PRT
   <213> Homo sapiens
<220>
   <221> unsure
   <222> 1-8
   <223> All "Xaa"s in the sequence represent unknown amino acids.
<220>
   <221> unsure
   <222> 10
   <223> All "Xaa"s in the sequence represent unknown amino acids.
<220>
   <221> unsure
   <222> 127
   <223> All "Xaa"s in the sequence represent unknown amino acids.
<400> 15
   Xaa Xaa Xaa Xaa Xaa Xaa Xaa Xaa Leu Xaa Leu Ser Gly Ser Pro Gly
   1               5                   10                  15
   Gln Ser Ile Thr Ile Ser Cys Thr Gly Thr Ser Ser Asp Val Gly Gly
               20                  25                  30
   Tyr Asn Tyr Val Ser Trp Tyr Gln Gln His Pro Gly Lys Ala Pro Lys
           35                  40                  45
   Leu Met Ile Tyr Asp Val Ser Asp Arg Pro Ser Gly Val Ser Asn Arg
       50                  55                  60
   Phe Ser Gly Ser Lys Ser Gly Asn Thr Ala Ser Leu Thr Ile Ser Gly
   65                  70                  75                  80
   Leu Gln Ala Glu Asp Glu Ala Asp Tyr Tyr Cys Ser Ser Tyr Thr Ser
                   85                  90                  95
   Ser Ser Ser Val Val Phe Gly Gly Gly Thr Lys Leu Thr Val Leu Gly
               100                 105                 110
   Gln Pro Lys Ala Ala Pro Ser Val Thr Leu Phe Pro Pro Pro Xaa
           115                 120                 125
<210> 16
   <211> 382
   <212> DNA
   <213> Homo sapiens
<220>
   <221> unsure
   <222> 1-19
   <223> All "n"s in the sequence represent unknown nucleotides.
<220>
   <221> unsure
   <222> 22
   <223> All "n"s in the sequence represent unknown nucleotides.
<220>
   <221> unsure
   <222> 28-30
   <223> All "n"s in the sequence represent unknown nucleotides.
<220>
   <221> unsure
   <222> 380
   <223> All "n"s in the sequence represent unknown nucleotides.
<400> 16
   nnnnnnnnnn nnnnnnnnnt tngcctcnnn ctgtctgggt ctcctggaca gtcgatcacc  60
   atctccctga ctggaaccag cagtgacgtt ggtggttata actatgtctc ctggtaccaa  120
   cagcacccag gcaaagcccc caaactcatg atttatgatg tcagtgatcg gccctcaggg  180
   gtttctaatc gcttctctgg ctccaagtct ggcaacacgg cctccctgac catctctggg  240
   ctccaggctg aggacgaggc tgattattac tgcagctcat atacaagcag cagctctgtg  300
   gtattcggcg gagggaccaa gctgaccgtc ctaggtcagc ccaaggctgc cccctcggtc  360
   actctgttcc cgcctccaan gg                                           382
<210> 17
   <211> 120
   <212> PRT
   <213> Mus musculus
<400> 17
   Gln Asp His Leu Gln Gln Ser Gly Pro Glu Leu Val Lys Pro Gly Ala
   1               5                   10                  15
   Phe Val Lys Ile Ser Cys Lys Ala Ser Gly Tyr Thr Phe Thr Asn Tyr
               20                  25                  30
   Asp Leu Asn Trp Val Arg Gln Arg Pro Gly Gln Gly Leu Glu Trp Ile
           35                  40                  45
   Gly Trp Ile Tyr Pro Gly Asn Asp Asn Thr Lys Tyr Asn Glu Lys Phe
       50                  55                  60
   Lys Gly Leu Ala Ser Leu Thr Ala Asp Lys Ser Ser Thr Thr Ala Tyr
   65                  70                  75                  80
   Leu His Leu Ser Ser Leu Thr Ser Glu Ser Ser Ala Val Tyr Phe Cys
                   85                  90                  95
   Ala Arg Gly Leu Pro Arg Gly Trp Tyr Phe Asp Val Trp Gly Ala Gly
               100                 105                 110
   Thr Thr Val Thr Val Ser Ser Ala
           115                 120
<210> 18
   <211> 360
   <212> DNA
   <213> Mus musculus
<400> 18
   caggatcacc tgcagcagtc tggacctgag ctggtgaagc ctggggcttt tgtgaagata  60
   tcctgcaagg cttctggtta caccttcaca aactacgatc taaactgggt gaggcagagg  120
   cctggacagg gccttgagtg gattggatgg atttatcctg gaaatgataa tactaagtac  180
   aatgagaagt tcaagggcct ggcctcactg actgcagaca agtcctccac cacagcctac  240
   ttgcatctca gcagcctgac ttctgagagc tctgcagtct atttctgtgc aagagggtta  300
   cctaggggct ggtacttcga tgtctggggc gcagggacca cggtcaccgt ctcctcagct  360
<210> 19
   <211> 101
   <212> PRT
   <213> Mus musculus
<400> 19
   Asn Ile Val Met Thr Gln Ser Pro Lys Ser Met Ser Met Ser Val Gly
   1               5                   10                  15
   Glu Arg Val Thr Leu Thr Cys Lys Ala Ser Glu Asn Val Val Thr Tyr
               20                  25                  30
   Val Ser Trp Tyr Gln Gln Lys Pro Glu Gln Ser Pro Lys Leu Leu Ile
           35                  40                  45
   Tyr Gly Ala Ser Asn Arg Tyr Thr Gly Val Pro Asp Arg Phe Thr Gly
       50                  55                  60
   Ser Gly Ser Ala Thr Asp Phe Thr Leu Thr Ile Ser Ser Val Gln Ala
   65                  70                  75                  80
   Glu Asp Leu Ala Asp Tyr His Cys Gly Gln Gly Tyr Ser Tyr Pro Tyr
                   85                  90                  95
   Thr Phe Gly Gly Gly
               100
<210> 20
   <211> 303
   <212> DNA
   <213> Mus musculus
<400> 20
   aacattgtaa tgacccaatc tcccaaatcc atgtccatgt cagtaggaga gagggtcacc  60
   ttgacctgca aggccagtga gaatgtggtt acttatgttt cctggtatca acagaaacca  120
   gagcagtctc ctaaactgct gatatacggg gcatccaacc ggtacactgg ggtccccgat  180
   cgcttcacag gcagtggatc tgcaacagat ttcactctga ccatcagcag tgtgcaggct  240
   gaagaccttg cagattatca ctgtggacag ggttacagct atccgtacac gttcggaggg  300
   ggg                                                                303
<210> 21
   <211> 101
   <212> PRT
   <213> Mus musculus
<400> 21
   Asp Val Gln Ile Thr Gln Ser Pro Ser Tyr Leu Ala Ala Phe Pro Gly
   1               5                   10                  15
   Glu Thr Ile Thr Ile Asn Cys Arg Ala Ser Lys Ser Ile Ser Lys Tyr
               20                  25                  30
   Leu Ala Trp Tyr Gln Glu Arg Pro Gly Lys Thr Asn Lys Leu Leu Ile
           35                  40                  45
   Tyr Ser Gly Ser Thr Leu Gln Ser Gly Ile Pro Ser Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Met Tyr Tyr Cys Gln Gln His Asn Glu Tyr Pro Tyr
                   85                  90                  95
   Thr Phe Gly Gly Gly
               100
<210> 22
   <211> 303
   <212> DNA
   <213> Mus musculus
<400> 22
   gatgtccaga taacccagtc tccatcttat cttgctgcat ttcctggaga aaccattact  60
   attaattgta gggcaagtaa gagcattagt aaatatttag cctggtatca agagagacct  120
   ggaaaaacta ataagcttct tatctactct ggatccactt tgcaatctgg aattccatca  180
   aggttcagtg gcagtggatc tggtacagat ttcactctca ccatcagtag cctggagcct  240
   gaagattttg caatgtatta ctgtcaacag cataatgaat acccgtatac gttcggaggg  300
   ggg                                                                303
<210> 23
   <211> 124
   <212> PRT
   <213> Homo sapiens
<400> 23
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Ser Phe Ile Asp Tyr
               20                  25                  30
   Ala Met Ser Trp Val Arg Gln Ala Pro Gly Lys Gly Leu Glu Trp Val
           35                  40                  45
   Ser Ser Leu Ser Gly Asp Ser Gly Ser Ser Tyr Tyr Ala Asp Ser Val
       50                  55                  60
   Lys Gly Arg Phe Thr Ile Ser Arg Asp Asn Ser Lys Ser Thr Val Phe
   65                  70                  75                      80
   Leu Gln Leu Ser Ser Leu Arg Ala Glu Asp Thr Ala Ile Tyr Tyr Cys
                   85                  90                  95
   Ala Gln Glu Thr Gly Pro Gln Arg Arg Trp Gly Gln Gly Thr Leu Val
               100                 105                 110
   Thr Val Ser Ser Gly Ser Ala Ser Ala Pro Thr Leu
           115                 120
<210> 24
   <211> 372
   <212> DNA
   <213> Homo sapiens
<400> 24
   gaggtgcaac tattggaatc tgggggaggc ttggtacagc ctggggggtc cctgagactc  60
   tcctgtgcag cctctggatt cagctttatc gactatgcca tgagctgggt ccgccaggct  120
   ccagggaagg gactggagtg ggtctcaagt cttagtggtg atagtggtag ttcatattat  180
   gcagactccg tgaagggccg attcaccatc tccagagaca attccaagag cacggtgttt  240
   ctgcaactga gcagcctgag agccgaggac acggccatat attactgtgc gcaggagacc  300
   ggtccccagc gtcgctgggg ccagggaacc ctggtcaccg tctcctcagg gagtgcatcc  360
   gccccaaccc tt                                                      372
<210> 25
   <211> 116
   <212> PRT
   <213> Homo sapiens
<400> 25
   Asp Ile Gln Met Thr Gln Ser Pro Ser Thr Leu Ser Ala Ser Val Gly
   1               5                   10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Phe Asn Leu Glu Ser Gly Val Pro Ser Arg Phe Arg Gly
       50                  55                  60
   Ser Gly Ser Gly Thr Glu Phe Thr Leu Thr Ile Ser Ser Leu Gln Pro
   65                  70                  75                  80
   Asp Asp Ser Ala Thr Tyr Tyr Cys Gln Gln Tyr Ser Ser Tyr Pro Leu
                   85                  90                  95
   Thr Phe Gly Gly Gly Thr Lys Val Asp Ile Lys Arg Thr Val Ala Ala
               100                 105                 110
   Pro Ser Val Phe
           115
<210> 26
   <211> 348
   <212> DNA
   <213> Homo sapiens
<400> 26
   gacatccaga tgacccagtc tccttccacc ctgtctgcat ctgtagggga cagagtcacc  60
   atcacttgcc gggccagtca gagtattagt agctggttgg cctggtatca gcagaaacca  120
   gggaaagccc ctaaactcct gatctataag gcgtttaatt tagaaagtgg ggtcccatca  180
   aggttcagag gcagtggctc tgggacagaa ttcactctca ccatcagcag cctgcagcct  240
   gatgattctg caacttatta ctgccagcag tatagtagtt accccctcac tttcggcgga  300
   gggaccaagg tggacattaa acgaactgtg gctgcaccat ctgtcttc               348
<210> 27
   <211> 119
   <212> PRT
   <213> Homo sapiens
<220>
   <221> unsure
   <222> 1-8
   <223> All "Xaa"s in the sequence represent unknown amino acids.
<220>
   <221> unsure
   <222> 10-13
   <223> All "Xaa"s in the sequence represent unknown amino acids.
<220>
   <221> unsure
   <222> 15
   <223> All "Xaa"s in the sequence represent unknown amino acids.
<400> 27
   Xaa Xaa Xaa Xaa Xaa Xaa Xaa Xaa Arg Xaa Xaa Xaa Xaa Lys Xaa Glu
   1               5                   10                  15
   Ala Ser Val Lys Val Ser Cys Lys Ala Ser Gly Tyr Thr Phe Thr Gly
               20                  25                  30
   Tyr Tyr Met His Trp Val Arg Gln Ala Pro Gly Gln Gly Leu Glu Trp
           35                  40                  45
   Met Gly Trp Ile Asn Pro Asn Ser Gly Gly Thr Asn Tyr Ala Gln Lys
       50                  55                  60
   Phe Gln Gly Arg Val Thr Met Thr Arg Asp Thr Ser Ile Ser Thr Ala
   65                  70                  75                  80
   Tyr Met Glu Leu Ser Arg Leu Arg Ser Asp Asp Thr Ala Val Tyr Tyr
                   85                  90                  95
   Cys Ala Arg Asp Arg Ser Tyr Pro Gly Arg Asn Tyr Phe Asp Tyr Trp
               100                 105                 110
   Gly Gln Gly Thr Leu Val Thr
           115
<210> 28
   <211> 357
   <212> DNA
   <213> Homo sapiens
<220>
   <221> unsure
   <222> 1-22
   <223> All "n"s in the sequence represent unknown nucleotides.
<220>
   <221> unsure
   <222> 28-31
   <223> All "n"s in the sequence represent unknown nucleotides.
<220>
   <221> unsure
   <222> 34
   <223> All "n"s in the sequence represent unknown nucleotides.
<220>
   <221> unsure
   <222> 36
   <223> All "n"s in the sequence represent unknown nucleotides.
<220>
   <221> unsure
   <222> 38
   <223> All "n"s in the sequence represent unknown nucleotides.
<220>
   <221> unsure
   <222> 43
   <223> All "n"s in the sequence represent unknown nucleotides.
<400> 28
   nnnnnnnnnn nnnnnnnnnn nnccaggnnn nagnananga aancggaggc ctcagtgaag  60
   gtctcctgca aggcttctgg atacaccttc accggctact atatgcactg ggtgcgacag  120
   gcccctggac aagggcttga gtggatggga tggatcaacc ctaacagtgg tggcacaaac  180
   tatgcacaga agtttcaggg cagggtcacc atgaccaggg acacgtccat cagcacagcc  240
   tacatggagc tgagcaggct gagatctgac gacacggccg tgtattactg tgcgagagat  300
   cgatcgtatc cgggaaggaa ctactttgac tactggggcc agggaaccct ggtcacc     357
<210> 29
   <211> 101
   <212> PRT
   <213> Homo sapiens
<400> 29
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser His
                   85                  90                  95
   Thr Phe Gly Gln Gly
               100
<210> 30
   <211> 303
   <212> DNA
   <213> Homo sapiens
<400> 30
   gaaattgtgt tgacgcagtc tccaggcacc ctgtctttgt ctccagggga aagagccacc  60
   ctctcctgca gggccagtca gagtgttagc agcagctact tagcctggta ccagcagaaa  120
   cctggccagg ctcccaggct cctcatctat ggtgcatcca gcagggccac tggcatccca  180
   gacaggttca gtggcagtgg gtctgggaca gacttcactc tcaccatcag cagactggag  240
   cctgaagatt ttgcagtgta ttactgtcag cagtatggta gctctcacac ttttggccag  300
   ggg                                                                303
<210> 31
   <211> 119
   <212> PRT
   <213> Homo sapiens
<220>
   <221> unsure
   <222> 1-10
   <223> All "Xaa"s in the sequence represent unknown amino acids.
<400> 31
   Xaa Xaa Xaa Xaa Xaa Xaa Xaa Xaa Xaa Xaa Gly Leu Val Lys Pro Gly
   1               5                   10                  15
   Gly Ser Leu Arg Leu Ser Cys Ala Ala Ser Gly Phe Thr Phe Ser Asp
               20                  25                  30
   Tyr Tyr Met Ser Trp Ile Arg Gln Ala Pro Gly Lys Gly Leu Glu Trp
           35                  40                  45
   Val Ser Tyr Ile Ser Ser Ser Ser Ser Tyr Thr Asn Tyr Ala Asp Ser
       50                  55                  60
   Val Lys Gly Arg Phe Thr Ile Ser Arg Asp Asn Ala Lys Asn Ser Leu
   65                  70                  75                  80
   Tyr Leu Gln Met Asn Ser Leu Arg Ala Glu Asp Thr Ala Val Tyr Tyr
                   85                  90                  95
   Cys Ala Arg Asp Arg Ser Ser Ser Ser Trp Tyr Tyr Tyr Tyr Tyr Gly
               100                 105                 110
   Met Asp Val Trp Gly Gln Gly
           115
<210> 32
   <211> 357
   <212> DNA
   <213> Homo sapiens
<220>
   <221> unsure
   <222> 1-25
   <223> All "n"s in the sequence represent unknown nucleotides.
<220>
   <221> unsure
   <222> 28-30
   <223> All "n"s in the sequence represent unknown nucleotides.
<400> 32
   nnnnnnnnnn nnnnnnnnnn nnnnngannn ggcttggtca agcctggagg gtccctgaga   60
   ctctcctgtg cagcctctgg attcaccttc agtgactact acatgagctg gatccgccag   120
   gctccaggga aggggctgga gtgggtttca tacattagta gtagtagtag ttacacaaac   180
   tacgcagact ctgtgaaggg ccgattcacc atctccagag acaacgccaa gaactcactg   240
   tatctgcaaa tgaacagcct gagagccgag gacacggctg tgtattactg tgcgagagat   300
   cggtcgagca gcagctggta ctactactac tacggtatgg acgtctgggg ccaaggg      357
<210> 33
   <211> 102
   <212> PRT
   <213> Homo sapiens
<400> 33
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Tyr
               20                  25                  30
   Leu Ala Trp Tyr Gln Gln Lys Pro Gly Lys Val Pro Lys Leu Leu Ile
           35                  40                  45
   Tyr Ala Ala Ser Thr Leu Gln Ser Gly Val Pro Ser Arg Phe Asn Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Lys Tyr Asn Lys Cys Pro Ser
                   85                  90                  95
   His Phe Arg Gly Arg Asp
               100
<210> 34
   <211> 306
   <212> DNA
   <213> Homo sapiens
<400> 34
   gacatccaga tgacccagtc tccatcctcc ctgtctgcat ctgtaggaga cagagtcacc  60
   atcacttgcc gggcgagtca gggcattagc aattatttag cctggtatca gcagaaacca  120
   gggaaagttc ctaagctcct gatctatgct gcatccactt tgcaatcagg ggtcccatct  180
   cggttcaatg gcagtggatc tgggacagat ttcactctca ccatcagcag cctgcaacct  240
   gaagatgttg caacttatta ctgtcaaaag tataacaagt gcccctctca ctttcggggg  300
   agggac                                                             306
<210> 35
   <211> 105
   <212> PRT
   <213> Homo sapiens
<400> 35
   Asp Ile Ala Met Thr Gln Ser Pro Asp Ser Leu Ala Val Ser Leu Gly
   1               5                   10                  15
   Glu Arg Ala Thr Ile Asn Cys Lys Ser Ser Arg Ser Val Leu Phe Ser
               20                  25                  30
   Ser Asn Asn Asn Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Ser Thr Pro Ile Thr Phe Gly
               100                 105
<210> 36
   <211> 315
   <212> DNA
   <213> Homo sapiens
<400> 36
   gacatcgcga tgacccagtc tccagactcc ctggcagtgt ctctgggcga gagggccacc  60
   atcaactgca agtccagccg gagtgtttta ttcagctcca acaataacaa ctacttagct  120
   tggtaccagc agaaaccagg acagcctcct aagctactca tttactgggc atctacccgg  180
   gaatccgggg tccctgaccg attcagtggc agcgggtctg ggacagattt cactctcacc  240
   atcagcagcc tgcaggctga agatgtggca gtttattact gtcagcaata ttatagtact  300
   ccaatcacct tcggc                                                  315
<210> 37
   <211> 101
   <212> PRT
   <213> Mus musculus
<210> 38
   <211> 303
   <212> DNA
   <213> Mus musculus
<400> 38
   gacatcgtaa tgacgcagtc tcacaaattc atgtccactt cagtaggaga cagggtcagc  60
   atcacctgca aggccagtca ggatgtgagt actgctgtag cctggtatca acagaaacca  120
   ggacaatctc ctaaactact gatttactcg gcatcctacc ggtacactgg agtccctgat  180
   cgcttcactg gcagtggatc tgggacggat ttcactttca ccatcagcag tgtgcaggct  240
   gaagacctgg cagtttatta ctgtcagcaa cattatacta ctccgctcac gttcggtgct  300
   ggg                                                               303
<210> 39
   <211> 78
   <212> DNA
   <213> Artificial Sequence
<220>
   <223> Description of Artificial Sequence: Primer.
<400> 39
   actcccaagt cggctcgctt tctcttcagt gacaaacaca gacatagaac attcaccatg 60
   ggatggagct gtatcact                                               78
<210> 40
   <211> 47
   <212> DNA
   <213> Artificial Sequence
<220>
   <223> Description of Artificial Sequence: Primer.
<400> 40
   actgactctc ttaattaaga ctcacctgag gagactgtga gagtggt 47
<210> 41
   <211> 48
   <212> DNA
   <213> Artificial Sequence
<220>
   <223> Description of Artificial Sequence: Primer.
<400> 41
   ttggcgcgcc aaagactcag cctggacatg atgtcctctg ctcagttc 48
<210> 42
   <211> 43
   <212> DNA
   <213> Artificial Sequence
<220>
   <223> Description of Artificial Sequence: Primer.
<400> 42
   atagtttagc ggccgcattc ttatctaaca ctctcccctg ttg 43
<210> 43
   <211> 155
   <212> DNA
   <213> Artificial Sequence
<220>
   <223> Description of Artificial Sequence: Primer.
<400> 43
   gactcggtcc gcccagccac tggaagtcgc cggtgtttcc attcggtgat catcactgaa 60
   cacagaggac tcaccatgga gtttgggctg agctgggttt tcctcgttgc tcttttaaga 120
   ggtgtccagt gtcaggtgca gctggtggag tctgg 155
<210> 44
   <211> 56
   <212> DNA
   <213> Artificial Sequence
<220>
   <223> Description of Artificial Sequence: Primer.
<400> 44
   ccttaattaa gacctggaga ggccattctt acctgaggag acggtgacca gggttc 56
<210> 45
   <211> 36
   <212> DNA
   <213> Artificial Sequence
<220>
   <223> Description of Artificial Sequence: Primer.
<400> 45
   ctagctagcg tcctaggtca gcccaaggct gccccc 36
<210> 46
   <211> 36
   <212> DNA
   <213> Artificial Sequence
<220>
   <223> Description of Artificial Sequence: Primer.
<400> 46
   atagtttagc ggccgcacct atgaacattc tgtagg 36
<210> 47
   <211> 111
   <212> DNA
   <213> Artificial Sequence
<220>
   <223> Description of Artificial Sequence: Primer.
<400> 47
   ctagctagcc cgaatttcgg gacaatcttc atcatgacct gctcccctct cctcctcacc 60
   cttctcattc actgcacagg gtcctgggcc cagtctgtgt tgacgcagcc g 111
<210> 48
   <211> 32
   <212> DNA
   <213> Artificial Sequence
<220>
   <223> Description of Artificial Sequence: Primer.
<400> 48
   gggcagcctt gggctgagct aggacggtca gc 32
<210> 49
   <211> 119
   <212> PRT
   <213> Homo sapiens
<400> 49
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Ser
               20                  25                  30
   Gly Met His Trp Val Arg Gln Ala Pro Gly Lys Gly Leu Glu Trp Val
           35                  40                  45
   Ala Ile Ile Ser Tyr Asp Gly Ser Arg Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Thr Ala Glu Asp Thr Ala Val Tyr Tyr Cys
                   85                  90                  95
   Ala Lys Gly Val Thr Gly Ser Pro Thr Leu Asp Tyr Trp Gly Gln Gly
               100                 105                 110
   Thr Leu Val Thr Val Ser Ser
           115
<210> 50
   <211> 114
   <212> PRT
   <213> Homo sapiens
<400> 50
   Gln Ser Val Leu Thr Gln Pro Pro Ser Val Ser Ala Ala Pro Gly Gln
    1               5                  10                  15
   Lys Val Thr Ile Ser Cys Ser Gly Ser Ser Ser Asn Ile Gly Asn Asn
               20                  25                  30
   Phe Val Ser Trp Tyr Gln Gln Leu Pro Gly Thr Ala Pro Lys Leu Leu
           35                  40                  45
   Ile Tyr Asp Ile Thr Lys Arg Pro Ser Gly Ile Pro Asp Arg Phe Ser
       50                  55                  60
   Gly Ser Lys Ser Gly Thr Ser Ala Thr Leu Gly Ile Thr Gly Leu Gln
   65                  70                  75                  80
   Thr Gly Asp Glu Ala Asp Tyr Tyr Cys Glu Thr Trp Asp Ser Ser Leu
                   85                  90                  95
   Ser Ala Val Val Phe Gly Gly Gly Thr Lys Leu Thr Val Leu Gly Gln
               100                 105                 110
   Pro Lys